# EUROPEAN PATENT APPLICATION

(11) **EP 4 386 035 A1**
(43) Date of publication of application: **19.06.2024**
(21) Application number: 22855856.5
(22) Date of filing: 05.08.2022
(51) Int. Cl.: C08G 65/26, C08G 65/333, C11D 1/94, C11D 3/37, C11D 17/08

(54) **POLYALKYLENE-OXIDE-CONTAINING COMPOUND**

(30) Priority: 10.08.2021 JP 2021130538; 25.04.2022 JP 2022071593
(71) Applicant: Nippon Shokubai Co., Ltd., Osaka-shi, Osaka 541-0043 (JP)
(72) Inventor: ONO, Yasuhiro, Suita-shi, Osaka 564-0034 (JP); HOSOTANI, Tsutomu, Suita-shi, Osaka 564-0034 (JP); TAKESHIMA, Hisaaki, Suita-shi, Osaka 564-0034 (JP)
(74) Representative: Cabinet Beau de Loménie
(86) International application number: PCT/JP2022/030129
(87) International publication number: WO 2023/017794

(57) **Abstract**

The present invention aims to provide a polyalkylene oxide-containing compound having an excellent particulate clay dispersing ability and an excellent anti-redeposition ability, a method for producing the compound, and a detergent or detergent composition containing the compound. The present invention relates to a polyalkylene oxide-containing compound containing a cationic group, a linking group, and a structural unit derived from a polyalkylene oxide.

## Description

### TECHNICAL FIELD

The present disclosure relates to a polyalkylene oxide-containing compound having an excellent particulate clay dispersing ability and an excellent anti-redeposition ability, a method for producing the compound, and a detergent composition containing the compound.

### BACKGROUND ART

A polymer containing a polyalkyleneimine as a main chain and ethylene oxide or the like attached to nitrogen atoms in the polyalkyleneimine has been known as a polyalkyleneimine polyalkylene oxide. This polymer has been known to act as a polymer builder and has been used as an ingredient of liquid detergents because it is soluble in liquid detergents. Detergents containing a polyalkyleneimine polyalkylene oxide together with an activator are capable of preventing redeposition of soil washed off by cleaning and exhibiting a high cleaning power.

Various studies have been conducted on polyalkyleneimine polyalkylene oxides. For example, Patent Literature 1 discloses a copolymer having a structure obtained by the addition reaction of maleic anhydride to a terminal group of the polyalkylene oxide in a polyalkyleneimine alkylene oxide copolymer containing an alkyleneimine monomer unit having a polyalkylene oxide.

Patent Literature 2 discloses a polyalkyleneamine alkylene oxide copolymer containing an alkyleneamine structural unit having a polyalkylene oxide chain. The polyalkyleneamine alkylene oxide copolymer has a structure in which one or more or all of the terminal groups of the polyalkylene oxide chain of the copolymer have been subjected to an addition reaction with lauryl glycidyl ether.

### CITATION LIST

### - Patent Literature

Patent Literature 1: JP 2010-168185 A
Patent Literature 2: JP 2012-149185 A

### SUMMARY OF INVENTION

### - Technical Problem

The inventors of the present application found, as a result of examination so far, that an anti-redeposition ability can be improved by a detergent composition containing a polyalkylene-polyalkylene oxide copolymer including an alkyleneamine structural unit having a polyalkylene oxide chain in which the alkylene oxide is terminally modified.

However, the polymers described in Patent Literature 1 and Patent Literature 2, in which the terminal groups of the alkylene oxides are modified in a polyalkylene aminealkylene oxide copolymer containing alkyleneamine structural units having polyalkylene oxide chains, have room for improvement in anti-redeposition ability.

The present disclosure has been made in view of such problems, and aims to provide a polyalkylene oxide-containing compound having a good anti-redeposition ability, a method for producing the compound, and a detergent composition containing the compound.

### - Solution to Problem

Specifically, a polyalkylene oxide-containing compound of the present disclosure is a polyalkylene oxide-containing compound containing:
a cationic group;
a linking group; and
a structural unit derived from a polyalkylene oxide,
the linking group being bonded to the structural unit derived from a polyalkylene oxide.

Preferably, the linking group is one or more substituents selected from ester, thioester, amide, thioamide, acetal, hemiacetal, and hemiketal groups.

Preferably, the polyalkylene oxide-containing compound contains:
a substituent represented by the following formula (1); and
a cationic group containing two or more nitrogen atoms,
the substituent represented by the following formula (1) being bonded to at least one of the nitrogen atoms in the cationic group,
the formula (1) being as follows: 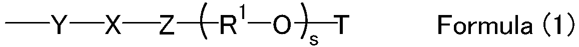 wherein R¹s are the same as or different from each other and each represent a C2-C6 hydrocarbon group; X represents one or more selected from a -C(=α¹)-β¹- group, a >C=α¹ group, a -α¹-CR²R³-β¹- group, and a -CR⁴(OH)-α¹- group, where α¹ and β¹ are the same as or different from each other and each represent a heteroatom or a group in which a hydrogen atom is bonded to a heteroatom, R², R³, and R⁴ are the same as or different from each other and each represent a hydrogen atom or a C1-C10 organic group; s is an integer from 1 to 300; Y represents a direct bond, a linear or branched C1-C10 hydrocarbon group, or a substituent represented by the following formula (2); Z represents a direct bond, a - (CH₂)ₙ-(S)ₘ-(CH₂)_{b}-O- group, a -(CH₂)ₙ-α²- group, or a substituent represented by the following formula (3), where α² represents a heteroatom or a group in which a hydrogen atom is bonded to a heteroatom, m is 0 or 1, n is an integer from 1 to 10, and p is an integer from 1 to 10; and T represents a hydrogen atom or a C1-C30 organic group,
the formula (2) being as follows: 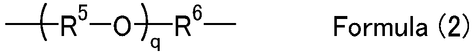 wherein q is an integer from 1 to 300; and R⁵ and R⁶ are the same as or different from each other and each represent a C2-C6 hydrocarbon group,
the formula (3) being as follows: 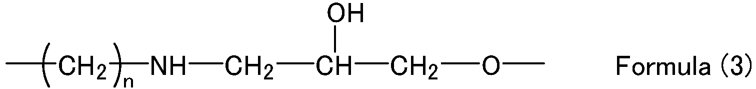 wherein n is an integer from 1 to 10.

Preferably, the heteroatom for or in α¹ and β¹ in X in the substituent represented by the formula (1) is an oxygen atom or a nitrogen atom.

Preferably, Y in the substituent represented by the formula (1) is a C2-C4 hydrocarbon group.

Preferably, the polyalkylene oxide-containing compound contains:
a cationic group to which no linking group is bonded; and
an unreacted NH group in the cationic group to which no linking group is bonded is present in an amount of 80 mol% or less based on the total number of moles of nitrogen atoms in the polyalkylene oxide-containing compound.

Preferably, in the polyalkylene oxide-containing compound, a number average molecular weight of a degradation product obtained by degrading the polyalkylene oxide-containing compound by a degradation test by alkaline hydrolysis or enzymatic degradation is 0.5 or less relative to a number average molecular weight of the polyalkylene oxide-containing compound before the degradation test.

The present invention also relates to a composition containing the polyalkylene oxide-containing compound of the present invention and an acid compound.

The present invention also relates to a method for producing the polyalkylene oxide-containing compound of the present invention, the method including

Michael addition of an α,β-unsaturated carbonyl compound having a polyalkylene oxide chain to an amino group as a cationic group.

The present invention also relates to a method for producing the polyalkylene oxide-containing compound of the present invention, the method including:
a first step of Michael addition of an α,β-unsaturated carbonyl compound to an amino group as a cationic group; and
a second step of introducing a polyalkylene oxide chain into a product obtained in the first step.

The present invention also relates to a method for producing the polyalkylene oxide-containing compound of the present invention, the method including:
a ring-opening addition reaction of one or more compounds selected from cyclic lactone compounds and cyclic lactam compounds with an amino group as a cationic group; and
introducing a polyalkylene oxide chain into active hydrogen generated in the ring-opening addition reaction.

The present invention also relates to a method for producing the polyalkylene oxide-containing compound of the present invention, the method including
reducing an amount of an unreacted NH group in a cationic group to which no linking group is bonded by one or more reactions selected from a Michael addition reaction, an acetylation reaction, an amidation reaction with a carboxylic acid anhydride, an amidation reaction with a carboxylic acid halide, and an epoxy compound addition reaction.

The present invention also relates to a method for producing the polyalkylene oxide-containing compound of the present invention, the method including
neutralizing with an acid compound an unreacted NH group in a cationic group to which no linking group is bonded.

The present invention also relates to a detergent or detergent composition containing the polyalkylene oxide-containing compound of the present invention.

Preferably, the detergent or cleaning composition is liquid and is selected from the group consisting of a laundry detergent composition, a hard surface cleaning composition, a hand dishwashing composition, and an automatic dishwashing composition.

Preferably, the detergent or cleaning composition is selected from the group consisting of a laundry detergent composition, a hard surface cleaning composition, a hand dishwashing composition, and an automatic dishwashing composition and is a liquid detergent or a liquid cleaning composition, and the liquid detergent or the liquid cleaning composition is enclosed in a single-compartment water-soluble pouch in a single-phase unit dose form or in a multi-compartment water-soluble pouch in a multiphase unit dose form.

Preferably, the detergent or cleaning composition further contains a surfactant selected from the group consisting of anionic surfactants, cationic surfactants, nonionic surfactants, amphoteric surfactants, zwitterionic surfactants, and mixtures thereof.

Preferably, in the detergent or cleaning composition, the surfactant is an anionic surfactant selected from the group consisting of alkylbenzene sulfonates, alkoxylated alkyl sulfates, alkyl sulfates, and mixtures thereof.

Preferably, the detergent or cleaning composition is a liquid laundry detergent composition further containing one or more cleaning adjunct additives selected from the group consisting of builders, structurants or thickeners, clay-soil removal or clay-soil anti-redeposition agents, stain release polymers, dispersant polymers, grease cleaning polymers, enzymes, enzyme stabilizing systems, bleaching compounds, bleaching agents, bleach activators, bleach catalysts, brighteners, dyes, hueing agents, dye transfer inhibitors, chelating agents, defoamers, softeners, fragrances, and mixtures thereof.

Preferably, the detergent or cleaning composition is substantially free of a zeolite builder nor a phosphate builder.

The present invention also relates to a softener containing the polyalkylene oxide-containing compound of the present invention.

The present invention also relates to a film composition containing the polyalkylene oxide-containing compound of the present invention.

The present invention also relates to a method for storing the polyalkylene oxide-containing compound of the present invention at a water concentration of 10% or less.

### - Advantageous Effects of Invention

The present disclosure can provide a polyalkylene oxide-containing compound having an excellent particulate clay dispersing ability and an excellent anti-redeposition ability, a method for producing the polyalkylene oxide-containing compound, and a detergent composition containing the polyalkylene oxide-containing compound.

### DESCRIPTION OF EMBODIMENTS

Embodiments of the present disclosure will be described in detail below. The preferred embodiments described below are merely exemplary in nature and are not intended to limit the present disclosure, its applications, or its uses.

In the following description, unless otherwise specified, the term "%" refers to "% by mass", the term "parts" refers to "parts by mass," and the phrase "A to B" indicating a range refers to "A or more and B or less". In the present disclosure, "(meth)acrylate" refers to "acrylate" or "methacrylate", and "(meth)acrylic" refers "acrylic" or "methacrylic".

### (Polyalkylene oxide-containing compound)

A polyalkylene oxide-containing compound of the present disclosure contains the following substituents and structural unit (1) to (3).
(1) Cationic group
(2) Linking group
(3) Structural unit derived from a polyalkylene oxide

The cationic group is an amino group-containing group. The amino group may be in the form of a primary amine, a secondary amine, a tertiary amine, or a quaternary amine.

The cationic group in the polyalkylene oxide-containing compound of the present disclosure may contain one amino group or two or more amino groups, and preferably contains two or more amino groups. When the polyalkylene oxide-containing compound contains two or more amino groups in its structure, the cationic group may be in the form of a polymer having two or more structural units each containing an amino group. Specific examples of the cationic group are described later.

Preferably, the polyalkylene oxide-containing compound of the present disclosure has a structure in which the structural unit derived from a polyalkylene oxide and the cationic group (or a structural unit having the cationic group) are bonded via the linking group without being directly bonded to each other. Preferably, the polyalkylene oxide-containing compound of the present disclosure has a structure in which the nitrogen atom of an amino group in the cationic group and the linking group are bonded to each other.

In the present disclosure, the structural unit derived from a polyalkylene oxide refers to a structural unit represented by -R-O- (R represents an alkylene group).

The linking group in the present disclosure is preferably one or more selected from ester, amide, thioester, thioamide, hemiacetal, hemiketal, and acetal groups.

The linking group is not limited as long as it is one or more selected from ester, amide, thioester, thioamide, hemiacetal, hemiketal, and acetal groups. The linking group is preferably one or more selected from ester, amide, thioester, thioamide, and acetal groups, more preferably one or more selected from an ester group and an amide group. The linking group is preferably any of these groups to increase the storage stability of the polyalkylene oxide-containing compound and the degradability thereof after use. Also, the particulate clay dispersing ability and the anti-redeposition ability of the polyalkylene oxide-containing compound of the present disclosure tend to improve.

In recent years, detergent compositions are required not only to have an excellent cleaning ability but also to be easily degraded in nature for environmental considerations. For this reason, the linking group is particularly preferably a hydrolyzable group or a functional group that can be degraded by an extracellular enzyme, but is not limited thereto. Particularly preferred among these linking groups described above are an ester group and an amide group.

Note that biodegradability means the properties of being able to be metabolized and degraded by, for example, bacteria, fungi, or other living organisms.

The polyalkylene oxide-containing compound of the present disclosure preferably contains a substituent represented by the following formula (1). The substituent represented by the following formula (1) has a structural site derived from a polyalkylene oxide represented by (-R¹-O-)ₛ and a linking group represented by -Y-X-Z-.

The polyalkylene oxide-containing compound of the present disclosure preferably has a structure in which a substituent represented by the following formula (1) is bonded to a cationic group. 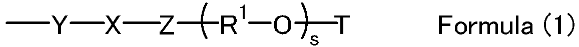 In the formula (1), R¹s are the same as or different from each other and each represent a C2-C6 hydrocarbon group; X represents one or more selected from a -C(=α¹)-β¹-group, a >C=α¹ group, a -α¹-CR²R³-β¹- group, and a -CR⁴(OH)-α¹- group, where α¹ and β¹ are the same as or different from each other and each represent a heteroatom or a group in which a hydrogen atom is bonded to a heteroatom, R², R³, and R⁴ are the same as or different from each other and each represent a hydrogen atom or a C1-C10 organic group; s is an integer from 1 to 300; Y represents a direct bond, a linear or branched C1-C10 hydrocarbon group, or a substituent represented by the following formula (2); Z represents a direct bond, a -(CH₂)ₙ-(S)ₘ-(CH₂)ₚ-O- group, a -(CH₂)ₙ-α²- group, or a substituent represented by the following formula (3), where α² represents a heteroatom or a group in which a hydrogen atom is bonded to a heteroatom, m is 0 or 1, n is an integer from 1 to 10, and p is an integer from 1 to 10; and T represents a hydrogen atom or a C1-C30 organic group.

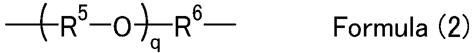

In the formula (2), q is an integer from 1 to 300; and R⁵ and R⁶ are the same as or different from each other and each represent a C2-C6 hydrocarbon group.

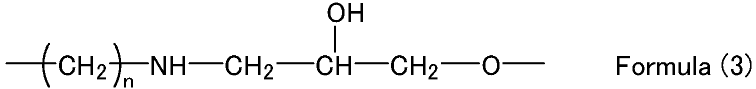

In the formula (3), n is an integer from 1 to 10.

X in the formula (1) is preferably one or more selected from a -C(=α¹)-β¹- group, a >C=α¹ group, a -α¹-CR²R³-β¹- group, and a -CR⁴(OH)-α¹- group. α¹ and β¹ each represent a heteroatom. X is preferably an ester group, a thioester group, an amide group, a thioamide group, an acetal group, or a hemiacetal group, more preferably an ester group, an amide group, or an acetal group.

When α¹ and β¹ each represent a heteroatom, each may be any heteroatom and is preferably one or more selected from an oxygen atom and a sulfur atom.

When α¹ and β¹ are each a group in which a hydrogen atom is bonded to a heteroatom, each is preferably an NH group.

To achieve both enhancement of the storage stability of the polyalkylene oxide compound and facilitation of degradation of the polyalkylene oxide compound, α¹ and β¹ are each preferably any of the substituents and atoms described above.

R¹ in the formula (1) is not limited as long as it is a C2-C6 hydrocarbon group. Preferably, R¹ is a C2-C4 hydrocarbon group. In the formula (1), s number of R⁴s may be hydrocarbon groups having the same carbon number or may be a combination of hydrocarbon groups having different carbon numbers. Preferably, the carbon number of R¹ is 2 to 4 as described above.

The -R¹-O- group in the formula (1) may be one or more selected from oxyethylene, oxypropylene, and oxybutylene groups.

To provide water solubility, better clay dispersing ability, and better compatibility with a liquid detergent to a polyalkylene oxide compound, the substituents and atoms described above are preferred.

R², R³, and R⁴ in the formula (1) are the same as or different from each other and are not limited as long as they are each a hydrogen atom or a C1-C10 organic group. Preferably, R², R³, and R⁴ in the formula (1) are each a hydrogen atom or a C1-C6 organic group, with a hydrogen atom or a C1-C6 hydrocarbon group being more preferred. Still more preferred is a hydrogen atom or a methyl group.

In the formula (1), s is not limited as long as it is an integer from 1 to 300. s is preferably from 1 to 200, more preferably from 2 to 100, still more preferably from 3 to 50.

To further improve the clay dispersing ability and anti-redeposition ability of the polyalkylene oxide compound, s preferably falls within the above range.

In the formula (1), Y is a direct bond, a linear or branched C1-C10 hydrocarbon group, or a group represented by the formula (2). When Y in the formula (1) is a C1-C10 hydrocarbon group, Y is preferably a C2-C6 hydrocarbon group, more preferably a C2-C4 hydrocarbon group. The hydrocarbon group is preferably an alkylene group.

In the formula (2), q is an integer from 1 to 300. The preferred range of q is the same as that of s in the formula (1).

R⁵ and R⁶ in the formula (2) are the same as or different from each other and are each a C2-C6 hydrocarbon group. Preferred hydrocarbon groups for R⁵ and R⁶ are the same as those for R¹ in the formula (1).

The polyalkylene oxide-containing compound of the present disclosure is preferably degraded at the linking group. In this case, in the linking group that connects the cationic group and the structural unit derived from a polyalkylene oxide, a site to be degraded is preferably bonded to a site at a certain distance from the cationic group without being directly bonded to the cationic group in order to ensure the degradability of the polyalkylene oxide compound.

In the formula (1), Z is not limited as long as it is a direct bond, a -(CH₂)ₙ-(S)ₘ-(CH₂)ₚ-O- group, a -(CH₂)ₙ-α²-group, or a group represented by the following formula (3).

α² may be a heteroatom. n and p each represent an integer from 1 to 10, and m is 0 or 1.

When α² is a heteroatom, it is not limited and is preferably an oxygen atom or a sulfur atom.

When α² is a group in which a hydrogen atom is bonded to a heteroatom, α² is preferably an NH group.

n and p are not limited as long as they are each an integer from 1 to 10, preferably from 1 to 5, more preferably from 1 to 3.

As long as m is 0 or 1, m is not limited.

From the viewpoint of ease of synthesis for introducing a linking group into the polyalkylene oxide, the above-mentioned substituents and ranges are preferred.

T in the formula (1) is the same as or different from each other and is not limited as long as T is a hydrogen atom or a C1-C30 organic group. To adjust hydrophilicity or hydrophobicity, the structure of T may be appropriately selected. The organic group is preferably a hydrocarbon group, more preferably a linear or branched alkyl, alkenyl, alkynyl, or aryl group.

The organic group is also preferably one having a structure in which a carboxy group, a phosphate group, or a sulfonate group is bonded to any of the above-described hydrocarbon groups.

When all the amino groups in the cationic group in the present disclosure are bonded to linking groups, no unreacted amino groups are present, i.e., no NH groups are present. In this case, the ratio of the NH groups is 0 mol%, and the ratio of the NH groups remaining unreacted is 0 mol% relative to the total number of moles of nitrogen atoms in the polyalkylene oxide-containing compound. On the other hand, unreacted amino groups may be present. In this case, the ratio of the unreacted NH groups in the cationic group to which no linking group is bonded is 100 mol% or less relative to the total number of moles of nitrogen atoms in the polyalkylene oxide-containing compound. It is more preferably 80 mol% or less, still more preferably 50 mol% or less, particularly preferably 20 mol% or less.

If the ratio of unreacted NH groups is high, the amount of a polyalkylene oxide group introduced into the polyalkylene oxide compound decreases. In view of reduction in clay dispersing ability and anti-redeposition ability, the ratio of unreacted NH groups preferably falls within the above-described range.

As described above, the cationic group may also be represented by an amino group-containing structural unit.

Specific examples thereof include structural units derived from compounds such as polyethylene polyamines (PEA) (e.g., diethylenetriamine, triethylenetetramine, and tetraethylenepentamine), tetrabutylenepentamine, polyethyleneimine (PEI), and polyamidoamine.

A precursor of the amino group-containing structural unit in the present disclosure may be one represented by the following formula (4). 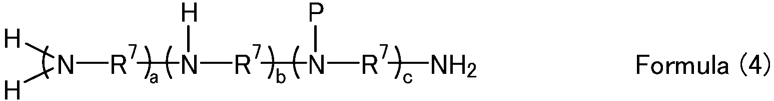 In the formula, R⁷s are the same as or different from each other and each represent a linear C2-C6 alkylene group or a branched C3-C6 alkylene group; P is the same as or different from each other and represents a hydrogen atom or a structural unit containing an amino group as a branch; and a, b, and c are the same as or different from each other and each represent an integer of 0 or 1 or more, with at least one of a, b, and c being an integer from 1 to 100. The amino group-containing structural unit contains at least two or more -N-R⁷- units.

When the precursor represented by the formula (4) is used, the cationic group in the polyalkylene oxide compound of the present disclosure is a group formed by removing a hydrogen atom bonded to the nitrogen atoms from the precursor.

Regarding the precursor of the amino group-containing structural unit, the structural formula of the structural unit constituting the precursor is, for example, H₂N-R⁷-, - NH-R⁷-, or -N(-)-R⁷-.

When P in the formula (4) is a structural unit containing an amino group, the structural unit containing an amino group is preferably one represented by the following formula (5), and is preferably bonded to a structure represented by the formula (4) via a R⁷' group.

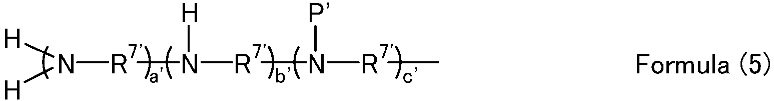

In the formula, a', b', c', P', and R⁷' are the same as a, b, c, P, and R⁷ in the formula (4), respectively.

In the structural formula of the precursor of the amino group-containing structural unit, R⁷s may consist of one type of alkylene groups or two or more types of alkylene groups. Preferably, R⁷s consist of one type of alkylene groups and are each an ethylene group. When each R⁷ is a branched C3-C6 alkylene group, it is preferably a 1,2-propylene group.

In the structural formula of the precursor of the amino group-containing structural unit, a, b, and c may be the same as or different from each other, may each be an integer of 0 or 1 or more, and are each preferably an integer from 0 to 100. The value of "a + b + c" is 1 or more. In a preferred embodiment of the precursor forming the amino group-containing structural unit, the value of "a + b + c" may be 1 to 4, 1 to 3, or 1 or 2. In another preferred embodiment of the precursor of the amino group-containing structural unit, the value of "a + b + c" is 5 or more.

An example of the polyalkylene oxide-containing compound of the present disclosure (having a substituent represented by the formula (1)) is a compound in which a linking group is bonded to the nitrogen atom of an amino group in a (poly)alkyleneamine having at least one selected from the group consisting of a primary amine nitrogen atom-containing constitutional unit, a secondary amine nitrogen atom-containing constitutional unit, and a tertiary amine nitrogen atom-containing constitutional unit and a structural unit derived from a polyalkylene oxide is bonded to the linking group via the other bond.

The primary amine nitrogen atom-containing constitutional unit is, for example, one represented by the following formula.

(H₂-N-R⁷)-

R⁷ is the same as that in the formula (4).

The primary amine nitrogen atom-containing structural unit may also be a structural unit represented by the following formula.

### -NH₂

The secondary amine nitrogen atom-containing constitutional unit is, for example, one represented by the following formula (6).

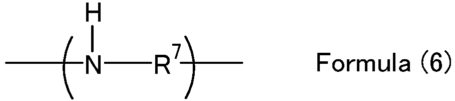

The tertiary amine nitrogen atom-containing constitutional unit is, for example, one represented by the following formula (7).

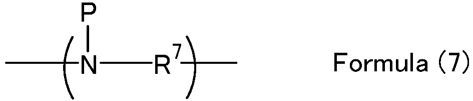

P and R⁷ in the formulas (6) and (7) are the same as those in the formula (4).

In a cationic group-containing structural unit, the structural unit may be present in any form. For example, the structural units may be present randomly. The nitrogen atom derived from an amino group in the cationic group may be quaternized or oxidized.

An example of the precursor of the cationic group-containing structural unit in the present disclosure represented by the formula (4) may be one represented by the following formula (8).

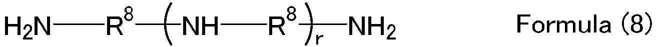

In the formula (8), R⁸s are the same as or different from each other and each represent a linear C2-C6 alkylene group or a branched C3-C6 alkylene group. In the formula, r is an integer from 0 to 10.

Specific examples of an amine compound having two primary amines are described below. The precursor of the cationic group-containing structural unit in the present disclosure is not limited to an amine compound having two primary amines.

The cationic group-containing structural unit in the present disclosure may be one represented by the following formula (9) in addition to the formulas (6) and (7).

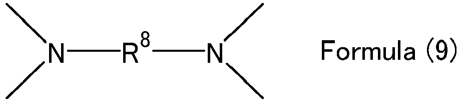

In the formula (9), R⁸s are the same as or different from each other and each represent a linear C2-C6 alkylene group or a branched C3-C6 alkylene group.

A diamine compound is a precursor of the structural unit represented by the formula (9).

When the diamine compound is reacted with a (meth)acrylic acid compound, the reaction proceeds through a compound represented by the following formula (10). The reaction of the diamine compound further proceeds to give a polyamine compound represented by the following formula (11) .

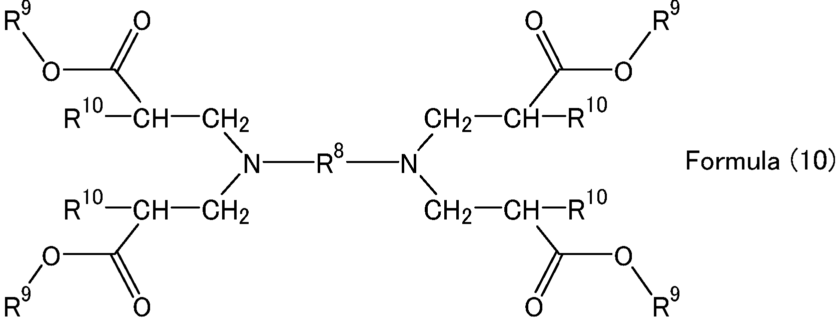

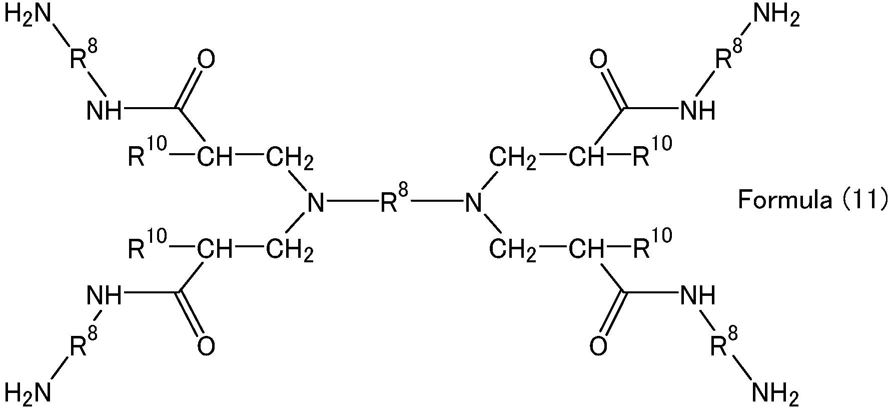

R⁸s in the formulas (10) and (11) are the same as R⁸s in the formulas (8) and (9). R⁹s are the same as or different from each other and each represent a hydrogen atom or a C1-C30 organic group, and R¹⁰s each represent a hydrogen atom or a methyl group.

R⁹s are the same as or different from each other and are not limited as long as R⁹s each represent a hydrogen atom or a C1-C30 organic group. R⁹s may be selected suitably to adjust hydrophilicity and hydrophobicity. The organic group is preferably a hydrocarbon group.

The compound represented by the formula (10) is produced by a Michael addition reaction of a (meth)acrylic acid compound to a diamine compound which is a precursor represented by the formula (9).

The compound represented by the formula (11) is produced by an ester-amide exchange reaction between the compound represented by the formula (10) and a diamine compound.

The polyalkylene oxide-containing compound of the present disclosure can be obtained by an addition reaction of a glycidyl ether compound having a polyalkylene oxide structure to the compound represented by the formula (11).

Alternatively, the polyalkylene oxide-containing compound of the present disclosure can be obtained by Michael addition reaction of a (meth)acrylic acid compound having a polyalkylene oxide structure to the compound represented by the formula (11).

Alternatively, the polyalkylene oxide-containing compound of the present disclosure can be obtained by addition polymerization of alkylene oxide to the compound represented by the formula (11).

### (Physical properties of polyalkylene oxide-containing compound of the present disclosure)

The molecular weight of the polyalkylene oxide-containing compound of the present disclosure can be reduced by a degradation reaction by at least one degradation technique selected from alkaline hydrolysis, enzymatic degradation, and active sludge treatment.

When the polyalkylene oxide-containing compound of the present disclosure is subjected to a degradation test by at least one degradation technique selected from alkaline hydrolysis, enzymatic degradation, and active sludge treatment, the number average molecular weight of a degradation product obtained by degrading the polyalkylene oxide-containing compound by a degradation test is preferably 0.5 or less relative to the number average molecular weight of the polyalkylene oxide-containing compound before the degradation test. The ratio is more preferably 0.4 or less, still more preferably 0.2 or less. When the number average molecular weight after the degradation test is smaller than that before the degradation test, the polyalkylene oxide-containing compound is preferably used as a detergent or a detergent composition. When such polyalkylene oxide-containing compound is used as a detergent or a detergent composition, environmental burden caused by compounds discharged after laundry can be reduced.

The weight average molecular weight of the polyalkylene oxide-containing compound of the present disclosure is preferably 1,000 to 1,000,000, more preferably 3,000 to 500,000, still more preferably 5,000 to 200,000. The weight average molecular weight of the polyalkylene oxide-containing compound of the present disclosure preferably falls within the above range. This is because such a polyalkylene oxide-containing compound has an excellent anti-redeposition ability and the degradation reaction can lead to a reduction in environmental burden.

The weight average molecular weight of the polyalkylene oxide-containing compound can be measured by the method described in the EXAMPLES.

### (Composition of polyalkylene oxide-containing compound of the present disclosure)

In the polyalkylene oxide-containing compound of the present disclosure, the unreacted NH groups in the cation group may be treated with an acid compound by the production method described below. The present invention also encompasses a composition containing a polyalkylene oxide-containing compound and an acid compound for treating the NH groups.

The amount of the acid compound is preferably 30% by mass or less based on the total amount, which is taken as 100% by mass, of the composition of a polyalkylene oxide-containing compound. The amount of the acid compound is more preferably 20% by mass or less, still more preferably 10% by mass or less.

The amount of the acid compound preferably falls within the above range because the stability of the polyalkylene oxide-containing compound of the present disclosure is improved.

Examples of the acid compound include acetic acid, citric acid, hydrochloric acid, phosphoric acid, nitric acid, sulfuric acid, and p-toluenesulfonic acid. The acid compound is not limited as long as it can react with an amino group.

### (Method for producing polyalkylene oxide-containing compound of the present disclosure)

An example of a method for producing the polyalkylene oxide-containing compound of the present disclosure is a production method (I) including a condensation reaction of a carboxy group-containing polyalkylene oxide compound with the hydrogen atoms of amino groups in a (poly)alkyleneamine or esterification of a carboxylic acid halide group-containing polyalkylene oxide compound with the hydrogen atoms of amino groups in a (poly)alkyleneamine.

The (poly)alkyleneamine used in the production method (I) is a compound having a structural formula represented by the formula (4). The carboxy group-containing polyalkylene oxide compound or the carboxylic acid halide group-containing polyalkylene oxide compound used in the production method (I) may be represented by the following formula (12).

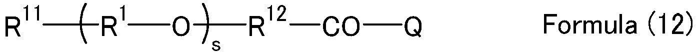

In the formula (12), R¹ and s are the same as those in the formula (1); R¹¹ represents a hydrogen atom or a C1-C20 organic group; and R¹² represents a C1-C6 organic group, where R¹¹ is preferably hydrogen, a linear or branched C1-C20 alkyl, alkenyl, alkynyl, or aryl group and R¹² is preferably a C1-C6 alkyl group; and Q represents OH, Cl, Br, or I.

Among compounds represented by the formula (12), a compound represented by the following formula (12') in which R¹² is an organic group terminated with a carbonyl group is preferred.

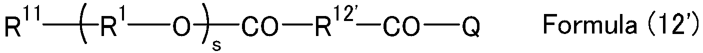

In the formula (12'), R¹, s, R¹¹, and Q are the same as those in the formula (12). R¹²' represents a C1-C5 organic group.

The organic group for R¹¹ in the formula (12) and the organic group for R¹²' in the formula (12') are each preferably a saturated or unsaturated hydrocarbon group.

Specific examples of the carboxy group-containing polyalkylene oxide compound include products obtained by an esterification reaction between an alkoxypolyalkylene glycol and a dicarboxylic acid anhydride such as succinic anhydride or maleic anhydride; products obtained by reaction between an alkoxypolyalkylene glycol and a carboxylic acid halide; and compounds obtained by oxidizing and carboxylating the terminal hydroxy group of alkoxy polyalkylene glycol with an oxidizing agent.

The temperature at which the (poly)alkyleneamine represented by the formula (4) is reacted with the carboxy group-containing polyalkylene oxide compound represented by the formula (12) is preferably 50°C to 200°C, more preferably 100°C to 200°C. The reaction temperature and reaction time preferably fall within the above ranges. This is because the reaction proceeds almost quantitatively and the reactivity is improved to reduce unreacted raw materials, whereby the particulate clay dispersing ability and the anti-redeposition ability of the polyalkylene oxide-containing compound of the present disclosure tend to improve.

Another example of the method for producing the polyalkylene oxide-containing compound of the present disclosure is a production method (II) including a ring-opening addition reaction of one or more compounds selected from cyclic lactone compounds and cyclic lactam compounds with an amino group in the cationic group, followed by introducing polyalkylene oxide chains into active hydrogen produced by the ring-opening addition reaction.

Specifically, ester bonds or amide bonds can be introduced by the ring-opening addition reaction of a caprolactone compound or a caprolactam compound with the hydrogen atoms of amino groups in a (poly)alkyleneamine. The terminal residue obtained by the ring-opening addition reaction of the caprolactone compound or the caprolactam compound is a hydroxy group or an NH₂ group. The hydroxy group or NH₂ group is subjected to a ring-opening addition reaction of an epoxy compound such as ethylene oxide, whereby the polyalkylene oxide-containing compound of the present disclosure can be produced.

The (poly)alkyleneamine used in the production method (II) is a compound having a structural formula represented by the formula (4).

The lactone compound to be used in the production method (II) may be α-lactone (three-membered ring), β-lactone (four-membered ring), γ-lactone (five-membered ring), δ-lactone (six-membered ring), ε-lactone (seven-membered ring), or a derivative thereof. Specific examples include α-acetolactone, β-propiolactone, γ-butyrolactone, δ-valerolactone, and ε-caprolactone. The lactam compound may be α-lactam (three-membered ring), β-lactam (four-membered ring), γ-lactam (five-membered ring), δ-lactam (six-membered ring), ε-lactam (seven-membered ring), or a derivative thereof. One or more of these may be used.

In the production method (II), at least one selected from the lactone compounds and the lactam compounds is attached to the hydrogen atoms of amino groups in the formula (4) by a ring-opening addition reaction of the at least one selected from the lactone compounds and the lactam compounds with the (poly)alkyleneamine represented by the formula (4). Thereby, a (poly)alkyleneamine-lactone adduct and/or a (poly)alkyleneamine-lactam adduct can be obtained. The following described the scheme of an example of this reaction.

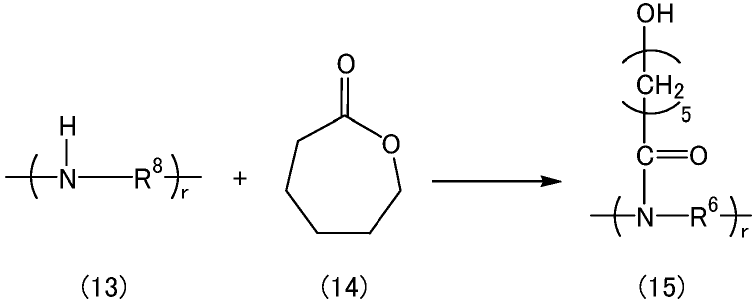

R⁸ and r in the formula (13), formula (14), and formula (15) are the same as those in the formula (8).

In the production method (II), the reaction temperature of the ring-opening addition reaction between the (poly)alkyleneamine represented by the formula (4) and at least one selected from the lactone compounds and the lactam compounds is preferably 0°C to 100°C, more preferably 20°C to 80°C.

The reaction time is preferably one hour or more.

In the (poly)alkyleneamine-lactone adduct and the (poly)alkyleneamine-lactam adduct obtained as intermediates in the production method (II), the terminal residue obtained by the addition reaction of at least one selected from the lactone compounds and the lactam compounds is a hydroxy group. The hydroxy group is subjected to a ring-opening addition reaction of alkylene oxide such as ethylene oxide or propylene oxide, whereby the polyalkylene oxide-containing compound of the present disclosure can be obtained. The reaction conditions for the ring-opening addition reaction of alkylene oxide are the same as those for conventional methods.

Yet another example of the method for producing the polyalkylene oxide-containing compound of the present disclosure may be a production method (III) for producing a compound in which a polyamine and alkylene oxide are bonded to each other via an ester bond by Michael addition of the double bonds of an (alkoxy)polyalkylene oxide (meth)acrylate to the amino groups of a polyalkyleneamine.

The (poly)alkyleneamine used in the production method (III) may be a compound having the structural formula represented by the formula (4). The (alkoxy)polyalkylene oxide (meth)acrylate compound or an (alkoxy)polyalkylene glycol (meth)acrylamide used in the production method (III) may be represented by the following formula (16).

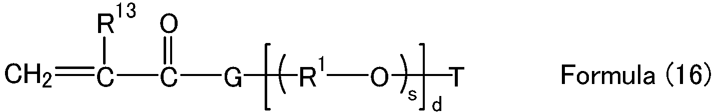

In the formula (16), R¹, s, and T are the same as those in the formula (1); R¹³ is the same as or different from each other and is a hydrogen atom or a methyl group; G represents O, N, or NH; and d is 1 or 2. When G is N, d is 2, and when G is O or NH, d is 1.

The formula (16) may also be represented by the following formula (17).

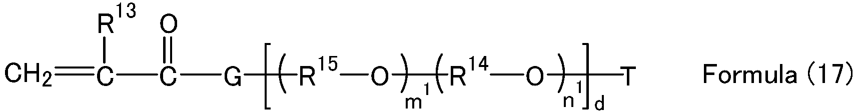

In the formula (17), R¹³, G, T, and d are the same as those in the formula (16).

m¹ and n¹ are each an integer from 0 to 300, and the value of "m¹ + n¹" is the same as s in the formula (1).

R¹⁴ and R¹⁵ are the same as or different from each other and each represent a C2-C6 hydrocarbon group.

R¹⁴ is a C2-C6 hydrocarbon group, preferably a C2-C4 hydrocarbon group, more preferably a C2-C3 hydrocarbon group, still more preferably a C2 hydrocarbon group.

R¹⁵ is a C3-C6 hydrocarbon group, preferably a C3-C4 hydrocarbon group.

m¹ is not limited as long as it is an integer from 0 to 300, preferably 1 to 10, more preferably 1 to 5, still more preferably 1 to 3.

When m¹ is 1 or more, R¹³ is preferably a hydrogen atom.

Specific examples of a compound represented by the formula (17) in which m¹ is 1 or more include (meth)acrylic acid esters in which an alkylene glycol having three or more carbon atoms is bonded to the ester bond, such as (alkoxy)polyethylene glycol (poly)propylene glycol (meth)acrylic acid ester and (alkoxy)polyethylene glycol (poly)butylene glycol (meth)acrylic acid ester. Particularly preferred are (alkoxy)polyethylene glycol (poly)propylene glycol acrylic acid ester, (alkoxy)polyethylene glycol (poly)butylene glycol acrylic acid ester, methoxypolyethylene glycol (poly)propylene glycol acrylic acid ester, and methoxypolyethylene glycol (poly)butylene glycol acrylic acid ester.

When m¹ is 0, (alkoxy)polyethylene glycol methacrylic acid ester and methoxypolyethylene glycol methacrylic acid ester are preferred.

The value of m¹ preferably falls within the above range. This is because the ester bond can have hydrophobicity and steric hindrance and the ester bond can have improved stability during storage in a protic solvent such as water, methanol, or ethanol.

n¹ is not limited as long as it is an integer from 0 to 300, preferably 1 to 200, more preferably 5 to 100, still more preferably 10 to 50.

The value of n¹ preferably falls within the above-mentioned range. This is because the particulate clay dispersing ability, clay anti-redeposition ability, and stability in blending into a liquid detergent are improved.

When R¹⁴ is a C2 hydrocarbon group, the amount of the -R¹⁴-O- group is preferably 70 mol or more based on the total amount, which is taken as 100 mol%, of the -R¹⁵-O-group and the -R¹⁴-O- group in the formula (17). The amount of the -R¹⁴-O- group is more preferably 75 mol or more, still more preferably 80 mol or more.

When the amount of the -R¹⁴-O- group in the formula (17) in which R¹⁴ is a C2 hydrocarbon group falls within the above-mentioned range, the hydrophilicity of the polyalkylene oxide chain is improved. This is preferred in terms of improvement in the particulate clay dispersing ability and the clay anti-redeposition ability.

In the production method (III), the (alkoxy)polyalkylene oxide (meth)acrylate represented by the formula (16) or (17) is bonded to the hydrogen atoms of amino groups in the (poly)alkyleneamine represented by the formula (4) by a Michael addition reaction. Thereby, a (poly)alkyleneamine-(alkoxy)polyalkylene oxide (meth)acrylate adduct can be obtained.

In the production method (III), the reaction temperature of the Michael addition reaction between the (poly)alkyleneamine represented by the formula (4) and the (alkoxy)polyalkylene oxide (meth)acrylate represented by the formula (16) or (17) is preferably 20°C to 100°C, more preferably 40°C to 80°C.

The reaction time is preferably one hour or more, more preferably five hours or more, still more preferably ten hours or more. The reaction temperature and reaction time preferably fall within the above-mentioned ranges. This is because the reaction proceeds almost quantitatively to be improved, thereby reducing the amount of unreacted raw material.

When production of the (alkoxy)polyalkylene oxide (meth)acrylate used in the production method (III) includes esterification of (meth)acrylic acid and an (alkoxy)polyalkylene oxide, the (meth)acrylic acid may remain. In this case, since the Michael addition of the (alkoxy)polyalkylene oxide (meth)acrylate proceeds more easily than that of the (meth)acrylic acid, the (alkoxy)polyalkylene oxide (meth)acrylate can be preferentially attached to the (poly)alkyleneamine represented by the formula (4) by reacting the (poly)alkyleneamine represented by the formula (4) with the (alkoxy)polyalkylene oxide (meth)acrylate at a low temperature. By increasing the reaction temperature or extending the reaction time, (meth)acrylic acid can also be attached to the (poly)alkyleneamine represented by the formula (4) by Michael addition.

Yet another example of the method for producing the polyalkylene oxide-containing compound of the present disclosure is a production method similar to the production method (III). The similar production method includes Michael addition of an α,β-unsaturated carbonyl compound having a polyalkylene oxide chain to the amino groups in cationic groups.

Yet another example of the method for producing the polyalkylene oxide-containing compound of the present disclosure is another production method similar to the production method (III). The another similar production method includes Michael addition of an α,β-unsaturated carbonyl compound to the amino groups in cationic groups, followed by introducing polyalkylene oxide chains thereto.

In the production method (III), the (poly)alkyleneamine represented by the formula (4) and the (alkoxy)polyalkylene oxide (meth)acrylate represented by the formula (16) or (17) may be reacted without a solvent. When the reaction is performed using a reaction solvent, the reaction solvent may be selected from alcohols such as methanol and ethanol; alkanes such as pentane, hexane, and cyclohexane; ethers such as diethyl ether and tetrahydrofuran; polar solvents such as dimethyl sulfoxide and dimethylformamide; water; and solvent mixtures of water and organic solvents.

Yet another example of the method for producing the polyalkylene oxide-containing compound of the present disclosure is a production method (IV) including Michael addition of the double bond of acrylamide to the amino groups of a (poly)alkyleneamine to introduce an amide bond, followed by a ring-opening addition reaction of an epoxy compound such as ethylene oxide to an amino group or a hydroxy group as a terminal residue obtained by the Michael addition.

Yet another example of the method for producing the polyalkylene oxide-containing compound of the present disclosure is a production method (V) in which a (poly)alkyleneamine alkoxylate is obtained by a ring-opening addition reaction of an epoxy compound such as ethylene oxide with the hydrogen atoms of amino groups in a (poly)alkyleneamine, and is acetalized with an (alkoxy)polyalkylene oxide, an acetalizing agent, and an acid catalyst.

Yet another example of the method for producing the polyalkylene oxide-containing compound of the present disclosure is a production method (VI) in which an ester-containing compound is obtained by Michael addition of the double bond of an alkyl (meth)acrylate to the amino groups in a (poly)alkyleneamine, and is subjected to ester-amide exchange with an (alkoxy)polyalkylene oxide-containing compound terminated with amine.

Yet another example of the method for producing the polyalkylene oxide-containing compound of the present disclosure is a production method (VII) in which an ester-containing compound is obtained by Michael addition of the double bond of an alkyl (meth)acrylate to the amino groups in a (poly)alkyleneamine, and is reacted with (alkoxy)polyalkylene glycol for transesterification.

The production methods (I) to (VII) preferably include, in addition to the above-mentioned steps, a step of reducing the amount of unreacted NH groups to which no linking group is bonded in the cationic groups by subjecting the unreacted NH groups to at least one reaction selected from a Michael addition reaction, an acetylation reaction, an amidation reaction with a carboxylic acid anhydride, an amidation reaction with a carboxylic acid halide, and an epoxy compound addition reaction.

Specifically, the amount of unreacted NH groups to which no linking group is bonded in the cationic groups can be reduced as follows: the unreacted NH groups are subjected to Michael addition of an acrylic acid alkyl ester such as methyl acrylate; the unreacted NH groups are subjected to an amidation reaction with a carboxylic acid anhydride (e.g., acetic anhydride, succinic anhydride, or maleic anhydride) or a carboxylic acid halide (e.g., acetyl chloride or propionyl chloride); or the unreacted NH groups are subjected to addition of an epoxy compound such as ethylene oxide or propylene oxide.

The amount of unreacted NH groups based on the total NH groups in the polyalkylene oxide-containing compound is 80 mol or less, preferably 50 mol% or less, more preferably 20 mol% or less, from the viewpoint of improvement in clay dispersing ability, anti-redeposition ability, and storage stability.

The production methods preferably further include neutralizing with an acid compound as another technique of reducing the amount of NH groups.

Specifically, the amount of NH groups to which no linking group is bonded in the cationic groups is preferably reduced by neutralizing the unreacted NH groups with an acid compound such as acetic acid, citric acid, hydrochloric acid, phosphoric acid, nitric acid, sulfuric acid, p-toluenesulfonic acid, or any of other common acid compounds.

The amount of unreacted NH groups based on the total NH groups in the polyalkylene oxide-containing compound is 80 mol or less, preferably 50 mol% or less, more preferably 20 mol% or less, from the viewpoint of improvement in clay dispersing ability, anti-redeposition ability, and storage stability.

The amount of the acid compound added is preferably 50 to 300 mol% relative to the NH groups in the cationic groups before adding the acid compound.

### (Uses of polyalkylene oxide-containing compound)

The polyalkylene oxide-containing compound of the present disclosure (also simply referred to as "the compound of the present disclosure") can be suitably used in detergent builders, detergents, water treatment agents, dispersants, fiber treatment agents, scale inhibitors (scale suppressors), cement additives, metal ion-chelating agents, thickeners, and various binders, for example. Of these, it can be suitably used in detergent builders, detergents, water treatment agents, and dispersants.

The present disclosure also provides a detergent builder, a detergent, a water treatment agent, or a dispersant, containing the polyalkylene oxide-containing compound of the present disclosure or a polyalkylene oxide-containing compound produced by the production method of the present disclosure as an essential ingredient.

### <Detergent or cleaning composition>

### (Detergent or cleaning composition containing polyalkylene oxide-containing compound of the present disclosure)

The present disclosure provides a detergent or cleaning composition, preferably a laundry detergent composition, more preferably a liquid laundry detergent composition, containing the polyalkylene oxide-containing compound of the present disclosure and optional other adjunct components.

The terms "detergent composition", "cleaning composition", and "detergent or cleaning composition" are broadly defined above and are hereinafter used interchangeably. Specifically, the detergent or cleaning composition may be in any solid product form or any liquid product form and may be a laundry detergent composition, a hard-surface cleaning composition, a hand dishwashing composition, or an automatic dishwashing composition. The detergent or cleaning composition is preferably liquid, more preferably in a single-phase unit dose form or in a multiphase unit dose form. In other words, the liquid detergent or cleaning composition is contained in a single-compartment water-soluble pouch or in a multi-compartment water-soluble pouch. In a certain embodiment, the detergent or cleaning composition is a liquid automatic dishwashing composition or a liquid laundry detergent composition, and the liquid automatic dishwashing composition or the liquid laundry detergent composition is enclosed in a single-compartment water-soluble pouch in a single-phase unit dose form or in a multi-compartment water-soluble pouch in a multiphase unit dose form, with each pouch being formed from a water-soluble polymer such as polyvinyl alcohol (PVA) and/or polyvinylpyrrolidone (PVP).

The detergent or cleaning composition may contain any amount of the polyalkylene oxide-containing compound of the present disclosure. To achieve high builder performance, the amount of the polyalkylene oxide-containing compound of the present disclosure is about 0.1% by mass to about 15% by weight, more preferably about 0.3% by weight to about 10% by weight, still more preferably about 0.5% by mass to about 5% by weight, based on the total amount of the detergent or cleaning composition.

When the detergent or cleaning composition is a liquid laundry detergent composition, the polyalkylene oxide-containing compound of the present disclosure may further contain one or more organic solvents in an amount from about 1% by weight to about 80% by weight, preferably from about 10% by weight to about 60% by weight, more preferably from about 15% by weight to about 50% by weight, even more preferably from about 20% by weight to about 45% by weight, based on the total weight of the composition.

Liquid laundry detergent compositions usually suffer from phase separation, especially when the compositions have a high salt content. In response to such a problem, the solvent system in the detergent or cleaning composition of the present disclosure is designed to stabilize the polyalkylene oxide-containing compound of the present disclosure to minimize the risk of phase separation. Specifically, the solvent system in the detergent or cleaning composition of the present disclosure preferably mainly contains a diol such as ethylene glycol, diethylene glycol, propylene glycol, dipropylene glycol, butylene glycol, pentanediol, or a combination thereof.

Preferably, the liquid laundry detergent composition of the present disclosure contains the diols in a total amount ranging from about 2% by weight to about 50% by weight. More preferably, the composition contains ethylene, diethylene glycol, and/or propylene glycol in a total amount ranging from about 5% by weight to about 40% by weight. Still more preferably, the composition contains propylene glycol in an amount ranging from about 15% by weight to about 35% by weight.

The detergent or cleaning composition of the present disclosure may further contain a different solvent. Examples of the different solvent include methanol, ethanol, glycerol, sodium cumene sulfonate, potassium cumene sulfonate, ammonium cumene sulfonate, sodium toluene sulfonate, potassium toluene sulfonate, sodium xylene sulfonate, potassium xylene sulfonate, ammonium xylene sulfonate, and mixtures thereof. The different solvent is not limited to these and a different organic solvent may be contained. Other lower alcohols such as C1-C4 alkanolamines (e.g., monoethanolamine and/or triethanolamine) may also be used. In a particularly preferred embodiment of the present disclosure, the liquid laundry detergent composition of the present disclosure may contain, in addition to the diols, glycerol in an amount from about 5% by weight to about 20% by weight, preferably 6% by weight to 18% by weight, more preferably 8% by weight to 16% by weight.

The liquid laundry detergent composition of the present disclosure preferably contains water as a carrier in combination with any of the organic solvents described above. In some embodiments, the liquid laundry detergent composition of the present disclosure contains water in an amount from about 20% by weight to about 70% by weight, preferably about 25% by weight to 60% by weight, more preferably about 30% by weight to about 50% by weight. In other embodiments, water is absent and the composition is anhydrous. A highly preferred composition provided by the present disclosure is a clear isotropic liquid.

Preferably, the detergent or cleaning composition of the present disclosure contains one or more surfactants and one or more cleaning adjunct additives. The specific forms of the surfactant and the cleaning adjunct additive are not limited, and are appropriately selected based on common knowledge in the field of detergents.

### <Anionic surfactants>

The detergent or cleaning composition of the present disclosure may contain one or more anionic surfactants. The detergent or cleaning composition of the present disclosure may essentially contain one or more anionic surfactants and may further contain a different surfactant system.

Non-limiting examples of the anionic surfactant include any known anionic surfactants. Examples include sulfate detersive surfactants for alkoxylated and/or non-alkoxylated alkyl sulfate materials, sulfonic detersive surfactants, and alkylbenzene sulfonates.

Examples of alkoxylated alkyl sulfates include ethoxylated alkyl sulfate surfactants known as alkyl ether sulfates or alkyl polyethoxylate sulfates. Examples of ethoxylated alkyl sulfates include water-soluble salts, particularly alkali metal, ammonium, and alkylolammonium salts, of organic sulfuric reaction products having in their molecular structure an alkyl group containing from about 8 to 30 carbon atoms; and a sulfonic acid and its salts.

The term "alkyl" includes the alkyl site of an acyl group. An example of the alkyl is a C15-C30 alkyl group.

The alkyl ether sulfate surfactant may be a mixture of alkyl ether sulfates. The mixture has an average (arithmetic mean) carbon chain length corresponding to about 12 to 30 carbon atoms. In another example, the average carbon chain length corresponds to about 25 carbon atoms. The average (arithmetic mean) number of moles of EO added is about 1 to 4 moles. In another example, the average (arithmetic mean) number of moles of EO added is about 1.8 moles.

In yet other examples, the alkyl ether sulfate surfactant may have a carbon chain length corresponding to about 10 to about 18 carbon atoms and a degree of ethoxylation of about 1 to about 6 moles of ethylene oxide. In yet additional examples, the alkyl ether sulfate surfactant may have a peaked ethoxylate distribution as described in WO 1995011212 A1, U.S. Patent No. 5120697, U.S. Patent No. 5210325, U.S. Patent No. 4946984, U.S. Patent No. 4902658, and WO 2010099303 A1.

Example of non-alkoxylated alkyl sulfates include non-ethoxylated alkyl sulfates. Non-ethoxylated alkyl sulfates may also be added to the disclosed detergent composition and used as an anionic surfactant component. Examples of non-alkoxylated (e.g., non-ethoxylated) alkyl sulfate surfactants include those produced by sulfation of higher C8-C20 fatty alcohols. In some examples, primary alkyl sulfate surfactants have the formula: ROSO₃⁻M⁺, where R is typically a linear C8-C20 hydrocarbyl group (which may be a linear or branched chain), and M is a water-solubilizing cation. In some examples, R is C10-C15 alkyl and M is an alkali metal. In other examples, R is C12-C14 alkyl and M is sodium.

Other useful anionic surfactants include alkali metal salts of alkylbenzene sulfonates containing an alkyl group containing from about 9 to about 15 carbon atoms in a linear or branched configuration. Examples include those described in U.S. Patent No. 2,220,099 and U.S. Patent No. 2,477,383.

In some examples, the alkyl group is linear. Such linear alkylbenzene sulfonates are known as "LASs." In other examples, the linear alkylbenzene sulfonate may have an average number of carbon atoms in the alkyl group of from about 11 to 14. In a specific example, the linear alkyl benzene sulfonates may have an alkyl group having an average number of carbon atoms of about 11.8, and may be abbreviated as C11.8 LAS. Such surfactants and their preparation are described, for example, in U.S. Patent No. 2,220,099 and U.S. Patent No. 2,477,383.

Suitable alkyl benzene sulfonates (LASs) may be obtained by sulfonating commercially available linear alkyl benzenes (LAB). Suitable LAB includes low 2-phenyl LAB, such as those supplied by Sasol under the trade name Isochem^{®} or those supplied by Petresa under the trade name Petrelab^{®}. Other suitable LAB includes high 2-phenyl LAB, such as those supplied by Sasol under the trade name Hyblene^{®}.

Suitable anionic detersive surfactants are alkyl benzene sulfonates obtained by DETAL catalyzed process. Other synthesis routes, such as HF, may also be suitable. In one aspect, a magnesium salt of LAS is used.

The detersive surfactant may be a medium-chain branched detersive surfactant. In one aspect, the detersive surfactant may be a medium-chain branched anionic detersive surfactant. In one aspect, the detersive surfactant may be a medium-chain branched alkyl sulfate and/or a medium-chain branched alkylbenzene sulfonate having a chain derived from the polyalkylene oxide-containing compound of the present disclosure (e.g., a medium-chain branched alkyl sulfate). In one aspect, the medium-chain branch is a C1-C4 alkyl group, typically a methyl group and/or an ethyl group.

Other anionic surfactants useful in the present disclosure include water-soluble salts such as paraffin sulfonates and secondary alkane sulfonates containing from about 8 to about 24 (and in some examples, about 12 to 18) carbon atoms; alkyl glyceryl ether sulfonates, especially sulfonates of C8-C18 alcohol ethers (e.g., those derived from tallow and coconut oil). Mixtures of the alkylbenzene sulfonates with the above-described paraffin sulfonates, secondary alkane sulfonates, and alkyl glyceryl ether sulfonates are also useful. Further additional suitable anionic surfactants include methyl ester sulfonates and alkyl ether carboxylates. Further additional suitable anionic surfactants useful in the present disclosure may be found in U.S. Patent No. 4,285,841 (Barrat et al., issued on August 25, 1981) and in U.S. Patent No. 3,919,678 (Laughlin et al., issued on December 30, 1975), both of which are herein incorporated by reference.

The anionic surfactant may be present in an acid form, and the anionic surfactant in an acid form may be neutralized to form a surfactant salt. Typical agents for neutralization include a metal counter ion base such as a hydroxide (e.g., NaOH or KOH). Further preferred agents for neutralizing anionic surfactants in an acid form include ammonia, amines, or alkanolamines. Non-limiting examples of the alkanolamines include monoethanolamine, diethanolamine, triethanolamine, and other linear or branched alkanolamines known in the art. Preferred alkanolamines include 2-amino-1-propanol, 1-aminopropanol, monoisopropanolamine, and 1-amino-3-propanol. Amine neutralization may be performed to a full or partial extent. For example, a portion of an anionic surfactant mixture may be neutralized with sodium or potassium or a portion of the anionic surfactant mixture may be neutralized with amines or alkanolamines.

### <Nonionic surfactants>

In some embodiments, the detergent or cleaning composition of the present disclosure contains one or more nonionic surfactants. In a certain embodiment, the detergent or cleaning composition contains one or more nonionic surfactants in an amount from about 0.1% by weight to about 40% by weight, preferably about 0.2% by weight to about 15% by weight, more preferably about 0.3% by weight to about 10% by weight.

Nonionic surfactants useful in the present disclosure include any conventional nonionic surfactants. Examples of these include alkoxylated fatty alcohols and amine oxide surfactants. In some examples, the cleaning composition may contain an ethoxylated nonionic surfactant. These substances are described in U.S. Patent No. 4,285,841 (Barrat et al., issued on August 25, 1981). The nonionic surfactant may be selected from ethoxylated alcohols and ethoxylated alkylphenols each represented by the formula: R(OC₂H₄)ₙOH (where R is selected from the group consisting of an aliphatic hydrocarbon group having about 8 to about 15 carbon atoms and an alkylphenyl group containing an alkyl group having about 8 to about 12 carbon atoms; and the average value of n is about 5 to about 15). These surfactants are described in more detail in U.S. Patent No. 4,284,532 (Leikhim et al., issued on August 18, 1981). In one example, the nonionic surfactant is selected from ethoxylated alcohols having an average of about 24 carbon atoms in the alcohol and an average degree of ethoxylation of about 9 moles of ethylene oxide per mole of alcohol.

Other non-limiting examples of nonionic surfactants useful in the present disclosure include: C8-C18 alkyl ethoxylates (e.g., NEODOL^{®} nonionic surfactants available from Shell); C6-C12 alkyl phenol alkoxylates (where the alkoxylate units are ethyleneoxy units, propyleneoxy units, or a mixture thereof); C12-C18 alcohol and C6-C12 alkyl phenols condensates of ethylene oxide/propylene oxide block polymers (e.g., Pluronic^{®} available from BASF); C14-C22 medium-chain branched alcohols (BA) discussed in U.S. Patent No. 6,150,322; C14-C22 medium-chain branched alkyl alcoxylates BAEx (where x is 1 to 30) discussed in U.S. Patent No. 6,153,577, U.S. Patent No. 6,020,303, and U.S. Patent No. 6,093,856; alkylpolysaccharides discussed in U.S. Patent No. 4,565,647 (Llenado, issued on January 26, 1986), specifically alkylpolyglycosides discussed in U.S. Patent No. 4,483,780 and U.S. Patent No. 4,483,779; polyhydroxy fatty acid amides discussed in U.S. Patent No. 5,332,528, WO 92/06162, WO 93/19146, WO 93/19038, and WO 94/09099; and ether capped poly(oxyalkylated) alcohol surfactants discussed in U.S. Patent No. 6,482,994 and WO 01/42408.

Preferred nonionic detersive surfactants also include alkyl polyglucosides and alkyl alkoxylated alcohols. Preferred nonionic surfactants also include those marketed by BASF under the trade name Lutensol^{®}.

In some aspects, the nonionic surfactant is selected from alkyl alkoxylated alcohols including C8-C18 alkyl alkoxylated alcohols such as C8-C18 alkyl ethoxylated alcohols. The alkyl alkoxylated alcohols may have an average degree of alkoxylation from about 1 to about 50, or about 1 to about 30, or about 1 to about 20, or about 1 to about 10. In certain aspects, the alkyl alkoxylated alcohols are C8-C18 alkyl ethoxylated alcohols having an average degree of ethoxylation from about 1 to about 10, or about 1 to about 7, or about 1 to about 5, or about 3 to about 7. The alkyl alkoxylated alcohol may be linear or branched, substituted or unsubstituted.

### <Cationic surfactants>

In some embodiments, the detergent or cleaning composition of the present disclosure include one or more cationic surfactants.

In a certain aspect, the detergent or cleaning composition of the present disclosure contains one or more cationic surfactants in an amount from about 0.1% by weight to about 10% by weight, preferably about 0.2% by weight to about 7% by weight, more preferably about 0.3% by weight to about 5% by weight.

In another aspect, the detergent or cleaning composition of the present disclosure may be essentially free of cationic surfactants and surfactants that are cationic at a pH of less than 7 or a pH of less than 6.

Non-limiting examples of cationic surfactants include quaternary ammonium surfactants which can have up to 26 carbon atoms. The quaternary ammonium surfactants include: alkoxylate quaternary ammonium (AQA) surfactants discussed in U.S. Patent No. 6,136,769; dimethyl hydroxyethyl quaternary ammonium discussed in U.S. Patent No. 6,004,922; dimethyl hydroxyethyl lauryl ammonium chloride; polyamine cationic surfactants discussed in WO 98/35002, WO 98/35003, WO 98/35004, WO 98/35005, and WO 98/35006; cationic ester surfactants discussed in U.S. Patent No. 4,228,042, U.S. Patent No. 4,239,660, U.S. Patent No. 4,260,529, and U.S. Patent No. 6,022,844; and amino surfactants, specifically amidopropyl dimethyl amine (APA), discussed in U.S. Patent No. 6,221,825 and WO 00/47708.

Preferred cationic detersive surfactants also include alkylpyridinium compounds, alkyl quaternary ammonium compounds, alkyl quaternary phosphonium compounds, alkyl tertiary sulfonium compounds, and mixtures thereof.

Preferred cationic detersive surfactants are quaternary ammonium compounds having the following formula: (R) (R^{a})(R^{b})(R^{c})N⁺X⁻
where R is a linear or branched, substituted or unsubstituted C6-C18 alkyl or alkenyl moiety; R^{a} and R^{b} are independently selected from methyl and ethyl moieties; R^{c} is a hydroxy, hydroxymethyl, or hydroxyethyl moiety; and X is an anion that provides neutrality of electric charge, with the anion being preferably a halide (e.g., chloride), a sulfate, or a sulfonate. Preferred cationic detersive surfactants include a mono-C6-C18 alkyl mono-hydroxyethyl dimethyl quaternary ammonium chloride. Highly preferred cationic detersive surfactants include a mono-C8-C10 alkyl mono-hydroxyethyl dimethyl quaternary ammonium chloride, a mono-C10-C12 alkyl mono-hydroxyethyl dimethyl quaternary ammonium chloride, and a mono-C10 alkyl mono-hydroxyethyl dimethyl quaternary ammonium chloride.

### <Zwitterionic surfactants>

Examples of zwitterionic surfactants include secondary amine derivatives, tertiary amine derivatives, heterocyclic secondary amine derivatives, heterocyclic tertiary amine derivatives, quaternary ammonium compound derivatives, quaternary phosphonium compound derivatives, and tertiary sulfonium compound derivatives. Zwitterionic surfactants include betaines such as alkyldimethyl betaines, coconut dimethylamidopropyl betaines, and C8-C18 (e.g., C12-C18) amine oxide and sulfo and hydroxy betaines (e.g., N-alkyl-N,N-dimethylamino-1-propanesulfonic acid (where the alkyl group can be a C8-C18 alkyl group, and in a certain embodiment, the alkyl group can be a C10-C14 alkyl group)). As for such betaines, see U.S. Patent No. 3,929,678, column 19, line 38 to column 22, line 48.

### <Ampholytic surfactants>

Examples of amphoteric surfactants include aliphatic derivatives in which the aliphatic groups may be linear or branched chains, at least one of the aliphatic substituents has at least about 8 carbon atoms, typically about 8 to about 18 carbon atoms, and at least one of the aliphatic substituents is an anionic water-soluble group such as a heterocyclic secondary or tertiary amine containing carboxy, sulfonate, or sulfate. Examples of compounds falling within the range of this definition are sodium 3-(dodecylamino)propionate, sodium 3-(dodecylamino)propane-1-sulfonate, sodium 2-(dodecylamino)ethyl sulfate, sodium 2-(dimethylamino)octadecanoate, disodium 3-(N-carboxymethyldodecylamino)propane 1-sulfonate, disodium octadecyl-iminodiacetate, sodium 1-carboxymethyl-2-undecylimidazole, and sodium N,N-bis(2-hydroxyethyl)-2-sulfato-3-dodecoxypropylamine. As for examples of amphoteric surfactants, see U.S. Patent No. 3,929,678 (Laughlin et al., issued on December 30, 1975), column 19, lines 18-35. Examples of preferred amphoteric surfactants also include sarcosinates, glycosinates, taurinates, and mixtures thereof.

### <Branched surfactants>

In some examples, the detergent or cleaning composition of the present disclosure contains one or more branched surfactants. Preferred branched surfactants include anionic branched surfactants selected from branched sulfate surfactants and branched sulfonate surfactants (e.g., branched alkyl sulfates, branched alkyl alkoxylated sulfates, and branched alkyl benzene sulfonates), containing one or more random alkyl branches (e.g., C1-C4 alkyl groups, typically methyl groups and/or ethyl groups).

In some aspects, the branched detersive surfactant may be a medium-chain branched detersive surfactant, typically a medium-chain branched anionic detersive surfactant such as a medium-chain branched alkyl sulfate and/or a medium-chain branched alkyl benzene sulfonate. In some aspects, the detersive surfactant is a medium-chain branched alkyl sulfate. In some aspects, the medium-chain branches are C1-C4 alkyl groups, typically methyl groups and/or ethyl groups.

In some aspects, the branched surfactants include medium-chain branched surfactant compounds having longer alkyl chains represented by the formula: A_{b}-X-B.

In the formula,
(a) A_{b} is a hydrophobic C9-C22 (total carbons in the moiety) medium-chain branched alkyl moiety, typically a medium-chain branched alkyl moiety having about 12 to about 18 carbon atoms. A_{b} has (1) a longest linear carbon chain attached to the -X-B moiety having from 8 to 21 carbon atoms and (2) one or more C1-C3 alkyl moieties branching from the longest linear carbon chain; (3) at least one of the branching alkyl moieties is attached directly to a carbon of the longest linear carbon chain at a position within the range of position 2 carbon (counting from position 1 carbon which is attached to the -X-B moiety) to position ω-2 carbon (the carbon at the terminal carbon minus 2 carbons, i.e., the third carbon from the end of the longest linear carbon chain); and (4) the surfactant composition contains the A_{b}-X moiety having an average total carbon atom number from 14.5 to about 17.5 (typically, about 15 to about 17),
(b) B is a hydrophobic moiety selected from sulfates, sulfonates, amine oxides, polyoxyalkylenes (e.g., polyoxyethylene and polyoxypropylene), alkoxylated sulfates, polyhydroxy moieties, phosphate esters, glycerol sulfonates, polygluconates, polyphosphate esters, phosphonates, sulfosuccinates, sulfosuccaminates, polyalkoxylated carboxylates, glucamides, taurinates, sarcosinates, glycinates, isethionates, dialkanolamides, monoalkanolamides, monoalkanolamide sulfates, diglycolamides, diglycolamide sulfates, glycerol esters, glycerol ester sulfates, glycerol ethers, glycerol ether sulfates, polyglycerol ethers, polyglycerol ether sulfates, sorbitan esters, polyalkoxylated sorbitan esters, ammonioalkanesulfonates, amidopropyl betaines, alkylated quats, alkyated/polyhydroxyalkylated quats, alkylated/polyhydroxylated oxypropyl quats, imidazolines, 2-yl-succinates, sulfonated alkyl esters, and sulfonated fatty acids (for example, in (A_{b}-X)_{z}-B, one or more hydrophobic moieties may be attached to B to provide dimethyl quats); and
(c) X is selected from -CH₂- and -C(O)-.

Generally, the A_{b} moiety in the above formula does not have any quaternary substituted carbon atoms (i.e., the four carbon atoms are directly bonded to one carbon atom). Depending on the selected hydrophilic moiety (B), the resulting surfactant may be anionic, nonionic, cationic, zwitterionic, or amphoteric, or may be an ampholyte. In some aspects, B is a sulfate and the resulting surfactant is anionic.

In some aspects, the branched surfactants include medium-chain branched surfactant compounds having longer alkyl chains represented by the above formula. The A_{b} moiety in the formula is a branched primary alkyl moiety represented by the formula:

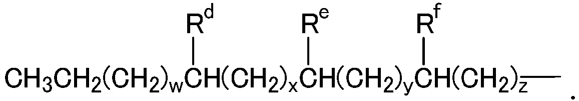

The total number of carbon atoms in the branched primary alkyl moieties (including R^{d}, R^{e}, and R^{f} branches) represented by the formula is 13 to 19. Provided that R^{d}, R^{e}, and R^{f} are not hydrogen, R^{d}, R^{e}, and R^{f} are each independently selected from hydrogen and C1-C3 alkyl (typically methyl). When z is 0, at least R^{d} or R^{e} is not hydrogen. w is an integer from 0 to 13, x is an integer from 0 to 13, y is an integer from 0 to 13, z is an integer from 0 to 13, and w + x + y + z is 7 to 13.

In a certain aspect, the branched surfactants include medium-chain branched surfactant compounds having longer alkyl chains represented by the above formula. The A_{b} moiety is a branched primary alkyl moiety selected from the moieties represented by the following formulas: 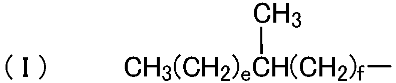 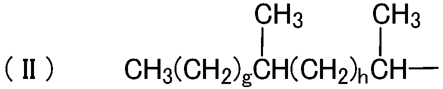 and the combination of these, where e, f, g, and h are integers, e + f is 10 to 16, and g + h is 8 to 14.
When e + f = 10, e is an integer from 2 to 9 and f is an integer from 1 to 8;
when e + f = 11, e is an integer from 2 to 10 and f is an integer from 1 to 9;
when e + f = 12, e is an integer from 2 to 11 and f is an integer from 1 to 10;
when e + f = 13, e is an integer from 2 to 12 and f is an integer from 1 to 11;
when e + f = 14, e is an integer from 2 to 13 and f is an integer from 1 to 12;
when e + f = 15, e is an integer from 2 to 14 and f is an integer from 1 to 13;
when e + f = 16, e is an integer from 2 to 15 and f is an integer from 1 to 14;
when g + h = 8, g is an integer from 2 to 7 and h is an integer from 1 to 6;
when g + h = 9, g is an integer from 2 to 8 and h is an integer from 1 to 7;
when g + h = 10, g is an integer from 2 to 9 and h is an integer from 1 to 8;
when g + h = 11, g is an integer from 2 to 10 and h is an integer from 1 to 9;
when g + h = 12, g is an integer from 2 to 11 and h is an integer from 1 to 10;
when g + h = 13, g is an integer from 2 to 12 and h is an integer from 1 to 11; and
when g + h = 14, g is an integer from 2 to 13 and h is an integer from 1 to 12.

In the medium-chain branched surfactant compounds described above, certain branching points (e.g., the locations along the chains of the R^{d}, R^{e}, and/or R^{f} moieties in the above formula) are better than other branching points along the backbone of the surfactant. The following formula illustrates the medium-chain branching range (i.e., where branching points occur), preferred medium-chain branching range, and more preferred medium-chain branching range for a mono-methyl branched alkyl A_{b} moiety.

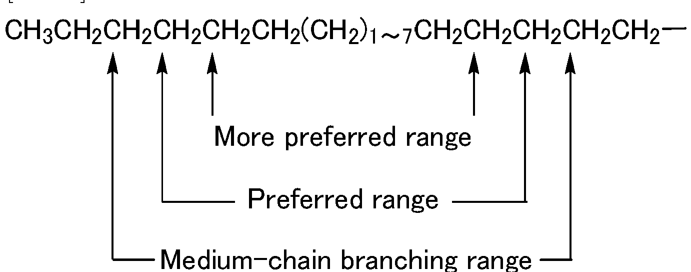

For a mono-methyl substituted surfactant, these ranges exclude the two terminal carbon atoms of the chain and the carbon atom immediately adjacent to the -X-B group.

The following formula illustrates the medium-chain branching range, preferred medium-chain branching range, and more preferred medium-chain branching range for a dimethyl substituted linear alkyl A_{b} moiety.

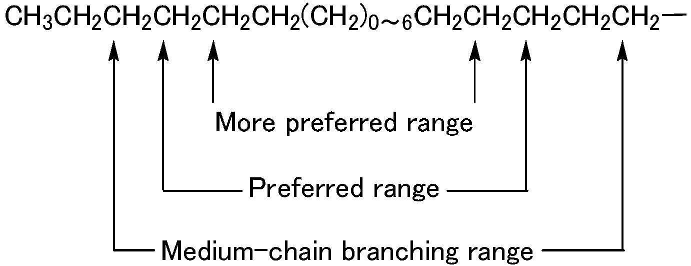

Additional suitable branched surfactants include those described in U.S. Patent No. 6008181, U.S. Patent No. 6060443, U.S. Patent No. 6020303, U.S. Patent No. 6153577, U.S. Patent No. 6093856, U.S. Patent No. 6015781, U.S. Patent No.6133222, U.S. Patent No.6326348, U.S. Patent No. 6482789, U.S. Patent No. 6677289, U.S. Patent No. 6903059, U.S. Patent No. 6660711, U.S. Patent No. 6335312, and WO 9918929. Still other suitable branched surfactants include those described in WO 9738956, WO 9738957, and WO 0102451.

In some aspects, the branched anionic surfactants include branched modified alkylbenzene sulfonates (MLAS), which are discussed in WO 99/05243, WO 99/05242, WO 99/05244, WO 99/05082, WO 99/05084, WO 99/05241, WO 99/07656, WO 00/23549, and WO 00/23548.

In some aspects, the branched anionic surfactants include a C12/13 alcohol-based surfactant containing methyl branches randomly distributed along the hydrophobic chain (e.g., Safol^{®} and Marlipal^{®} available from Sasol).

Other suitable branched surfactants include those described in U.S. Patent No. 6037313 (P&G), WO 9521233 (P&G), U.S. Patent No. 3480556 (Atlantic Richfield), U.S. Patent No. 6683224 (Cognis), US 20030225304 A1 (Kao), US 2004236158 A1 (R&H), U.S. Patent No. 6818700 (Atofina), US 2004154640 (Smith et al.), European Patent No. 1280746 (Shell), European Patent No. 1025839 (L'Oreal), U.S. Patent No. 6765119 (BASF), European Patent No. 1080084 (Dow), U.S. Patent No. 6723867 (Cognis), EP 1401792 A1 (Shell), EP 1401797 A2 (Degussa AG), US 2004048766 (Raths et al.), U.S. Patent No. 6596675 (L'Oreal), European Patent No. 1136471 (Kao), European Patent No. 961765 (Albemarle), U.S. Patent No. 6580009 (BASF), US 2003105352 (Dado et al.), U.S. Patent No. 6573345 (Cryovac), DE 10155520 (BASF), U.S. Patent No. 6534691 (duPont), U.S. Patent No. 6407279 (ExxonMobil), U.S. Patent No. 5831134 (Peroxid-Chemie), U.S. Patent No. 5811617 (Amoco), U.S. Patent No. 5463143 (Shell), U.S. Patent No. 5304675 (Mobil), U.S. Patent No. 5227544 (BASF), U.S. Patent No. 5446213 A (MITSUBISHI KASEI CORPORATION), EP 1230200 A2 (BASF), European Patent No. 1159237 B1 (BASF), US 20040006250 A1 (NONE), European Patent No. 1230200 B1 (BASF), WO 2004014826 A1 (SHELL), U.S. Patent No. 6703535 B2 (CHEVRON), European Patent No. 1140741 B1 (BASF), WO 2003095402 A1 (OXENO), U.S. Patent No. 6765106 B2 (SHELL), US 20040167355 A1 (NONE), U.S. Patent No. 6700027 B1 (CHEVRON), U.S. Patent No. 20040242946 A1 (NONE), WO 2005037751 A2 (SHELL), WO 2005037752 A1 (SHELL), U.S. Patent No. 6906230 B1 (BASF), and WO 2005037747 A2 (SHELL OIL COMPANY).

Additional suitable branched anionic detersive surfactants include surfactant derivatives of isoprenoid-based polybranched detergent alcohols as described in US 2010/0137649. Isoprenoid-based surfactants and isoprenoid derivatives are also described in the book entitled "Comprehensive Natural Products Chemistry: Isoprenoids Including Carotenoids and Steroids (Vol. two)", Barton and Nakanishi, Copyright 1999, Elsevier Science Ltd and are included in the structure E, and are hereby incorporated by reference.

Further additional suitable branched anionic detersive surfactants include those derived from anteiso and iso-alcohols. Such surfactants are disclosed in WO 2012009525.

Further additional suitable branched anionic detersive surfactants include those described in US 2011/0171155 (A1) and US 2011/0166370 (A1).

Suitable branched anionic surfactants also include Guerbet-alcohol-based surfactants. Guerbet alcohols are branched primary monofunctional alcohols that have two linear carbon chains with the branching point always at the second carbon position. Guerbet alcohols are chemically described as 2-alkyl-1-alkanols. Guerbet alcohols generally have 12 to 36 carbon atoms. The Guerbet alcohols may be represented by the formula: (R^{g})(R^{h})CHCH₂OH, where R^{g} is a linear alkyl group, R^{h} is a linear alkyl group, the sum of the carbon atoms in R^{g} and R^{h} is 10 to 34, and both R^{g} and R^{h} are present. Guerbet alcohols are commercially available under the trade name Isofol^{®} alcohols from Sasol and under the trade name Guerbetol from Cognis.

The branched surfactants may contain bio-based materials. In some aspects, the branched surfactants contain bio-based materials in an amount of at least about 50%, at least about 60%, at least about 70%, at least about 80%, at least about 90%, at least about 95%, at least about 97%, or about 100%.

### <Combination of surfactants>

In some aspects, the detergent or cleaning composition of the present disclosure may contain an anionic surfactant and a nonionic surfactant such as a C12-C18 alkyl ethoxylate. In another aspect, the detergent or cleaning composition of the present disclosure may contain a C10-C15 alkyl benzene sulfonate (LAS) and another anionic surfactant such as a C10-C18 alkyl alkoxy sulfate (AExS) (where x is 1 to 30). In a certain aspect, the detergent or cleaning composition of the present disclosure may contain an anionic surfactant and a cationic surfactant such as dimethyl hydroxyethyl lauryl ammonium chloride. In another aspect, the detergent or cleaning composition of the present disclosure may contain an anionic surfactant and a zwitterionic surfactant such as a C12-C14 dimethyl amine oxide.

When the detergent or cleaning composition of the present disclosure contains a combination of an anionic surfactant material and a nonionic surfactant material, the weight ratio of anionic surfactant to nonionic surfactant is at least about 1.5:1, more preferably at least about 2:1 or 5:1 or 25:1, most preferably at least about 100:1.

### <Cleaning adjunct additives>

The detergent or cleaning composition of the present disclosure may further contain a cleaning adjunct additive. Suitable cleaning adjunct additives include builders, surfactants or thickeners, clay-soil removal or clay-soil anti-redeposition agents, stain release polymers, dispersant polymers, grease cleaning polymers, enzymes, enzyme stabilizing systems, bleaching compounds, bleaching agents, bleach activators, bleach catalysts, brighteners, dyes, hueing agents, dye transfer inhibitors, chelating agents, defoamers, softeners, fragrances, and mixtures of these.

### <Enzymes>

The detergent or cleaning composition of the present disclosure may contain one or more enzymes which provide cleaning performance and/or fabric care benefits. Non-limiting examples of suitable enzymes include hemicellulases, peroxidases, proteases, cellulases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, β-glucanases, arabinosidases, hyaluronidases, chondroitinases, laccases, and amylases, and mixtures thereof. A typical combination is an enzyme cocktail that may contain, for example, protease and lipase in combination with amylase.

When present in the detergent or cleaning composition, additional enzymes may be present at concentrations enzyme protein from about 0.00001% by weight to about 2% by weight, from about 0.0001% by weight to about 1% by weight, or from about 0.001% by weight to about 0.5% by weight.

In one aspect, preferred enzymes include proteases. Suitable proteases include metalloproteases and serine proteases including neutral or alkaline microbial serine proteases such as subtilisins (EC 3.4.21.62). Suitable proteases include those of animal, vegetable, or microbial origin. In one aspect, such suitable protease may be of microbial origin. The suitable proteases include chemically or genetically modified mutants of the aforementioned suitable proteases. In one aspect, the suitable protease may be a serine protease such as an alkaline microbial protease and/or a trypsin-type protease. Examples of suitable neutral or alkaline proteases are as follows:
(a) subtilisins (EC 3.4.21.62) including those derived from *Bacillus* such as *Bacillus lentus, B. alkalophilus, B. subtilis, B. amyloliquefaciens, Bacillus pumilus,* and *Bacillus gibsonii,* described in U.S. Patent No. 6,312,936 B1, U.S. Patent No. 5,679,630, U.S. Patent No. 4,760,025, U.S. Patent No. 7,262,042, and WO 09/021867;
(b) trypsin-type or chymotrypsin-type proteases such as trypsin (e.g., of porcine or bovine origin), including the *Fusarium* protease described in WO 89/06270 and the chymotrypsin proteases derived from *Cellumonas* described in WO 05/052161 and WO 05/052146; and
(c) metalloproteases including those derived from *Bacillus amyloliquefaciens* described in WO 07/044993.

Preferred proteases include those derived from *Bacillus gibsonii* or *Bacillus lentus.*

Suitable commercially available protease enzymes include those marketed under the trade names Alcalase^{®}, Savinase^{®}, Primase^{®}, Durazym^{®}, Polarzyme^{®}, Kannase^{®}, Liquanase^{®}, Liquanase Ultra^{®}, Savinase Ultra^{®}, Ovozyme^{®}, Neutrase^{®}, Everlase^{®}, and Esperase^{®} by Novozymes A/S (Denmark), those marketed under the trade name Maxatase^{®}, Maxacal^{®}, Maxapem^{®}, Properase^{®}, Purafect^{®}, Purafect Prime^{®}, Purafect Ox^{®}, FN3^{®}, FN4^{®}, Excellase^{®}, and Purafect OXP^{®} by Genencor International, those marketed under the trade name Opticlean^{®} and Optimase^{®} by Solvay Enzymes, those available from Henkel/Kemira, namely BLAP (the sequence with the mutations S99D + S101 R + S103A + V104I + G159S, shown in FIG. 29 in U.S. Patent No. 5,352,604, hereinafter referred to as BLAP), BLAP R (BLAP with S3T + V4I + V199M + V205I + L217D), BLAP X (BLAP with S3T + V4I + V205I), and BLAP F49 (BLAP with S3T + V4I + A194P + V199M + V205I + L217D) (all available from Henkel/Kemira); and KAP *(Bacillus alkalophilus* subtilisin with mutations A230V + S256G + S259N) from Kao.

Suitable α-amylases include those of bacterial or fungal origin. Chemically or genetically modified mutants (variants) are included. A preferred alkaline α-amylase is derived from *Bacillus* sp. such as *Bacillus licheniformis, Bacillus amyloliquefaciens, Bacillus stearothermophilus, Bacillus subtilis,* or other *Bacillus* sp. (e.g., *Bacillus* sp. NCIB 12289, NCIB 12512, NCIB 12513, DSM 9375 (U.S. Patent No. 7,153,818), DSM 12368, DSMZ no. 12649, KSM AP1378 (WO 97/00324), and KSM K36 or KSM K38 (European Patent No. 1,022,334). Preferred amylases are described as follows:
(a) the variants described in WO 94/02597, WO 94/18314, WO 96/23874, and WO 97/43424, especially variants with substitutions in one or more of the positions: 15, 23, 105, 106, 124, 128, 133, 154, 156, 181, 188, 190, 197, 202, 208, 209, 243, 264, 304, 305, 391, 408, and 444 relative to enzyme listed as SEQ ID No. 2 in WO 96/23874;
(b) the variants described in U.S. Patent No. 5,856,164, WO 99/23211, WO 96/23873, WO 00/60060, and WO 06/002643, especially variants with substitutions in one or more of the positions: 26, 30, 33, 82, 37, 106, 118, 128, 133, 149, 150, 160, 178, 182, 186, 193, 203, 214, 231, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, and 484 versus the AA560 enzyme listed as SEQ ID No. 12 in WO 06/002643, with the variants preferably having deletions of D183* and G184*;
(c) variants exhibiting at least 90% identity with the wild-type enzyme from *Bacillus* SP722 (SEQ ID No. 4 in WO 06/002643), especially variants with deletions in the 183 and 184 positions and variants described in WO 00/60060, which are incorporated herein by reference;
(d) variants exhibiting at least 95% identity with the wild-type enzyme from *Bacillus* sp. 707 (SEQ ID NO:7 in U.S. Patent No. 6,093,562), especially those containing one or more of the mutations M202, M208, S255, R172, and M261, with the amylase preferably containing one or more of M202L, M202V, M202S, M202T, M202I, M202Q, M202W, S255N and R172Q, with those containing the M202L or M202T mutations being particularly preferred; and
(e) variants described in WO 09/149130, preferably those exhibiting at least 90% identity with SEQ ID NO: 1 or SEQ ID NO:2 in WO 09/149130, the wild-type enzyme from *Geobacilles Stearophermophilus* or a truncated variant thereof.

Suitable commercially available α-amylases include DURAMYL^{®}, LIQUEZYME^{®}, TERMAMYL^{®}, TERM AM YL ULTRA^{®}, NATALASE^{®}, SUPRAMYL^{®}, STAINZYME^{®}, STAINZYME PLUS^{®}, FUNGAMYL^{®}, and BAN^{®} (Novozymes A/S, Bisgaard, Denmark), KEMZYM^{®} AT 9000 (Boozy Biotech Trading GmbH (Elastase 27b A-1200 Wien Austria)), RAPIDASE^{®}, PURASTAR^{®}, ENZYSIZE^{®}, OPTISIZE HT PLUS^{®}, POWERASE^{®}, and PURASTAR OXAM^{®} (Genesco International Inc., Palo Alto, California) and KAM^{®} (Kao, 14-10 Nihonbashi Karabachos, 1-chome, Chuo-ku Tokyo 103-8210, Japan). In one aspect, suitable amylases include NATALASE^{®}, STAINZYME^{®}, STAINZYME PLUS^{®}, and mixtures thereof.

In one aspect, such enzymes may be selected from the group consisting of lipases including "first cycle lipases" such as those described in U.S. Patent No. 6,939,702 B1 and US 2009/0217464. In one aspect, the lipase is a first-wash lipase, preferably a variant of the wild-type lipase from *Thermomyces lanuginosus* containing the T231R and N233R mutations. The wild-type sequence is the 269 amino acids (amino acids 23-291) of the Swissport accession number Swiss-Prot 059952 *(Thermomyces lanuginosus* (derived from *Humicola lanuginosa)).* Preferred lipases include those marketed under the trade names Lipez^{®} and Lipolex^{®}.

In one aspect, other preferred enzymes include microbial-derived endoglucanases exhibiting endo-β-1,4-glucanase activity (E.C. 3.2.1.4), including a bacterial polypeptide endogenous to a member of the genus *Bacillus* which has a sequence of at least 90%, preferably 94%, more preferably 97%, even more preferably 99% identity to the amino acid sequence SEQ ID NO:2 in U.S. Patent No. 7,141,403 B2 and mixtures thereof. Suitable endoglucanases are marketed under the trade names Celluclean^{®} and Whitezyme^{®} (Novozymes A/S, Bagsvaerd, Denmark).

Other preferred enzymes include pectate lyases marketed under the trade names Petawatt^{®}, Pectaway^{®}, and Xpect^{®} and mannanases marketed under the trade names Mannaway^{®} (all from Novozymes A/S, Bisgaard, Denmark) and Purabrite^{®} (Genencor International Inc., Palo Alto, California).

### <Enzyme stabilizing systems>

The enzyme-containing compositions described in the present disclosure may optionally contain an enzyme stabilizing system in an amount from about 0.001% by weight to about 10% by weight, in some examples from about 0.005% by weight to about 8% by weight, in other examples from about 0.01% by weight to about 6% by weight, of the composition. The enzyme stabilizing system can be any stabilizing system which is compatible with the detersive enzyme. Such a system may be inherently provided by other formulation active substances, or may be added separately by, for example, the formulator or a manufacturer of enzymes for detergents. Such stabilizing systems can, for example, contain calcium ion, boric acid, propylene glycol, short chain carboxylic acids, boronic acids, chlorine bleach scavengers, and mixtures thereof, and are designed to address different stabilization problems depending on the type and physical form of the cleaning composition. See U.S. Patent No. 4,605,783 for a review of borate stabilizers. In the case of aqueous detergent or cleaning compositions containing protease, reversible protease inhibitors such as a boron compound containing borate, 4-formyl phenylboronic acid, phenylboronic acid, or a derivative thereof, or compounds such as calcium formate, sodium formate, and 1,2-propane diol may be added to further improve stability.

### <Builders>

The detergent or cleaning composition of the present disclosure may optionally contain a builder in addition to the above-described sulfonic acid group-containing copolymer. Builder-containing cleaning compositions typically contain at least about 1% by weight of a builder, based on the total weight of the composition. Liquid cleaning compositions may contain a builder in an amount of up to about 10% by weight, in some examples up to about 8% by weight, based on the total weight of the composition. Granular cleaning compositions may contain a builder in an amount of up to about 30% by weight, in some examples up to about 5% by weight, based on the total weight of the composition.

Builders selected from aluminosilicates (e.g. zeolite builders such as zeolite A, zeolite P, and zeolite MAP) and silicates assist in controlling mineral hardness in cleaning water, especially calcium and/or magnesium, or assist in removing particulate soils from surfaces. Suitable builders may be selected from the group consisting of phosphates such as polyphosphates (e.g., sodium tripolyphosphate), especially sodium salts thereof; carbonates other than sodium carbonate and sodium sesquicarbonate, bicarbonates, sesquicarbonates, and carbonate minerals; organic mono-, di-, tri-, and tetracarboxylates, especially water-soluble nonsurfactant carboxylates in acid, sodium, potassium, or alkanolammonium salt form, as well as aliphatic or aromatic water-soluble low molecular polymer carboxylates; and phytic acid. These may be complemented, for example, for pH-buffering purposes, by borates, sulfates, especially sodium sulfate or any other fillers or carriers which may be important for the mass production of stable surfactants and/or builder-containing cleaning compositions.

Additional suitable detergent builders may be selected from citric acid, lactic acid, fatty acids, polycarboxylate builders, for example, copolymers of acrylic acid, copolymers of acrylic acid and maleic acid, copolymers of acrylic acid and/or maleic acid, and other suitable ethylenic monomers with various types of additional functional groups. Also suitable builders for use as builders in the present disclosure are synthesized crystalline ion exchange materials or hydrates thereof having a chain structure and a composition represented by the general anhydride form: x(M₂O)·ySiO₂·zM'O. In the formula, M is Na and/or K, M' is Ca and/or Mg; y/x is 0.5 to 2.0; and z/x is 0.005 to 1.0 as taught in U.S. Patent No. 5,427,711.

Preferably, the detergent or cleaning composition of the present disclosure is essentially free of other builders, especially inorganic builders, specifically zeolite builders and phosphate builders.

### <Structurants/Thickeners>

When the detergent or cleaning composition of the present disclosure is in the form of liquid and is, for example, a liquid laundry detergent composition, the detergent or cleaning composition may contain a dibenzylidene polyol acetal derivative (DBPA) in an amount from about 0.01% by weight to about 1% by weight, or from about 0.05% by weight to about 0.8% by weight, or from about 0.1% by weight to about 0.6% by weight, or from about 0.3% by weight to about 0.5% by weight. Non-limiting examples of suitable DBPA molecules are disclosed in U.S. Patent No. 61/167604. In one aspect, the DBPA derivative may include a dibenzylidene sorbitol acetal derivative (DBS). The DBS derivative may be selected from the group consisting of 1,3:2,4-dibenzylidene sorbitol; 1,3:2,4-di(p-methylbenzylidene) sorbitol; 1,3:2,4-di(p-chlorobenzylidene) sorbitol; 1,3:2,4-di(2,4-dimethyldibenzylidene) sorbitol; 1,3:2,4-di(p-ethylbenzylidene) sorbitol; and 1,3:2,4-di(3,4-dimethyldibenzylidene) sorbitol, and mixtures thereof. These and other suitable DBS derivatives are disclosed in U.S. Patent No. 6,102,999, column 2, line 43 to column 3, line 65.

The liquid laundry detergent composition of the present disclosure may further contain a bacterial cellulose network in an amount from about 0.005% by weight to about 1% by weight. The term "bacterial cellulose" encompasses any type of cellulose produced via fermentation of a bacteria of the genus Acetobacter such as CELLULON^{®} by CP Kelco U.S. and materials referred to generally as microfibrillated cellulose, reticulated bacterial cellulose, and the like. Some examples of suitable bacterial cellulose can be found in U.S. Patent No. 6,967,027, U.S. Patent No. 5,207,826, U.S. Patent No. 4,487,634, U.S. Patent No. 4,373,702, U.S. Patent No. 4,863,565, and US 2007/0027108.

In one aspect, the fibers have cross sectional dimensions of 1.6 nm to 3.2 nm by 5.8 nm to 133 nm. Additionally, the bacterial cellulose fibers have an average microfiber length of at least about 100 nm, or from about 100 to about 1,500 nm.

In one aspect, the bacterial cellulose microfibers have an aspect ratio, which is obtained by dividing the average microfiber length by the widest cross sectional microfiber width, from about 100:1 to about 400:1, or from about 200:1 to about 300:1.

In one aspect, the bacterial cellulose is at least partially coated with a polymeric thickener. The at least partially coated bacterial cellulose can be prepared in accordance with the methods disclosed in US 2007/0027108, columns 8 to 19.

In one aspect, the at least partially coated bacterial cellulose contains bacterial cellulose in an amount from about 0.1% by weight to about 5% by weight, or from about 0.5% by weight to about 3% by weight; and a polymeric thickener in an amount from about 10% by weight to about 90% by weight.

Suitable bacterial cellulose may include the bacterial cellulose described above. Suitable polymeric thickeners include carboxymethylcellulose, cationic hydroxymethylcellulose, and mixtures thereof.

In one aspect, the composition may further contain a cellulosic fiber in an amount from about 0.01% by weight to about 5% by weight of the composition. The cellulosic fiber may be extracted from vegetables, fruits, or wood. Commercially available examples are Avicel^{®} from FMC, Citri-Fi from Fiberstar, or Betafib from Cosun.

In one aspect, the composition may further contain a non-polymeric crystalline hydroxyl functional structurant in an amount from about 0.01% by weight to about 1% by weight of the composition.

The non-polymeric crystalline hydroxyl functional structurant may generally contain a crystallizable glyceride which can be pre-emulsified to aid dispersion into the final liquid detergent or cleaning composition. In one aspect, crystallizable glycerides may include hydrogenated castor oil or "HCO" or derivatives thereof, provided that it can crystalize in the liquid detergent or cleaning composition.

The liquid detergent or cleaning composition of the present disclosure may contain a naturally derived and/or synthetic polymeric structurant in an amount from about 0.01% by weight to about 5% by weight.

Examples of naturally derived polymeric structurants used in the present disclosure include hydroxyethyl cellulose, hydrophobically modified hydroxyethyl cellulose, carboxymethyl cellulose, polysaccharide derivatives, and mixtures thereof. Suitable polysaccharide derivatives include pectin, alginate, arabinogalactan (gum Arabic), carrageenan, gellan gum, xanthan gum, guar gum, and mixtures thereof.

Examples of synthetic polymeric structurants used in the present disclosure include polycarboxylates, polyacrylates, hydrophobically modified ethoxylated urethanes, hydrophobically modified non-ionic polyols, and mixtures thereof. In one aspect, the polycarboxylate polymer is polyacrylate, polymethacrylate, or a mixture thereof. In another aspect, the polyacrylate is a copolymer of unsaturated mono- or di-carbonic acid and C1-C30 alkyl ester of (meth)acrylic acid. The copolymer is available from Noveon inc. under the brand name Carbopol Aqua 30.

In one aspect, the external structuring system may contain a diamide gellant having a molecular weight from about 150 g/mol to about 1,500 g/mol, or from about 500 g/mol to about 900 g/mol.

Such a diamide gellant may contain at least two nitrogen atoms, where at least two of the nitrogen atoms form amide functional substituents. In one aspect, the amide groups are different from each other. In another aspect, the amide functional groups are the same as each other. The diamide gellant has the following formula: 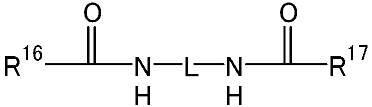 where:
R¹⁶ and R¹⁷ are each an amino functional end group or an amide functional end group. In one aspect, R¹⁶ and R¹⁷ may contain a pH adjustable group. A pH adjustable amide-gellant may have a pKa from about 1 to about 30, or from about 2 to about 10. In one aspect, the pH adjustable group may contain pyridine. In one aspect, R¹⁶ and R¹⁷ may be different from each other. In another aspect, R¹⁶ and
R¹⁷ may be the same as each other.

L is a linking moiety having a molecular weight from 14 to 500 g/mol. In one aspect, L may contain a carbon chain containing 2 to 20 carbon atoms. In another aspect, L may contain a pH-adjustable group. In one aspect, the pH adjustable group is a secondary amine.

In one aspect, at least one of R¹⁶, R¹⁷, or L may contain a pH-adjustable group.

Non-limiting examples of diamide gellant include: N,N'-(2S,2'S)-1,1'-(dodecane-1,12-diylbis(azanediyl))bis(3-methyl-1-oxobutane-2,1-diyl)diisonicotinamide, the compound represented by 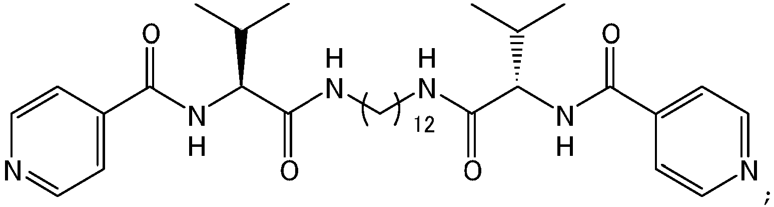 dibenzyl (2S,2'S)-1,1'-(propane-1,3-diylbis(azanediyl))bis(3-methyl-1-oxobutane-2,1-diyl)dicarbamate, the compound represented by 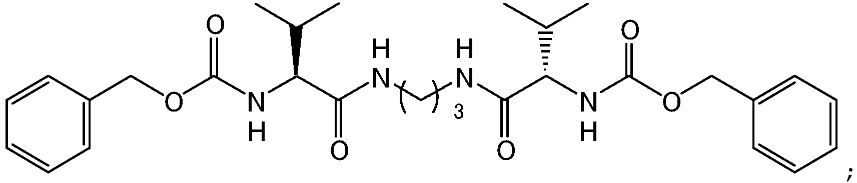 and dibenzyl (2S,2'S)-1,1'-(dodecane-1,12-diylbis(azanediyl))bis(1-oxo-3-phenylpropane-2,1-diyl)dicarbamate, the compound represented by

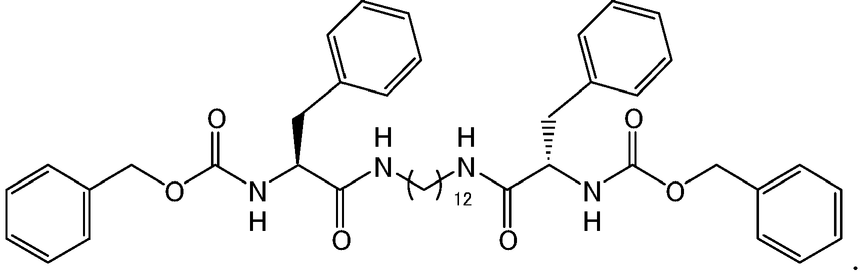

### <Dispersant polymers>

The detergent or cleaning composition may contain one or more dispersant polymers. Examples include carboxymethylcellulose, poly(vinylpyrrolidone), poly(ethylene glycol), poly(vinyl alcohol), poly(vinylpyridine-N-oxide), poly(vinylimidazole), polycarboxylates such as polyacrylates, maleic acid/acrylic acid copolymers, and lauryl methacrylate/acrylic acid copolymers.

The detergent or cleaning composition may contain one or more amphiphilic cleaning polymers such as a compound having the following structure: bis((C₂H₅O)(C₂H₄O)ₙ)(CH₃)-N⁺-CₓH₂ₓ-N⁺-(CH₃)-bis((C₂H₅O)(C₂H₄O)ₙ) (where n = 20 to 30, and x = 3 to 8), or sulfated or sulfonated variants thereof.

The detergent or cleaning composition may contain an amphiphilic alkoxylated grease cleaning polymer which has balanced hydrophilic and hydrophobic properties such that it removes grease particles from fabrics and surfaces.

In a specific embodiment of the amphiphilic alkoxylated grease cleaning polymer in the present disclosure, the amphiphilic alkoxylated grease cleaning polymer contains a core structure and a plurality of alkoxylate groups attached to the core structure. These may contain alkoxylated polyalkylenimines having an inner polyethylene oxide block and an outer polypropylene oxide block. These may include, but are not limited to, ethoxylated polyethyleneimine, ethoxylated hexamethylene diamine, and sulfated versions thereof. Polypropoxylated derivatives may be also included. A wide variety of amines and polyaklyeneimines can be alkoxylated to various degrees. A useful example is a 600 g/mol polyethyleneimine core ethoxylated to 20 EO groups per NH and is available from BASF.

The detergent composition described in the present disclosure may contain alkoxylated polyamines in an amount from about 0.1% to about 10%, in some examples from about 0.1% to about 8%, in other examples from about 0.1% to about 6%, based on the weight of the detergent composition.

Alkoxylated polycarboxylates such as those prepared from polyacrylates are useful in the present disclosure to provide additional grease removal performance. Such materials are described in WO 91/08281 and WO 90/01815. Chemically, these materials contain polyacrylates having one ethoxy side-chain per every 7 to 8 acrylate units. The side-chains have the formula: -(CH₂CH₂O)ₘ(CH₂)ₙCH₃, where m is 2 to 3 and n is 6 to 12. The side-chains are ester-linked to the polyacrylate "backbone" to provide a "comb" polymer-type structure.

The molecular weight can vary. It is typically in the range from about 2000 to about 50,000.

The detergent or cleaning composition of the present disclosure may contain alkoxylated polycarboxylates in an amount from about 0.1% by weight to about 10% by weight, in some examples from about 0.25% by weight to about 5% by weight, in other examples from about 0.3% by weight to about 2% by weight.

A suitable, preferred amphiphilic graft copolymer contains (i) a polyethylene glycol backbone and (ii) at least one pendant moiety selected from polyvinyl acetate, polyvinyl alcohol, and mixtures thereof. A preferred amphiphilic graft copolymer is Sokalan^{®} HP22 supplied from BASF. Suitable polymers include random graft copolymers, preferably a polyvinyl acetate grafted polyethylene oxide copolymer having a polyethylene oxide backbone and multiple polyvinyl acetate side chains.

The molecular weight of the polyethylene oxide backbone is typically about 6000, the weight ratio of polyethylene oxide to polyvinyl acetate is about 40 to 60, and the number of grafting points is 1 or less per 50 ethylene oxide units.

Carboxylate polymer - The detergent or cleaning composition of the present disclosure may further contain one or more carboxylate polymers such as a maleate/acrylate random copolymer or a polyacrylate homopolymer. In one aspect, the carboxylate polymer is a polyacrylate homopolymer having a molecular weight from 4,000 Da to 9,000 Da or from 6,000 Da to 9,000 Da.

Soil release polymer - The detergent or cleaning composition of the present disclosure may further contain one or more soil release polymers having a structure as defined by one of the following structures (I), (II), and (III) :
(I) -[(OCHR¹⁸-CHR¹⁹)ᵢ-O-OC-Ar-CO-]ₒ;
(II) -[(OCHR²⁰-CHR²¹)ⱼ-O-OC-sAr-CO-]_{q};
(III) -[(OCHR²²-CHR²³)ₖ-OR²⁴]ₜ,
where:
i, j, and k are each from 1 to 200;
p, o, and t are each from 1 to 50;
Ar is a 1,4-substituted phenylene;
sAr is 1,3-substituted phenylene substituted in position 5 with SO₃Me;
Me is Li, K, Mg/2, Ca/2, Al/3, ammonium, mono-, di-, tri-, or tetraalkylammonium (where the alkyl groups are C1-C18 alkyl or C2-C10 hydroxyalkyl), or mixtures thereof;
R¹⁸, R¹⁹, R²⁰, R²¹, R²², and R²³ are independently selected from H or n- or iso-C1-C18 alkyl; and
R²⁴ is a linear or branched C1-C18 alkyl, a linear or branched C2-C30 alkenyl, a C5-C9 cycloalkyl group, a C8-C30 aryl group, or a C6-C30 arylalkyl group.

Suitable soil release polymers are polyester soil release polymers such as Repel-o-tex polymers (e.g., Repel-o-tex SF, SF-2, and SRP6 supplied by Rhodia). Other suitable soil release polymers include Texcare polymers (e.g., Texcare SRA100, SRA300, SRN100, SRN170, SRN240, SRN300, and SRN325 supplied by Clariant). Other suitable soil release polymers are Marloquest polymers (e.g., Marloquest SL supplied by Sasol).

Cellulosic polymer - The consumer product of the present disclosure may further contain one or more cellulosic polymers including those selected from alkyl cellulose, alkyl alkoxyalkyl cellulose, carboxyalkyl cellulose, and alkyl carboxyalkyl cellulose. In one aspect, the cellulosic polymers are selected from the group consisting of carboxymethyl cellulose, methyl cellulose, methyl hydroxyethyl cellulose, methyl carboxymethyl cellulose, and mixtures thereof.

In one aspect, the carboxymethyl cellulose has a degree of carboxymethyl substitution from 0.5 to 0.9 and a molecular weight from 100,000 Da to 300,000 Da.

Examples of dispersant polymers are found in U.S. Patent No. 3,308,067, European Patent No. 66915, European Patent No. 193,360, and European Patent No. 193,360.

### <Amines>

In the detergent or cleaning composition of the present disclosure, various amines to be added for removal of grease and particulates from soiled materials may be used. The detergent or cleaning composition of the present disclosure may contain additional amines in an amount from about 0.1% by weight to about 10% by weight, in some examples from about 0.1% by weight to about 4% by weight, in other examples from about 0.1% by weight to about 2% by weight, of the detergent composition. Non-limiting examples of the amines include polyamines, oligoamines, triamines, diamines, pentamines, tetraamines, polyetheramines, and combinations thereof. Specific examples of suitable additional amines include tetraethylenepentamine, triethylenetetraamine, diethylenetriamine, polyetheramines, and mixtures thereof.

### <Bleaching agents>

The detergent or cleaning composition of the present disclosure may contain one or more bleaching agents. Suitable bleaching agents other than bleaching catalysts include photocatalysts, bleach activators, hydrogen peroxide, hydrogen peroxide sources, pre-formed peracids, and mixtures thereof. In general, when a bleaching agent is used, the detergent or cleaning composition of the present disclosure may contain a bleaching agent in an amount from about 0.1% by weight to about 50% by weight or from about 0.1% by weight to about 25% by weight, of the detergent or cleaning composition of the present disclosure. The following describes suitable examples of the bleaching agents.
(1) Photobleaching agents such as sulfonated zinc phthalocyanines, sulfonated aluminum phthalocyanines, xanthene dyes, and mixtures thereof.
(2) Pre-formed peracids include, but not limited to, as suitable pre-formed peracids, compounds selected from the group consisting of percarboxylic acids and salts thereof, percarbonic acids and salts thereof, perimidic acids and salts thereof, peroxymonosulfuric acids and salts thereof (e.g., Oxone^{®}), and mixtures thereof; and as suitable percarboxylic acids, hydrophobic or hydrophilic peracids having the formula R²⁵-(C=O)O-O-M (where R²⁵ is an optionally branched alkyl group, and when the peracid is hydrophobic, the alkyl group has 6 to 14 carbon atoms or 8 to 12 carbon atoms, and when the peracid is hydrophilic, the alkyl group has less than 6 carbon atoms or even less than 4 carbon atoms, and M is a counterion (e.g., sodium, potassium, or hydrogen)).
(3) Hydrogen peroxide sources are inorganic perhydrate salts, and include alkali metal salts such as sodium perborate (usually mono- or tetra-hydrate) salts, sodium percarbonate salts, sodium persulfate salts, sodium perphosphate salts, sodium persilicate salts, and mixtures thereof. In one aspect of the present disclosure, the inorganic perhydrate salts are selected from the group consisting of sodium perborate salts, sodium percarbonate salts, and mixtures thereof. When used, the inorganic perhydrate salts are typically present in amounts from 0.05 to 40% by weight, or 1 to 30% by weight, of the overall fabric care products or the overall home care products and are typically incorporated, in a crystalline solid form that may be coated, into such fabric care products or home care products. Suitable coatings include inorganic salts (e.g., alkali metal silicate salts, alkali metal carbonate salts, alkali metal borate salts, or mixtures thereof) and organic materials (e.g., water-soluble or dispersible polymers, waxes, oils, or fatty soaps).
(4) Bleach activators having R²⁶-(C=O)-L¹ (where R²⁶ is an alkyl group, which may be optionally branched, and when the bleach activator is hydrophobic, the alkyl group contains 6 to 14 carbon atoms or 8 to 12 carbon atoms, and when the bleach activator is hydrophilic, the alkyl group contains less than 6 carbon atoms or even less than 4 carbon atoms; and L¹ is a leaving group). Examples of suitable leaving groups are benzoic acid and derivatives thereof, especially benzene sulfonate. Suitable bleach activators include dodecanoyl oxybenzene sulfonate, decanoyl oxybenzene sulfonate, decanoyl oxybenzoic acid or salts thereof, 3,5,5-trimethyl hexanoyloxybenzene sulfonate, tetraacetyl ethylene diamine (TAED), and nonanoyloxybenzene sulfonate (NOBS). Suitable bleach activators are also disclosed in WO 98/17767. While any suitable bleach activator may be used, in one aspect of the present disclosure, the detergent or cleaning composition may contain NOBS, TAED, or a mixture thereof.

When present, the peracid and/or bleach activator is generally present in the detergent or cleaning composition in an amount from about 0.1 to about 60% by weight, from about 0.5 to about 40% by weight, or from about 0.6 to about 10% by weight, of the fabric care products or the home care products. One or more selected from hydrophobic peracids and precursors thereof may be used in combination with one or more selected from hydrophilic peracids and precursors thereof.

The amounts of hydrogen peroxide sources and peracids or bleach activators may be selected such that the molar ratio of available oxygen (from the peroxide sources) to peracid is 1:1 to 35:1, or even 2:1 to 10:1.

### <Bleaching catalysts>

The detergent or cleaning composition of the present disclosure may further contain one or more bleaching catalysts capable of accepting an oxygen atom from a peroxy acid and/or a salt thereof and transferring the oxygen atom to an oxidizable substrate. Non-limiting examples of suitable bleaching catalysts include iminium cations and polyions, iminium zwitterions, modified amines, modified amine oxides, N-sulfonyl imines, N-phosphonyl imines, N-acyl imines, thiadiazole dioxides, perfluoroimines, cyclic sugar ketones, and mixtures thereof.

In another aspect, the laundry detergent composition contains a bleach component. The bleach component has a log Po/w of 0 or less, -0.5 or less, -1.0 or less, -1.5 or less, -2.0 or less, -2.5 or less, -3.0 or less, or even - 3.5 or less. The method for determining log Po/w is described in more detail below.

Typically, the bleach component is capable of generating a bleaching species having a XSO from 0.01 to about 0.30, from 0.05 to about 0.25, or from about 0.10 to 0.20. The method for determining a XSO is described in more detail below. For example, bleaching components having an isoquinolinium structure are capable of generating a bleaching species that has an oxaziridinium structure. In this example, the XSO is that of the oxaziridinium bleaching species.

Without wishing to be bound by theory, the inventors of the present disclosure believe that controlling the electophilicity and hydrophobicity in the above-described manner enables the bleach component to be delivered substantially only to areas of the fabric that are more hydrophobic, and that contain electron rich soils containing visible chromophores and susceptible to bleaching by highly electrophilic oxidants.

In one aspect, the bleaching catalyst has a structure corresponding to the following formula: 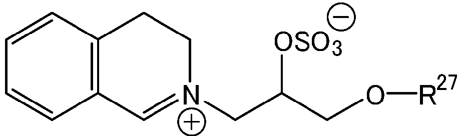 where R²⁷ is selected from the group consisting of 2-ethylhexyl, 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl, and iso-pentadecyl.

Log Po/w is determined according to the method found in Brooke, D.N., Dobbs, A.J., Williams, N, Ecotoxicology and Environmental Safety (1986) 11(3): 251-260. The parameter XSO is determined according to the method described in Adam, W., Haas, W., Lohray, B.B. Journal of the American Chemical Society (1991) 113(16) 6202-6208.

### <Brighteners>

Fluorescent brighteners or other brighteners or whitening agents may be incorporated into the cleaning composition of the present disclosure at concentrations from about 0.01% by weight to about 1.2% by weight of the composition. Commercial fluorescent brighteners suitable in the present disclosure can be classified into subgroups including, but not limited to, stilbene, pyrazoline, coumarin, benzoxazoles, carboxylic acid, methinecyanine, dibenzothiophene-5,5-dioxide, azoles, 5- and 6-membered-ring heterocycles, and derivatives of other various substances. Examples of such brighteners are disclosed in "The Production and Application of Fluorescent Brightening Agents," M. Zahradnik, John Wiley & Sons, New York (1982). Specific, non-limiting examples of fluorescent brighteners which may be useful in the composition of the present disclosure are those identified in U.S. Patent No. 4,790,856, U.S. Patent No. 3,646,015, U.S. Patent No. 7863236, and the counterpart CN 1764714.

In some examples, the fluorescent brightener in the present disclosure contains a compound represented by the formula (a): 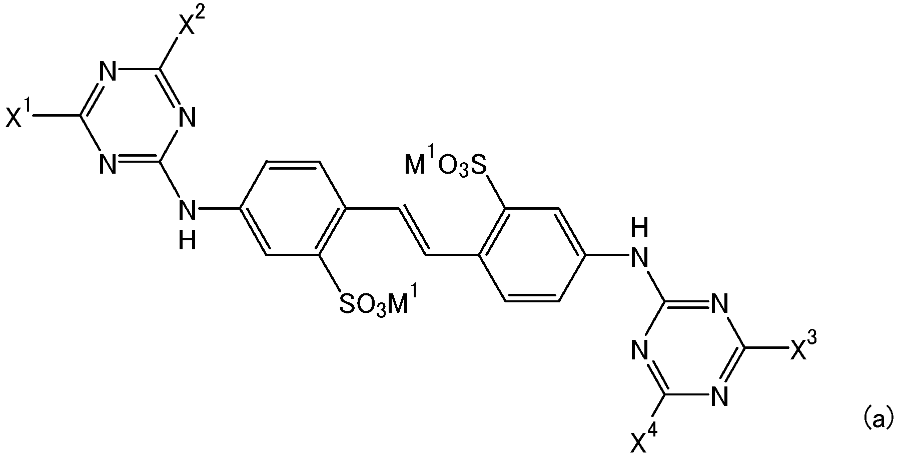 where X¹, X², X³, and X⁴ are each -N(R²⁸)R²⁹, where R²⁸ and R²⁹ are each independently selected from hydrogen, phenyl, hydroxyethyl, and unsubstituted or substituted C1-C8 alkyl, or -N(R²⁸) R²⁹ forms a heterocycle; preferably, R²⁸ and R²⁹ are each independently selected from hydrogen and phenyl, or -N(R²⁸)R²⁹ forms an unsubstituted or substituted monophorine ring; and M¹ is hydrogen or a cation, preferably sodium or potassium, more preferably sodium.

In some examples, the fluorescent brightener is selected from the group consisting of disodium 4,4'-bis{ [4-anilino-6-morpholino-s-triazin-2-yl]-amino}-2,2'-stilbenedisulfonate (brightener 15, commercially available under the trade name Tinopal AMS-GX by Ciba Geigy Corporation), disodium 4,4'-bis{ [4-anilino-6-(N-2-bis-hydroxyethyl)-s-triazin-2-yl]-amino}-2,2'-stilbenedisulonate (commercially available under the trade name Tinopal UNPA-GX by Ciba-Geigy Corporation), and disodium 4,4'-bis{ [4-anilino-6-(N-2-hydroxyethyl-N-methylamino)-s-triazin-2-yl]-amino}-2,2'-stilbenedisulfonate (commercially available under the trade name Tinopal 5BM-GX by Ciba-Geigy Corporation). More preferably, the fluorescent brightener is disodium 4,4'-bis{[4-anilino-6-morpholino-s-triazin-2-yl]-amino}-2,2'-stilbenedisulfonate.

The brighteners may be added in particulate form or as a premix with a suitable solvent, for example, nonionic surfactant, monoethanolamine, or propane diol.

### <Fabric hueing agents>

The composition may contain a fabric hueing agent (sometimes referred to as shading agents, bluing agents, or whitening agents). Typically, the hueing agent provides a blue or violet shade to fabric. Hueing agents can be used either alone or in combination to create a specific shade of hueing and/or to shade different fabric types. This may be provided, for example, by mixing a red dye and a green-blue dye to yield a blue or violet shade. Hueing agents may be selected from any known chemical class of dye, including, but not limited to, acridine, anthraquinone (including polycyclic quinones), azines, azo compounds including metallized azo compounds (e.g., monoazo, disazo, trisazo, tetrakisazo, and polyazo compounds), benzodifurane and benzodifuranone, carotenoid, coumarin, cyanine, diazahemicyanine, diphenylmethane, formazan, hemicyanine, indigoids, methane, naphthalimides, naphthoquinone, nitro and nitroso, oxazine, phthalocyanine, pyrazoles, stilbene, styryl, triarylmethane, triphenylmethane, xanthenes, and mixtures thereof.

Suitable fabric hueing agents include dyes, dye-clay conjugates, organic pigments, and inorganic pigments. Suitable dyes include small molecule dyes and polymeric dyes. Suitable small molecule dyes include small molecule dyes selected from the group consisting of dyes classified into the color index (C.I.) classifications of direct, basic, reactive, or hydrolysed reactive, solvent, or disperse dyes, which are classified as blue, violet, red, green, or black and provide the desired shade either alone or in combination. In another aspect, suitable small molecule dyes include small molecule dyes selected from the group consisting of those with color index (Society of Dyers and Colourists, Bradford, UK) numbers such as direct violet dyes such as 9, 35, 48, 51, 66, and 99, direct blue dyes such as 1, 71, 80, and 279, acid red dyes such as 17, 73, 52, 88, and 150, acid violet dyes such as 15, 17, 24, 43, 49, and 50, acid blue dyes such as 15, 17, 25, 29, 40, 45, 75, 80, 83, 90, and 113, acid black dyes such as 1, basic violet dyes such as 1, 3, 4, 10, and 35, basic blue dyes such as 3, 16, 22, 47, 66, 75, and 159, disperse or solvent dyes such as those described in European Patent No. 1794275 or European Patent No. 1794276, dyes such as those disclosed in U.S. Patent No. 7,208,459 B2, and mixtures thereof. In another aspect, suitable small molecule dyes include small molecule dyes selected from the group consisting of those with C.I. numbers such as acid violet 17, direct blue 71, direct violet 51, direct blue 1, acid red 88, acid red 150, acid blue 29, acid blue 113, and mixtures thereof.

Suitable polymeric dyes include polymeric dyes selected from the group consisting of polymers containing covalently bound (sometimes referred to as conjugated) chromogens (dye-polymer conjugates), such as polymers in which the backbone is prepared by copolymerizing polymers with chromogens, and mixtures thereof. Polymeric dyes include those described in WO 2011/98355, WO 2011/47987, US 2012/090102, WO 2010/145887, WO 2006/055787, and WO 2010/142503.

In another aspect, suitable polymeric dyes include polymeric dyes selected from the group consisting of fabric-direct colorants marketed under the name of Liquitint^{®} (Milliken, Spartanburg, South Carolina, USA), dye-polymer conjugates formed from at least one reactive dye and a polymer selected from the group consisting of polymers containing a moiety selected from the group consisting of a hydroxyl moiety, a primary amine moiety, a secondary amine moiety, a thiol moiety, and mixtures thereof. In still another aspect, suitable polymeric dyes include polymeric dyes selected from the group consisting of: Liquitint^{®} Violet CT; carboxymethyl cellulose (CMC) covalently bound to a reactive blue, reactive violet, or reactive red dye, such as CMC conjugated with C.I. reactive blue 19 marketed by Megazyme (Wicklow, Ireland) under the product name AZO-CM-CELLULOSE, product code S-ACMC; alkoxylated triphenyl-methane polymeric colorants; alkoxylated thiophene polymeric colorants; and mixtures thereof.

Preferred hueing dyes include the brighteners found in WO 08/87497 A1, WO 2011/011799, and WO 2012/054835. Preferred hueing agents for use in the present disclosure may be the preferred dyes disclosed in these references, including dyes selected from Examples 1 to 42 in Table 5 of WO 2011/011799. Other preferred dyes are disclosed in U.S. Patent No. 8138222. Other preferred dyes are disclosed in WO 2009/069077.

Suitable dye-clay conjugates include dye-clay conjugates selected from the group consisting of at least one cationic/basic dye and a smectite clay, and mixtures thereof. In another aspect, suitable dye-clay conjugates include: dye-clay conjugates selected from the group consisting of one cationic/basic dye selected from the group consisting of C.I. Basic Yellow 1 to 108, C.I. Basic Orange 1 to 69, C.I. Basic Red 1 to 118, C.I. Basic Violet 1 to 51, C.I. Basic Blue 1 to 164, C.I. Basic Green 1 to 14, C.I. Basic Brown 1 to 23, and CI Basic Black 1 to 11; and a clay selected from the group consisting of Montmorillonite clay, Hectorite clay, Saponite clay, and mixtures thereof. In still another aspect, suitable dye-clay conjugates include dye-clay conjugates selected from the group consisting of Montmorillonite Basic Blue B7 C.I. 42595 conjugate, Montmorillonite Basic Blue B9 C.I. 52015 conjugate, Montmorillonite Basic Violet V3 C.I. 42555 conjugate, Montmorillonite Basic Green G1 C.I. 42040 conjugate, Montmorillonite Basic Red R1 C.I. 45160 conjugate, Montmorillonite C.I. Basic Black 2 conjugate, Hectorite Basic Blue B7 C.I. 42595 conjugate, Hectorite Basic Blue B9 C.I. 52015 conjugate, Hectorite Basic Violet V3 C.I. 42555 conjugate, Hectorite Basic Green G1 C.I. 42040 conjugate, Hectorite Basic Red R1 C.I. 45160 conjugate, Hectorite C.I. Basic Black 2 conjugate, Saponite Basic Blue B7 C.I. 42595 conjugate, Saponite Basic Blue B9 C.I. 52015 conjugate, Saponite Basic Violet V3 C.I. 42555 conjugate, Saponite Basic Green G1 C.I. 42040 conjugate, Saponite Basic Red R1 C.I. 45160 conjugate, Saponite C.I. Basic Black 2 conjugate, and mixtures thereof.

Suitable pigments include pigments selected from the group consisting of flavanthrone, indanthrone, chlorinated indanthrone containing from 1 to 4 chlorine atoms, pyranthrone, dichloropyranthrone, monobromodichloropyranthrone, dibromodichloropyranthrone, tetrabromopyranthrone, perylene-3,4,9,10-tetracarboxylic acid diimide (where the imide groups may be unsubstituted or substituted by C1-C3-alkyl or a phenyl or heterocyclic group, and where the phenyl and heterocyclic groups may additionally have substituents which do not confer solubility in water), anthrapyrimidinecarboxylic acid amides, violanthrone, isoviolanthrone, dioxazine pigments, copper phthalocyanine which may contain up to 2 chlorine atoms per molecule, polychloro-copper phthalocyanine or polybromochloro-copper phthalocyanine containing up to 14 bromine atoms per molecule, and mixtures thereof.

In another aspect, suitable pigments include pigments selected from the group consisting of Ultramarine Blue (C.I. Pigment Blue 29), Ultramarine Violet (C.I. Pigment Violet 15), and mixtures thereof.

The fabric hueing agents can be used in combination (any mixture of fabric hueing agents can be used).

### <Capsules>

The composition may contain a capsule. In some aspects, the capsule may contain a core and a shell having an inner surface and an outer surface, where the shell encapsulates the core.

In an aspect, the capsule may contain a core and a shell. The core contains a material selected from perfumes, brighteners, dyes, insect repellants, silicones, waxes, flavors, vitamins, fabric softening agents, skin care agents (e.g., paraffins), enzymes, anti-bacterial agents, bleaching agents, sensates, or mixtures thereof. The shell contains a material selected from polyethylenes, polyamides, polyvinylalcohols optionally containing other co-monomers, polystyrenes, polyisoprenes, polycarbonates, polyesters, polyacrylates, polyolefins, polysaccharides (e.g., alginate and/or chitosan), gelatin, shellac, epoxy resins, vinyl polymers, water insoluble inorganics, silicones, aminoplasts, and mixtures thereof. When the shell contains an aminoplast, the aminoplast may contain polyurea, polyurethane, and/or polyureaurethane. The polyurea may contain polyoxymethyleneurea and/or melamine formaldehyde.

In some aspects, the capsule may encapsulate the core, and may contain a perfume. In a certain aspect, the capsule may contain a shell, and the shell may contain melamine formaldehyde and/or cross linked melamine formaldehyde. In some aspects, the capsule may contain a core containing a perfume and a shell containing melamine formaldehyde and/or cross-linked melamine formaldehyde.

Suitable capsules may contain a core material and a shell, where the shell at least partially surrounds the core material. At least 75%, or at least 85%, or even at least 90% of the capsules may have a fracture strength from about 0.2 MPa to about 10 MPa, from about 0.4 MPa to about 5 MPa, from about 0.6 MPa to about 3.5 MPa, or from about 0.7 MPa to about 3MPa; and a benefit agent leakage from 0% to about 30%, from 0% to about 20%, or even from 0% to about 5%.

In some aspects, at least 75%, 85%, or even 90% of the capsules may have a particle size from about 1 um to about 80 um, about 5 um to 60 um, from about 10 um to about 50 µm, or from about 15 um to about 40 µm.

In some aspects, at least 75%, 85%, or even 90% of the capsules may have a particle wall thickness from about 30 nm to about 250 nm, from about 80 nm to about 180 nm, or from about 100 nm to about 160 nm.

In some aspects, the core of the capsule contains a material selected from perfume raw materials and/or optionally a material selected from vegetable oil including neat and/or blended vegetable oils including castor oil, coconut oil, cottonseed oil, grape oil, rapeseed, soybean oil, corn oil, palm oil, linseed oil, safflower oil, olive oil, peanut oil, coconut oil, palm kernel oil, castor oil, lemon oil, and mixtures thereof; esters of vegetable oils (esters including dibutyl adipate, dibutyl phthalate, butyl benzyl adipate, benzyl octyl adipate, tricresyl phosphate, trioctyl phosphate, and mixtures thereof); linear or branched hydrocarbons including linear or branched hydrocarbons having a boiling point of greater than about 80°C; partially hydrogenated terphenyls, dialkyl phthalates, alkyl biphenyls including monoisopropylbiphenyl, alkylated naphthalene including dipropylnaphthalene, petroleum spirits including kerosene, mineral oil, or mixtures thereof; aromatic solvents including benzene, toluene, or mixtures thereof; silicone oils; and mixtures thereof.

In some aspects, the wall of the capsule contains a suitable resin such as the reaction product of an aldehyde and an amine. Suitable aldehydes include formaldehyde. Suitable amines include melamine, urea, benzoguanamine, glycoluril, or mixtures thereof. Suitable melamines include methylol melamine, methylated methylol melamine, imino melamine, and mixtures thereof. Suitable ureas include dimethylol urea, methylated dimethylol urea, urea-resorcinol, or mixtures thereof.

In some aspects, suitable formaldehyde scavengers may be used with the capsules, for example, in a capsule slurry and/or added to the composition of the present disclosure before, during, or after the capsules are added to the composition of the present disclosure.

Suitable capsules are disclosed in US 2008/0305982 A1 and/or US 2009/0247449 A1. Alternatively, suitable capsules can be purchased from Appleton Papers Inc. (Appleton, Wisconsin USA).

In addition, the materials for making the capsules can be available from Solutia Inc. (St Louis, Missouri USA), Cytec Industries (West Paterson, New Jersey USA), sigma-Aldrich (St. Louis, Missouri USA), CP Kelco Corp. (San Diego, California, USA), BASF AG (Ludwigshafen, Germany), Rhodia Corp. (Cranbury, New Jersey, USA), Hercules Corp. (Wilmington, Delaware, USA), Agrium Inc. (Calgary, Alberta, Canada), ISP (New Jersey USA), Akzo Nobel (Chicago, IL, USA), Stroever Shellac Bremen (Bremen, Germany), Dow Chemical Company (Midland, MI, USA), Bayer AG (Leverkusen, Germany), and Sigma-Aldrich Corp. (St. Louis, Missouri, USA) .

### <Perfumes>

Perfumes and perfumery components may be used in the cleaning composition of the present disclosure. Non-limiting examples of perfumes and perfumery components include aldehydes, ketones, esters, and the like. Other examples include various natural extracts and essences which can contain complex mixtures of components such as orange oil, lemon oil, rose extract, lavender, musk, patchouli, balsamic essence, sandalwood oil, and pine oil, cedar. Finished perfumes can contain extremely complex mixtures of such components. Finished perfumes may be included at a concentration ranging from about 0.01% by weight to about 2% by weight of the cleaning composition.

### <Dye transfer inhibiting agents>

The cleaning composition may further contain one or more materials effective for inhibiting the transfer of dyes from one fabric to another during the cleaning process. Generally, such dye transfer inhibiting agents may include polyvinyl pyrrolidone polymers, polyamine N-oxide polymers, copolymers of N-vinylpyrrolidone and N-vinylimidazole, manganese phthalocyanine, peroxidases, and mixtures thereof. If used, these agents may be used at a concentration from about 0.0001% by weight to about 10% by weight of the composition, in some examples from about 0.01% by weight to about 5% by weight of the composition, in other examples from about 0.05% by weight to about 2% by weight, of the composition.

### <Chelating agents>

The detergent or cleaning composition of the present disclosure may further contain one or more metal ion-chelating agents. Suitable molecules include copper chelating agents, iron-chelating agents, and/or manganese chelating agents, and mixtures thereof. Such chelating agents can be selected from the group consisting of phosphonates, amino carboxylates, amino phosphonates, succinates, polyfunctionally-substituted aromatic chelating agents, 2-pyridinol-N-oxide compounds, hydroxamic acids, carboxymethyl inulins, and mixtures thereof. Chelating agents can be present in the acid or salt form including alkali metal, ammonium, and substituted ammonium salts thereof, and mixtures thereof. Non-limiting examples of chelating agents for use in the present disclosure are found in U.S. Patent No. 7445644, U.S. Patent No. 7585376, and US 2009/0176684 A1.

Aminocarboxylates useful as chelating agents include, but are not limited to, ethylenediaminetetracetates (EDTA), N-(hydroxyethyl)ethylenediaminetriacetates (HEDTA), nitrilotriacetates (NTA), ethylenediamine tetraproprionates, triethylenetetraaminehexacetates, diethylenetriamine-pentaacetates (DTPA), methylglycinediacetic acid (MGDA), glutamic acid diacetic acid (GLDA), ethanoldiglycines, triethylenetetraaminehexaacetic acid (TTHA), N-hydroxyethyliminodiacetic acid (HEIDA), dihydroxyethylglycine (DHEG), ethylenediaminetetrapropionic acid (EDTP), and derivatives thereof.

Phosphorus containing chelating agents include, but are not limited to, diethylene triamine penta(methylene phosphonic acid) (DTPMP CAS 15827-60-8), ethylene diamine tetra(methylene phosphonic acid) (EDTMP CAS 1429-50-1), 2-phosphonobutane 1,2,4-tricarboxylic acid (Bayhibit^{®} AM), hexamethylene diamine tetra(methylene phosphonic acid) (CAS 56744-47-9), hydroxy-ethane diphosphonic acid (HEDP CAS 2809-21-4), hydroxyethane dimethylene phosphonic acid, 2-phosphono-1,2,4-butanetricarboxylic acid (CAS 37971-36-1), 2-hydroxy-2-phosphono-acetic acid (CAS 23783-26-8), aminotri(methylenephosphonic acid) (ATMP CAS 6419-19-8), P,P'-(1,2-ethanediyl)bis-phosphonic acid (CAS 6145-31-9), P,P'-methylenebis-phosphonic acid (CAS 1984-15-2), triethylenediaminetetra(methylene phosphonic acid) (CAS 28444-52-2), p-(1-hydroxy-1-methylethyl)-phosphonic acid (CAS 4167-10-6), bis(hexamethylene triamine penta(methylenephosphonic acid)) (CAS 34690-00-1), N2,N2,N6,N6-tetrakis(phosphonomethyl)-lysine (CAS 194933-56-7, CAS 172780-03-9), salts thereof, and mixtures thereof. Preferably, these amino phosphonic acids do not contain alkyl or alkenyl groups having more than about 6 carbon atoms.

A biodegradable chelating agent that may also be used in the present disclosure is ethylenediamine disuccinate ("EDDS"). In some examples, but of course not limited to this particular example, the [S,S] isomer as described in U.S. Patent No. 4,704,233. In other examples, the trisodium salt of EDDA may be used, though other forms such as magnesium salts may also be useful. Polymeric chelating agents such as Trilon P^{®} from BASF may also be useful.

Polyfunctionally-substituted aromatic chelating agents may also be used in the cleaning composition. See U.S. Patent No. 3,812,044 (issued on May 21, 1974, Connor et al.) Preferred compounds of this type in acid form are dihydroxydisulfobenzenes such as 1,2-dihydroxy-3,5-disulfobenzene also known as Tiron. Other sulfonated catechols may also be used. In addition to disulfonic acid, the term "Tiron" may also encompass mono- or di-sulfonates of the acid (e.g., disodium sulfonate), which shares the same core molecular structure with the disulfonic acid.

The detergent or cleaning composition of the present disclosure may contain a substituted or unsubstituted 2-pyridinol-N-oxide compound or a salt thereof, as a chelating agent. Included within the scope of the present disclosure are tautomers of this compound (e.g., 1-hydroxy-2(1H)-pyridinone) as chelating agents. In a certain aspect, the detergent or cleaning composition contains a 2-pyridinol-N-oxide compound selected from the group consisting of 2-hydroxypyridine-1-oxide; 3-pyridinecarboxylic acid, 2-hydroxy-, 1-oxide; 6-hydroxy-3-pyridinecarboxylic acid, 1-oxide; 2-hydroxy-4-pyridinecarboxylic acid, 1-oxide; 2-pyridinecarboxylic acid, 6-hydroxy-, 1-oxide; 6-hydroxy-3-pyridinesulfonic acid, 1-oxide; and mixtures thereof.

In a certain aspect, the detergent or cleaning composition contains a 2-pyridinol-N-oxide compound selected from the group consisting of: 1-hydroxy-2(1H)-pyridinone (CAS 822-89-9); 1,6-dihydro-1-hydroxy-6-oxo-3-pyridinecarboxylic acid (CAS 677763-18-7); 1,2-dihydro-1-hydroxy-2-oxo-4-pyridinecarboxylic acid (CAS 119736-22-0); 1,6-dihydro-1-hydroxy-6-oxo-2-pyridinecarboxylic acid (CAS 94781-89-2); 1-hydroxy-4-methyl-6-(2,4,4-trimethylpentyl)-2(1H)-pyridinone (CAS 50650-76-5); 6-(cyclohexylmethyl)-1-hydroxy-4-methyl-2(1H)-pyridinone (CAS 29342-10-7); 1-hydroxy-4,6-dimethyl-2(1H)-pyridinone (CAS 29342-02-7); 1-hydroxy-4-methyl-6-(2,4,4-trimethylpentyl)-2-pyridone monoethanolamine (CAS 68890-66-4); 1-hydroxy-6-(octyloxy)-2(1H)-pyridinone (CAS 162912-64-3); 1-hydroxy-4-methyl-6-cyclohexyl-2-pyridinone ethanolamine salt (CAS 41621-49-2); 1-hydroxy-4-methyl-6-cyclohexyl-2-pyridinone (CAS 29342-05-0); 6-ethoxy-1,2-dihydro-1-hydroxy-2-oxo-4-pyridinecarboxylic acid, methyl ester (CAS 36979-78-9); 1-hydroxy-5-nitro-2(1H)-pyridinone (CAS 45939-70-6); and mixtures thereof. These compounds are commercially available from, for example, Sigma-Aldrich (St. Louis, MO.), Princeton Building Blocks (Monmouth Junction, NJ), 3B Scientific Corporation (Libertyville, IL), SynFine Research (Richmond Hill, ON), Ryan Scientific, Inc. (Mt. Pleasant, SC), and/or Aces Pharma (Branford, CT).

Hydroxamic acids are a class of chemical compounds in which a hydroxylamine is inserted into a carboxylic acid and are used as chelating agents. The general structure of a hydroxamic acid is represented by the following formula.

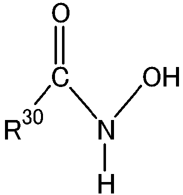

The preferred hydroxamates are those where R³⁰ is C4-C14 alkyl, preferably normal alkyl, most preferably saturated salts thereof and mixtures thereof. When the C8 material is used, it is called octyl hydroxamic acid.

Suitable chelating agents used in the present disclosure are the commercial DEQUEST series, and chelating agents from Monsanto, Akzo-Nobel, DuPont, Dow, and the Trilon^{®} series from BASF and Nalco.

The chelating agent may be present in the detergent or cleaning composition of the present disclosure in an amount from about 0.005% by weight to about 15% by weight, about 0.01% by weight to about 5% by weight, about 0.1% by weight to about 3.0% by weight, or from about 0.2% by weight to about 0.7% by weight, or from about 0.3% by weight to about 0.6% by weight, of the detergent or cleaning composition of the present disclosure.

### <Defoamers>

Compounds for reducing or suppressing the formation of foams can be incorporated into the detergent or cleaning composition of the present disclosure. Defoamers can be of particular importance in the so-called "high concentration cleaning process" as described in U.S. Patent No. 4,489,455 and U.S. Patent No. 4,489,574, and in front-loading style washing machines.

A wide variety of materials may be used as defoamers, and defoamers are known to those skilled in the art. See, for example, Kirk Othmer Encyclopedia of Chemical Technology, Third Edition, Volume 7, pages 430-447 (John Wiley & Sons, Inc., 1979). Examples of defoamers include monocarboxylic fatty acids and salts of soluble monocarboxylic fatty acids, high molecular weight hydrocarbons such as paraffin, fatty acid esters (e.g., fatty acid triglycerides), fatty acid esters of monovalent alcohols, aliphatic C18-C40 ketones (e.g., stearone), N-alkylated amino triazines, waxy hydrocarbons preferably having a melting point below about 100°C, silicone defoamers, and secondary alcohols. Defoamers are described in U.S. Patent No. 2,954,347, U.S. Patent No. 4,265,779, U.S. Patent No. 4,265,779, U.S. Patent No. 3,455,839, U.S. Patent No. 3,933,672, U.S. Patent No. 4,652,392, U.S. Patent No. 4,978,471, U.S. Patent No. 4,983,316, U.S. Patent No. 5,288,431, U.S. Patent No. 4,639,489, U.S. Patent No. 4,749,740, U.S. Patent No. 4,798,679, U.S. Patent No. 4,075,118, U.S. Patent No. 8,492,325, EP 89307851.9, European Patent No. 150,872, and DOS 2,124,526.

Additional suitable antifoams are those derived from phenylpropylmethyl substituted polysiloxanes.

In a certain example, the laundry or cleaning composition may contain a defoamer selected from organomodified silicone polymers with aryl or alkylaryl substituents combined with silicone resin and a primary filler, which is modified silica. The detergent or cleaning composition may contain a defoamer in an amount from about 0.001% by weight to about 4.0% by weight of the composition.

In another example, the detergent or cleaning composition contains a defoamer selected from: a) mixtures from about 80 to about 92% ethylmethyl, methyl(2-phenylpropyl) siloxane; from about 5 to about 14% MQ resin in octyl stearate; and from about 3 to about 7% modified silica; b) mixtures from about 78 to about 92% ethylmethyl, methyl(2-phenylpropyl) siloxane; from about 3 to about 10% MQ resin in octyl stearate; and from about 4 to about 12% modified silica; or c) mixtures thereof, where the percentages are by weight of the antifoamer.

The detergent or cleaning composition of the present disclosure may contain a defoamer in an amount from 0.1% by weight to about 10% by weight of the composition. When used as defoamers, aliphatic monocarboxylic acids and salts thereof may be present in an amount of up to about 5% by weight of the cleaning composition, in some examples from about 0.5% by weight to about 3% by weight of the detergent or cleaning composition. Silicone defoamers may be used in an amount of up to about 2.0% by weight of the cleaning composition, although higher amounts may be acceptable. Monostearyl phosphate defoamers may be used in an amount ranging from about 0.1% by weight to about 2% by weight of the cleaning composition. Hydrocarbon defoamers may be used in an amount ranging from about 0.01% by weight to about 5.0% by weight of the cleaning composition, although higher amounts may be acceptable. Alcohol defoamers may be used at a concentration ranging from about 0.2% by weight to about 3% by weight of the detergent or cleaning composition.

### <Unit dose product form using water-soluble film>

The composition of the present disclosure may also be encapsulated in a unit dose product form. In other words, a pouch made from a water-soluble film has a single compartment or multi compartments. Preferred film materials are preferably polymeric materials. The film material can, for example, be obtained by casting, blow molding, extrusion molding, or blown extrusion molding of the polymeric materials, as known in the art.

Preferred polymers, copolymers or derivatives thereof suitable for use as a pouch material are selected from polyvinyl alcohols, polyvinyl pyrrolidone, polyalkylene oxides, acrylamide, acrylic acid, cellulose, cellulose ethers, cellulose esters, cellulose amides, polyvinyl acetates, polycarboxylic acids and salts thereof, polyaminoacids or peptides, polyamides, polyacrylamide, copolymers of maleic/acrylic acids, polysaccharides including starch and gelatine, and natural gums such as xanthum and carragum. More preferred polymers are selected from polyacrylates and water-soluble acrylate copolymers, methylcellulose, carboxymethylcellulose sodium, dextrin, ethylcellulose, hydroxyethyl cellulose, hydroxypropyl methylcellulose, maltodextrin, and polymethacrylates, and most preferably selected from polyvinyl alcohols, polyvinyl alcohol copolymers, hydroxypropyl methyl cellulose (HPMC), and combinations thereof.

Preferably, the concentration of polymer in the pouch material, for example, a PVA polymer, is at least 60%. The polymer can have any weight average molecular weight, preferably from about 1000 to 1,000,000, more preferably from about 10,000 to 300,000, still more preferably from about 20,000 to 150,000. Mixtures of polymers can also be used as the pouch material.

Naturally, different film materials and/or films of different thickness may be employed in making the compartments of the present disclosure. A benefit in selecting different films is that the resulting compartments may exhibit different solubilities or release characteristics.

Most preferred film materials are PVA films known under the MonoSol trade reference M8630, M8900, H8779 (as described in the Applicants co-pending applications, reference numbers 44528 and 11599), those described in U.S. Patent No. 6166117 and U.S. Patent No. 6787512, and PVA films of corresponding solubility and deformability characteristics.

The film material in the present disclosure can further contain one or more additive components. For example, it can be beneficial to add plasticisers such as glycerol, ethylene glycol, diethyleneglycol, propylene glycol, sorbitol, and mixtures thereof. Other additives include functional detergent additives to be delivered to the cleaning water, such as organic dispersant polymers.

### <Foaming boosters>

When high foaming is desired, foaming boosters such as C10-C16 alkanolamides may be incorporated into the cleaning composition at a concentration ranging from about 1% by weight to about 10% by weight of the cleaning composition. Some examples include C10-C14 monoethanol and diethanol amides. If desired, water-soluble magnesium and/or calcium salts such as MgCl₂, MgSO₄, CaCl₂, and CaSO₄ may be added at a concentration from about 0.1% by weight to about 2% by weight of the cleaning composition, to provide additional foams and to enhance grease removal performance.

### <Conditioning agents>

The composition of the present disclosure may contain a high melting point fatty compound. The high melting point fatty compound useful in the present disclosure has a melting point of 25°C or higher, and is selected from the group consisting of fatty alcohols, fatty acids, fatty alcohol derivatives, fatty acid derivatives, and mixtures thereof. Such compounds of low melting point are not intended to be included in this section. Non-limiting examples of the high melting point fatty compounds are found in International Cosmetic Ingredient Dictionary, Fifth Edition, 1993 and CTFA Cosmetic Ingredient Handbook, Second Edition, 1992.

Considering improved conditioning benefits to be provided by a high melting point fatty compound, such as slippery feel during the application to wet hair and softness and moisturized feel on dry hair, the composition contains a high melting point fatty compound at a concentration from about 0.1% by weight to about 40% by weight, preferably from about 1% by weight to about 30% by weight, more preferably from about 1.5% by weight to about 16% by weight, about 1.5% by weight to about 8% by weight based on the composition.

The composition of the present disclosure may contain a cationic polymer. The concentration of the cationic polymer in the composition typically ranges from about 0.05% to about 3%, in another embodiment from about 0.075% to about 2.0%, in yet another embodiment from about 0.1% to about 1.0%. Suitable cationic polymers have cationic charge densities of at least about 0.5 meq/gm, in another embodiment at least about 0.9 meq/gm, in another embodiment at least about 1.2 meq/gm, in yet another embodiment at least about 1.5 meq/gm, while in one embodiment also less than about 7 meq/gm, in another embodiment less than about 5 meq/gm, when the pH of intended use of the composition is generally from about pH 3 to about pH 9, in one embodiment from about pH 4 to about pH 8.

In the present disclosure, the "cationic charge density" of a polymer refers to the ratio of the number of positive charges on the polymer to the molecular weight of the polymer. The average molecular weight of such suitable cationic polymers is generally from about 10,000 to 10,000,000, in one embodiment from about 50,000 to about 50,000,000, in another embodiment from about 100,000 to about 3,000,000.

Suitable cationic polymers for use in the composition of the present disclosure contain cationic nitrogen-containing moieties such as quaternary ammonium or cationic protonated amino moieties. Any anionic counterions can be used in combination with the cationic polymers so long as the polymers remain soluble in water, in the composition, or in a coacervate phase of the composition, and so long as the counterions are physically and chemically compatible with the essential components of the composition of the present disclosure or do not otherwise unduly impair product performance, stability, or aesthetics. Non-limiting examples of such counterions include halides (e.g., chloride, fluoride, bromide, iodide), sulfates, and methylsulfates.

Non-limiting examples of such polymers are described in the CTFA Cosmetic Ingredient Dictionary, 3rd edition, edited by Estrin, Crosley, and Haynes, (The Cosmetic, Toiletry, and Fragrance Association, Inc., Washington, D.C., 1982) .

Other suitable cationic polymers for use in the composition include polysaccharide polymers, cationic guar gum derivatives, quaternary nitrogen-containing cellulose ethers, synthetic polymers, copolymers of etherified cellulose, guar, and starch. When used, the cationic polymers in the present disclosure are either soluble in the composition of the present disclosure or are soluble in a complex coacervate phase in the composition of the present disclosure formed by the cationic polymer and the anionic, amphoteric and/or zwitterionic surfactant component described above. Complex coacervates of the cationic polymer can also be formed with other charged materials in the composition of the present disclosure.

The suitable cationic polymers are described in U.S. Patent No. 3,962,418, U.S. Patent No. 3,958,581, and US 2007/0207109 A1.

The composition of the present disclosure may contain a nonionic polymer as a conditioning agent. Polyalkylene glycols having a molecular weight of more than about 1000 are useful in the present invention. Useful are those having the following formula: 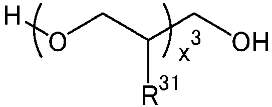 where R³¹ is selected from the group consisting of H, methyl, and mixtures thereof. Conditioning agents (in particular, silicones) may be contained in the composition of the present disclosure. The conditioning agents useful in the composition of the present disclosure typically contain a water insoluble, water dispersible, non-volatile liquid that forms emulsified, liquid particles. Suitable conditioning agents for use in the composition of the present disclosure are conditioning agents characterized generally as silicones (e.g., silicone oils, cationic silicones, silicone gums, high refractive silicones, and silicone resins), organic conditioning oils (e.g., hydrocarbon oils, polyolefins, and fatty esters), or combinations thereof or conditioning agents which otherwise form liquid, dispersed particles in the aqueous surfactant matrix in the present disclosure. Such conditioning agents should be physically and chemically compatible with the essential components of the composition, and should not otherwise unduly impair product stability, aesthetics, or performance.

The concentration of the conditioning agent in the composition should be sufficient to provide the desired conditioning benefits. Such a concentration can vary with the conditioning agent, the conditioning performance desired, the average size of the conditioning agent particles, the type and concentration of other components, and other like factors.

The concentration of the silicone conditioning agent typically ranges from about 0.01% to about 10%. Non-limiting examples of suitable silicone conditioning agents, and suspending agents for silicones are described in U.S. Reissue Pat. No. 34,584, U.S. Patent No. 5,104,646, U.S. Patent No. 5,106,609, U.S. Patent No. 4,152,416, U.S. Patent No. 2,826,551, U.S. Patent No. 3,964,500, U.S. Patent No. 4,364,837, U.S. Patent No. 6,607,717, U.S. Patent No. 6,482,969, U.S. Patent No. 5,807,956, U.S. Patent No. 5,981,681, U.S. Patent No. 6,207,782, U.S. Patent No. 7,465,439, U.S. Patent No. 7,041,767, U.S. Patent No. 7,217,777, US 2007/0286837 A1, US 2005/0048549 A1, US 2007/0041929 A1, GB 849,433, and DE 10036533, which are all incorporated herein by reference; "Chemistry and Technology of Silicones" (New York: Academic Press (1968)); General Electric Silicone Rubber Product Data Sheets SE 30, SE 33, SE 54 and SE 76; "Silicon Compounds" (Petrarch Systems, Inc. (1984)); and "Encyclopedia of Polymer Science and Engineering" (vol. 15, 2d ed., pp 204-308, John Wiley & Sons, Inc. (1989)).

The composition of the present disclosure may further contain from about 0.05% to about 3% of at least one organic conditioning oil as the conditioning agent, either alone or in combination with other conditioning agents such as the silicones (described in the present disclosure). Suitable conditioning oils include hydrocarbon oils, polyolefins, and fatty esters. Also suitable for use in the composition of the present disclosure are the conditioning agents described by the Procter & Gamble Company in U.S. Patent No. 5,674,478 and U.S. Patent No. 5,750,122. Also suitable for use in the present disclosure are the conditioning agents described in U.S. Patent No. 4,529,586, U.S. Patent No. 4,507,280, U.S. Patent No. 4,663,158, U.S. Patent No. 4,197,865, U.S. Patent No. 4,217,914, U.S. Patent No. 4,381,919, and U.S. Patent No. 4,422,853.

### <Hygiene and deodorization>

The composition of the present disclosure may further contain one or more of zinc ricinoleate, thymol, quaternary ammonium salts such as Bardac^{®}, polyethylenimines (e.g., Lupasol^{®} from BASF) and zinc complexes thereof, silver and silver compounds, especially those designed to slowly release Ag⁺ or nano-silver dispersions.

### <Probiotics>

The composition may contain probiotics such as those described in WO 2009/043709.

### <Fillers and carriers>

Fillers and carriers may be used in the cleaning composition of the present disclosure. In the present disclosure, the terms "filler" and "carrier" have the same meaning and can be used interchangeably.

Liquid cleaning compositions and cleaning compositions of other forms containing a liquid component (e.g., liquid-containing unit dose cleaning compositions) may contain water and other solvents as fillers or carriers. Suitable solvents also include lipophilic fluids including siloxanes, other silicones, hydrocarbons, glycol ethers, glycerol derivatives such as glycerol ethers, perfluorinated amines, perfluorinated and hydrofluoroether solvents, low-volatility non-organic solvents, diol solvents, and mixtures thereof.

Low molecular weight primary or secondary alcohols exemplified by methanol, ethanol, propanol, and isopropanol are suitable. In some examples, monohydric alcohols may be used for solubilizing surfactants, and polyols such as those containing from 2 to about 6 carbon atoms and from 2 to about 6 hydroxy groups (e.g., 1,3-propanediol, ethylene glycol, glycerol, and 1,2-propanediol) may also be used. Amine-containing solvents such as monoethanolamine, diethanolamine, and triethanolamine may also be used.

The detergent cleaning composition may contain such carriers in an amount from about 5% by weight to about 90% by weight, in some examples from about 10% by weight to about 50% by weight, of the composition. For compacted or super-compacted heavy duty liquid or cleaning compositions of other forms, regarding the use of water, the cleaning compositions may contain free water in an amount of less than about 40% by weight, less than about 20% by weight, less than about 5% by weight, less than about 4% by weight, less than about 3% by weight, or less than about 2% by weight, of the composition, or may be substantially free of free water (i.e., anhydrous).

For powder or bar cleaning compositions, or forms that contain a solid or powder component (e.g., powder-containing unit dose cleaning compositions), suitable fillers may include, but are not limited to, sodium sulfate, sodium chloride, clay, or other inert solid components. Fillers may also include biomass or decolorized biomass.

The amount of granular, bar, or other fillers in solid cleaning compositions may be less than about 80% of the weight of the cleaning composition, in some examples less than 50% of the weight of the cleaning composition. Compacted or super-compacted powder or solid cleaning compositions may contain fillers in an amount of less than about 40%, less than about 20%, or less than about 10%, of the weight of the cleaning composition.

For either compacted or super-compacted liquid or powder cleaning compositions, or other forms, the concentration of liquid or solid filler in the product is reduced, so that the same amount of active chemical substances is delivered to the cleaning liquid as compared to non-compacted cleaning compositions. Alternatively, in some examples, the cleaning composition is more efficient, so that a lesser amount of active chemical substances is delivered to the cleaning liquid as compared to non-compacted compositions. For example, the cleaning liquid may be formed by contacting the cleaning composition to water in an amount to achieve the concentration of cleaning composition in the cleaning liquid from more than 0 g/L to about 6 g/L. In some examples, the concentration may be from about 0.5 g/L to about 5 g/L, or to about 3.0 g/L, or to about 2.5 g/L, or to about 2.0 g/L, or to about 1.5 g/L, or from about 0 g/L to about 1.0 g/L, or from about 0 g/L to about 0.5 g/L. It is apparent to those skill in the art that these dosages are not intended to be limiting, and other dosages may be acceptable.

### <Buffer system>

The detergent or cleaning composition of the present disclosure may be formulated such that, during use in aqueous cleaning operations, the cleaning water has a pH of from about 7.0 to about 12, in some examples from about 7.0 to 11. Techniques for controlling pH at recommended usage levels include the use of buffers, alkalis, or acids, and are known to those skilled in the art. These techniques include, but are not limited to, the use of sodium carbonate, citric acid, or sodium citrate, lactic acid or lactates, monoethanol amine or other amines, boric acid or borates, and other pH-adjusting compounds known in the art.

The detergent or cleaning composition of the present disclosure may contain dynamic in-wash pH profiles. Such a cleaning composition may use wax-covered citric acid in combination with other pH adjusters such that (i) about 3 minutes after contact with water, the pH of the cleaning liquid is greater than 10; (ii) about 10 minutes after contact with water, the pH of the cleaning liquid is less than 9.5; (iii) about 20 minutes after contact with water, the pH of the cleaning liquid is less than 9.0; and (iv) optionally, the equilibrium pH of the cleaning liquid is in the range from about 7.0 to about 8.5.

### <Catalytic metal complex>

The detergent or cleaning composition may include catalytic metal complexes. One type of metal-containing bleaching catalyst is a catalyst system including a transition metal cation having a defined bleach catalytic activity, such as copper, iron, titanium, ruthenium, tungsten, molybdenum, or manganese cations; an auxiliary metal cation having little or no bleach catalytic activity, such as zinc or aluminum cations; and a metal ion sequestrate having a defined stability constant for the catalytic and auxiliary metal cations, particularly ethylenediaminetetraacetic acid, ethylenediaminetetra(methylenephosphonic acid) and water-soluble salts thereof. Such catalysts are disclosed in U.S. Patent No. 4,430,243.

If desired, the composition of the present disclosure can be catalyzed by means of a manganese compound. Such a compound and the concentration of the compound during use are known in the art and include, for example, the manganese-based catalysts disclosed in U.S. Patent No. 5,576,282.

Cobalt bleaching catalysts useful in the present disclosure are known, and are described, for example, in U.S. Patent No. 5,597,936 and U.S. Patent No. 5,595,967. Such cobalt catalysts are readily prepared by known procedures as taught in U.S. Patent No. 5,597,936 and U.S. Patent No. 5,595,967, for example.

The composition of the present disclosure may also suitably contain a transition metal complex with ligands such as bispidones (WO 05/042532 A1) and/or a transition metal complex with macropolycyclic rigid ligands (abbreviated as "MRLs"). The compositions and processes in the present disclosure can be adjusted to provide the active MRL species on the order of at least one per hundred million in the aqueous cleaning medium, and provide MRLs from about 0.005 ppm to about 25 ppm, from about 0.05 ppm to about 10 ppm, or from about 0.1 ppm to about 5 ppm in the cleaning solution. Examples of suitable transition metals in the transition-metal bleaching catalyst include manganese, iron, and chromium. Suitable MRLs include 5,12-diethyl-1,5,8,12-tetraazabicyclo[6.6.2]hexadecane. Suitable transition metal MRLs are readily prepared by known procedures as taught in WO 00/32601 and U.S. Patent No. 6,225,464, for example.

### <Other adjunct components>

The detergent or cleaning composition of the present disclosure may contain a wide variety of other components including other active components, carriers, hydrotropes, processing aids, dyes or pigments, solvents for liquid formulations, and solid or other liquid fillers, erythrosine, colliodal silica, waxes, probiotics, surfactin, aminocellulosic polymers, zinc ricinoleate, perfume microcapsules, rhamnolipids, sophorolipids, glycopeptides, methyl ester sulfonates, methyl ester ethoxylates, sulfonated estolides, cleavable surfactants, biopolymers, silicones, modified silicones, aminosilicones, deposition aids, locust bean gum, cationic hydroxyethylcellulose polymers, cationic guars, hydrotropes (especially cumenesulfonate salts, toluenesulfonate salts, xylenesulfonate salts, and naphalene salts), antioxidants, BHT, PVA particle-encapsulated dyes or perfumes, pearlescent agents, effervescent agents, color change systems, silicone polyurethanes, opacifiers, tablet disintegrants, biomass fillers, fast-dry silicones, glycol distearate, hydroxyethylcellulose polymers, hydrophobically modified cellulose polymers or hydroxyethylcellulose polymers, starch perfume encapsulates, emulsified oils, bisphenol antioxidants, microfibrous cellulose structurants, properfumes, styrene/acrylate polymers, triazines, soaps, superoxide dismutase, benzophenone protease inhibitors, functionalized TiO₂, dibutyl phosphate, silica perfume capsules, and other adjunct components, silicate salts (e.g., sodium silicate, potassium silicate), choline oxidase, pectate lyase, mica, titanium dioxide coated mica, bismuth oxychloride, and other actives.

The detergent or cleaning composition of the present disclosure may further contain vitamins and amino acids such as water soluble vitamins and their derivatives, water soluble amino acids and their salts and/or derivatives, water insoluble amino acids viscosity modifiers, dyes, nonvolatile solvents or diluents (water soluble and insoluble), pearlescent aids, foam boosters, additional surfactants or nonionic cosurfactants, pediculocides, pH adjusters, perfumes, preservatives, chelating agents, proteins, skin active agents, sunscreens, UV absorbers, vitamins, niacinamide, caffeine, and minoxidil.

The detergent or cleaning composition of the present disclosure may further contain pigment materials such as nitroso, monoazo, disazo, carotenoid, triphenyl methane, triaryl methane, xanthene, quinoline, oxazine, azine, anthraquinone, indigoid, thionindigoid, quinacridone, and phthalocyanine; and botanical and natural colors including water soluble components such as those having C.I. names. The cleaning composition of the present disclosure may further contain antimicrobial agents.

### <Processes of preparing detergent or cleaning composition>

The detergent or cleaning composition of the present disclosure can be formulated into any suitable form and prepared by any process chosen by the formulator. Non-limiting examples thereof include those described in U.S. Patent No. 4,990,280, US 20030087791 A1, US 20030087790 A1, US 20050003983 A1, US 20040048764 A1, U.S. Patent No. 4,762,636, U.S. Patent No. 6,291,412, US 20050227891 A1, EP 1070115 A2, U.S. Patent No. 5,879,584, U.S. Patent No. 5,691,297, U.S. Patent No. 5,574,005, U.S. Patent No. 5,569,645, U.S. Patent No. 5,565,422, U.S. Patent No. 5,516,448, U.S. Patent No. 5,489,392, and U.S. Patent No. 5,486,303.

### <Methods of using detergent or cleaning composition>

The present disclosure includes a method of using the detergent or cleaning composition for cleaning soiled materials. As will be appreciated by one skilled in the art, the detergent or cleaning composition of the present disclosure is suited for use in laundry pretreatment applications, laundry cleaning applications, and home care applications.

Such methods include, but are not limited to, contacting a neat detergent or cleaning composition or a detergent or cleaning composition diluted in cleaning liquid with at least a portion of a soiled material and then optionally rinsing the soiled material. The soiled material may be subjected to a washing step prior to the optional rinsing step.

For use in laundry pretreatment applications, the method may include contacting the detergent or cleaning composition of the present disclosure with soiled fabric. Following pretreatment, the soiled fabric may be laundered in a washing machine or otherwise rinsed.

Machine laundry methods may include treating soiled laundry with an aqueous cleaning solution in a washing machine containing an effective amount of a machine laundry cleaning composition of the present disclosure dissolved or dispensed therein. The "effective amount" of the cleaning composition means from about 20 g to about 300 g of a product dissolved or dispersed in about 5 L to about 65 L of the cleaning solution.

The water temperatures may range from about 5°C to about 100°C.

The water to soiled material (e.g., fabric) ratio may be from about 1:1 to about 30:1. The composition may be used at a concentration from about 500 ppm to about 15,000 ppm in solution. In the context of a fabric laundry composition, the concentration of the composition during use may also vary depending not only on the type and severity of the soils and stains, but also on the cleaning water temperature, the volume of cleaning water, and the type of washing machine (e.g., top-loading washing machine, front-loading washing machine, top-loading, vertical-axis Japanese-type automatic washing machine).

The cleaning composition of the present disclosure may be used for laundering fabric at low cleaning temperatures. These methods of laundering fabric include delivering a laundry cleaning composition to water to form a cleaning liquid and adding a laundering fabric to the cleaning liquid. The cleaning liquid has a temperature from about 0°C to about 20°C, or from about 0°C to about 15°C, or from about 0°C to about 9°C. The fabric may be contacted to water prior to, or after, or simultaneous with, contacting the laundry detergent composition with water.

Another method includes contacting a nonwoven substrate impregnated with the detergent or cleaning composition of one embodiment, with a soiled material. When used in the present disclosure, the "nonwoven substrate" can include any conventionally fashioned nonwoven sheet or web having suitable basis weight, caliper (thickness), absorbency, and strength characteristics. Non-limiting examples of suitable commercially available nonwoven substrates include those marketed under the trade names SONTARA^{®} by DuPont and POLYWEB^{®} by James River Corp.

Hand washing/soak methods, and combined handwashing with semi-automatic washing machines are also included.

### <Water treatment agent>

The polyalkylene oxide-containing compound of the present disclosure may be used not only in detergent or cleaning compositions, but also in water treatment agents. The water treatment agent may optionally contain other additives such as polymeric phosphoric acid salts, phosphonic acid salts, anticorrosive agents, slime controlling agents, and chelating agents.

The water treatment agent is useful to prevent the scales in a cool water circulation system, a boiler water circulation system, a seawater desalting unit, a pulp digester, a black liquor evaporator, etc. The water treatment agent may optionally contain a suitable water-soluble polymer within a range that does not affect performance or benefits.

### <Fiber treatment agent>

The detergent or cleaning composition of the present disclosure may be used in fiber treatment agents. The fiber treatment agent contains at least one selected from the group consisting of dyeing agents, peroxides, and surfactants and any of the polymers (or polymer compositions) of the present disclosure.

The fiber treatment agent contains the compound of the present disclosure in an amount preferably from 1 to 100% by weight, more preferably from 5 to 100% by weight, based on the entire fiber treatment agent. The fiber treatment agent may further contain a suitable water-soluble compound within a range that does not affect the performance and benefits.

The following describes a blending example of fiber treatment agents that is more similar to the embodiment. The fiber treatment agent can be used in scouring, dyeing, bleaching, or soaping in fiber treatment. The dyeing agent, peroxide, and surfactant include those commonly used in fiber treatment agents.

The blending ratio between the compound of the present disclosure and at least one selected from the group consisting of dyeing agents, peroxides, and surfactants is as follows. For example, in order to improve the degree of whiteness, color evenness, and degree of dyed colorfastness of fibers, the fiber treatment agent is preferably a composition containing 0.1 to 100 parts by weight of at least one selected from the group consisting of dyeing agents, peroxides, and surfactants per 1 part by weight of the compound of the present disclosure in terms of the pure content of fiber treatment agent.

The fiber treatment agent can be used for any appropriate fiber. Examples thereof include cellulose fibers such as cotton and hemp, chemical fibers such as nylon and polyester, animal fibers such as wool and silk, semisynthetic fibers such as rayon, woven fabrics thereof, and blended fabrics thereof.

In the case where the fiber treatment agent is applied to the scouring step, the compound of the present disclosure is preferably mixed with alkaline agents and surfactants. In the case where the fiber treatment agent is applied to the bleaching step, the compound of the present disclosure is preferably mixed with peroxides and silicic chemicals, which are used as inhibitors of alkaline bleaching agents from decomposition, such as sodium silicate.

### <Inorganic pigment-dispersing agents>

The compound of the present disclosure can be used in inorganic pigment-dispersing agents. The inorganic pigment-dispersing agent may optionally contain, as other compounding agents, condensed phosphoric acid and salts thereof, phosphonic acid and salts thereof, or polyvinyl alcohol.

The inorganic pigment-dispersing agent preferably contains the compound of the present disclosure in an amount from 5 to 100% by weight based on the entire inorganic pigment-dispersing agent. The inorganic pigment-dispersing agent may contain any suitable water-soluble compound within a range that does not affect performance or benefits.

The inorganic pigment-dispersing agent exhibits good performance as a dispersant for inorganic pigments such as a heavy or light calcium carbonate used for paper-coating or clay. For example, a small amount of the inorganic pigment-dispersing agent is added to disperse inorganic pigments in water. Thereby, a high-concentrated inorganic pigment slurry, like a high-concentrated calcium carbonate slurry, can be produced which has a low viscosity and high fluidity, with these properties being highly stable over time.

When the inorganic pigment-dispersing agent is used as a dispersant for inorganic pigments, a preferred amount of the inorganic pigment-dispersing agent used is from 0.05 to 2.0 parts by weight per 100 parts by weight of the inorganic pigment. When the amount of the inorganic pigment-dispersing agent used is within the above range, a sufficient dispersion effect can be obtained, and an effect commensurate with the amount added can be obtained, which may be economically advantageous.

### EXAMPLES

### <Molecular weight measurement conditions>

The molecular weights described in production examples were determined by gel permeation chromatography (GPC). The following describes the measurement conditions. Apparatus: HLC-8320GPC available from Tosoh Corporation Detector: RI
Column: TSKgel α-2500 + TSKgel α-M + TSK guardcolumn α, available from Tosoh Corporation
Column temperature: 40°C
Flow rate: 0.8 ml/min
Calibration curve: Polyethylene glycol, polyethylene oxide, available from GL Sciences Inc.
Eluent: 0.2 M Na nitrate, 0.5 M aqueous acetic acid solution/acetonitrile = 50/50 vol%

### <Production Example 1>

A glass reaction vessel equipped with a nitrogen inlet tube, a reflux condenser, and a stirrer was charged with 0.90 g of polyethyleneimine (Nippon Shokubai Co., Ltd., average molecular weight 600) and 20.6 g of methoxy polyethylene glycol #1000 acrylate (SHIN-NAKAMURA CHEMICAL Co., Ltd.), and was purged with nitrogen. Thereafter, under stirring, the temperature was raised to 60°C and the reaction was continued for 40 hours. After completion of the reaction, the complete consumption of the methoxy polyethylene glycol #1000 acrylate was confirmed by liquid chromatography. Thus, an adduct of methoxy polyethylene glycol #1000 acrylate with polyethyleneimine (polymer (1)) was obtained as a polyalkylene oxide-containing compound. The polymer (1) had a number average molecular weight of 8930 as determined by GPC analysis.

### <Production Example 2>

A glass reaction vessel equipped with a nitrogen inlet tube, a reflux condenser, and a stirrer was charged with 0.72 g of polyethyleneimine (Nippon Shokubai Co., Ltd., average molecular weight 600) and 13.39 g of methoxy polyethylene glycol #1000 acrylate (SHIN-NAKAMURA CHEMICAL Co., Ltd.), and was purged with nitrogen. Thereafter, under stirring, the temperature was raised to 60°C and the reaction was continued for 20 hours. After completion of the reaction, the complete consumption of the methoxy polyethylene glycol #1000 acrylate was confirmed by liquid chromatography. Thus, an adduct of methoxy polyethylene glycol #1000 acrylate with polyethyleneimine (polymer (2)) was obtained as a polyalkylene oxide-containing compound.

### <Production Example 3>

A glass reaction vessel equipped with a nitrogen inlet tube, a reflux condenser, and a stirrer was charged with 1.5 g of polyethyleneimine (Nippon Shokubai Co., Ltd., average molecular weight 600) and 19.17 g of methoxy polyethylene glycol #1000 acrylate (SHIN-NAKAMURA CHEMICAL Co., Ltd.), and was purged with nitrogen. Thereafter, under stirring, the temperature was raised to 60°C and the reaction was continued for 20 hours. After completion of the reaction, the complete consumption of the methoxy polyethylene glycol #1000 acrylate was confirmed by liquid chromatography. Thus, an adduct of methoxy polyethylene glycol #1000 acrylate with polyethyleneimine (polymer (3)) was obtained as a polyalkylene oxide-containing compound. The polymer (3) had a number average molecular weight of 8630 as determined by GPC analysis.

### <Production Example 4>

A glass reaction vessel equipped with a nitrogen inlet tube, a reflux condenser, and a stirrer was charged with 55.09 g of polyethyleneimine (Nippon Shokubai Co., Ltd., average molecular weight 600), and was purged with nitrogen at 60°C under stirring. Thereafter, under stirring, 97.90 g of ε-caprolactone (Tokyo Chemical Industry Co., Ltd.) was slowly added dropwise to the reaction vessel from a dropping funnel over 60 minutes. After completion of the dropwise addition, the reaction was continued for 28 hours. Furthermore, the temperature of the reaction solution was raised to 80°C and the reaction was continued for 12 hours. After completion of the reaction, the complete consumption of the ε-caprolactone was confirmed by liquid chromatography. Thus, a polyethyleneimine-ε-caprolactone condensate was obtained.

Subsequently, an autoclave was charged with 98.3 g of the obtained polyethyleneimine-ε-caprolactone condensate and 2.25 g of potassium hydroxide (FUJIFILM Wako Pure Chemical Corporation) and the temperature was raised to 120°C. The system of the autoclave was purged with nitrogen and then depressurized to 1.8 kPa using a vacuum pump to remove water for one hour from the autoclave. After completion of the removal of water, the pressure in the autoclave was increased to 0.3 MPa with nitrogen, and 181.8 g of ethylene oxide (Air Water Inc.) was gradually introduced into the autoclave for reaction. The reaction product was taken out and 101.8 g of the reaction product and 1.59 g of potassium hydroxide were placed in the autoclave again. Then, 198.1 g of ethylene oxide was reacted by the same procedure. After completion of the reaction, the reaction product was taken out to obtain an adduct of ethylene oxide with polyethyleneimine-ε-caprolactone condensate (polymer (4)) as a polyalkylene oxide-containing compound.

### <Production Example 5>

A glass reaction vessel equipped with a nitrogen inlet tube and a stirrer was charged with 10.0 g of ethylenediamine (Tokyo Chemical Industry Co., Ltd.) and 100 ml of methanol (FUJIFILM Wako Pure Chemical Corporation). Then, the reaction vessel was ice-cooled and purged with nitrogen. Under ice cooling, 94.6 g of methyl acrylate (Tokyo Chemical Industry Co., Ltd.) combined with 0.01 g of methoquinone was added dropwise thereto over one hour. After completion of the dropwise addition, the temperature was returned to room temperature and the contents were stirred for 24 hours. Residual methyl acrylate and residual methanol were distilled off under reduced pressure to obtain a polyamide amine precursor.

Subsequently, a glass reaction vessel equipped with a nitrogen inlet tube and a stirrer was charged with 120 g of ethylenediamine and 200 ml of methanol. A solution prepared by dissolving the polyamide amine precursor (1) in 20 ml of methanol was added dropwise to the reaction vessel over 30 minutes. After completion of the dropwise addition, the solution was stirred for three hours. Thereafter, the solution was stirred at room temperature for two to seven days. Then, residual methanol and residual ethylenediamine were distilled off under reduced pressure to obtain a polyamide amine.

### <Production Example 6> Synthesis of glycidyl ether compound having polyethylene oxide structure

A glass reaction vessel equipped with a nitrogen inlet tube and a stirrer was charged with 5.8 g of methoxy polyethylene glycol (ethylene oxide 25 mol) (Nippon Shokubai Co., Ltd.) and 166 ml of tetrahydrofuran (FUJIFILM Wako Pure Chemical Corporation). The contents were stirred under a nitrogen atmosphere while the temperature was raised to 50°C until they form a homogeneous solution. Subsequently, a slurry of 5.27 g of sodium hydride (FUJIFILM Wako Pure Chemical Corporation) in 5 to 10 ml of tetrahydrofuran was added little by little to the reaction vessel, followed by stirring for 30 minutes. Then, 44.3 g of epichlorohydrin (Tokyo Chemical Industry Co., Ltd.) was added dropwise little by little to the reaction vessel and the temperature was raised to 66°C. The contents were heated under reflux for five hours and cooled to room temperature. The reaction was stopped by adding 2 g of water. Thereafter, the tetrahydrofuran in the reaction mixture was distilled off under reduced pressure to obtain a glycidyl ether compound having a polyethylene oxide structure (compound (1)).

### <Production Example 7>

A glass reaction vessel was charged with 1.00 g of the polyamide amine produced in Production Example 5 and 9.20 g of the glycidyl ether compound (compound (1)) having a polyethylene oxide structure. The temperature was raised to 60°C and the contents were stirred for 21 hours. After completion of the reaction, it was confirmed by liquid chromatography that the glycidyl ether compound having a polyethylene oxide structure reacted. Thus, an adduct of the compound (1) with polyamide amine (polymer (5)) was obtained.

### <Production Example 8>

A glass reaction vessel equipped with a nitrogen inlet tube, a reflux condenser, and a stirrer was charged with 70.0 g of polyethyleneimine (Nippon Shokubai Co., Ltd., average molecular weight 600), and was purged with nitrogen at 60°C under stirring. Thereafter, under stirring, 93.7 g of N-(hydroxyethyl)acrylamide (Tokyo Chemical Industry Co., Ltd.) was slowly added dropwise to the reaction vessel from a dropping funnel over 120 minutes. After completion of the dropwise addition, the reaction was continued for nine hours. After completion of the reaction, the complete consumption of the N-(hydroxyethyl)acrylamide was confirmed by liquid chromatography. Thus, a polyethyleneimine-N-(hydroxyethyl)acrylamide adduct was obtained.

Subsequently, an autoclave was charged with 100.9 g of the obtained polyethyleneimine-N-(hydroxyethyl)acrylamide adduct and 2.40 g of potassium hydroxide (FUJIFILM Wako Pure Chemical Corporation) and the temperature was raised to 120°C. The system of the autoclave was purged with nitrogen and then depressurized to 1.8 kPa using a vacuum pump to remove water for one hour from the autoclave. After completion of the removal of water, the pressure in the autoclave was increased to 0.3 MPa with nitrogen, and 199.1 g of ethylene oxide (Air Water Inc.) was gradually introduced into the autoclave for reaction. The reaction product was taken out and 103.0 g of the reaction product and 1.71 g of potassium hydroxide were placed in the autoclave again. Then, 203.1 g of ethylene oxide was reacted by the same procedure. After completion of the reaction, the reaction product was taken out to obtain an adduct (polymer (6)) of ethylene oxide with polyethyleneimine-N-(hydroxyethyl)acrylamide as a polyalkylene oxide-containing compound.

### <Production Example 9>

A glass reaction vessel equipped with a reflux condenser and a stirrer was charged with 8.0 g of the polymer (2). The temperature was raised to 40°C under stirring. After raising the temperature, 1.98 g of methyl acrylate was added dropwise over 30 minutes, followed by stirring at 40°C for 15 hours. After the reaction, the pressure was reduced at 40°C through a trap tube to remove the remaining methyl acrylate. Thereby, a polymer (polymer (7)) was obtained in which the NH groups in the cationic groups to which no linking group was bonded were reduced by Michael addition.

### <Production Example 10>

A glass reaction vessel equipped with a reflux condenser and a stirrer was charged with 10.0 g of the polymer (2). The temperature was raised to 50°C under stirring. After raising the temperature, 0.74 g of acetic anhydride was added dropwise over 34 minutes, followed by stirring at 50°C for six hours. Thereby, a polymer (polymer (8)) was obtained in which the NH groups in the cationic groups to which no linking group was bonded were reduced by acetylation.

### <Production Example 11>

A glass reaction vessel equipped with a reflux condenser and a stirrer was charged with 3.0 g of the polymer (2). The temperature was raised to 50°C under stirring. After raising the temperature, 0.28 g of citric acid was added, followed by stirring at 50°C for three hours. Thereby, a polymer (polymer (9)) was obtained in which the NH groups in the cationic groups to which no linking group was bonded were reduced by neutralization with an acid compound.

### <Production Example 12>

A glass reaction vessel equipped with a nitrogen inlet tube, a reflux condenser, and a stirrer was charged with 1.3 g of polyethyleneimine (Nippon Shokubai Co., Ltd., average molecular weight 600) and 10.1 g of methoxy polyethylene glycol #1000 methacrylate (SHIN-NAKAMURA CHEMICAL Co., Ltd.), and was purged with nitrogen. Thereafter, under stirring, the temperature was raised to 80°C and the reaction was continued for 40 hours. After completion of the reaction, the consumption of the methoxy polyethylene glycol #1000 methacrylate was confirmed by liquid chromatography. Thus, an adduct of methoxy polyethylene glycol #1000 methacrylate with polyethyleneimine (polymer (10)) was obtained as a polyalkylene oxide-containing compound.

### <Production Example 13>

A glass reaction vessel equipped with a nitrogen inlet tube, a reflux condenser, and a stirrer was charged with 4.0 g of the polymer 10. The temperature was raised to 50°C under stirring. After raising the temperature, 1.18 g of acetic anhydride was added dropwise over 55 minutes, followed by stirring at 50°C for five hours. Thereby, a polymer (polymer (11)) was obtained in which the NH groups in the cationic groups to which no linking group was bonded were reduced by acetylation.

### <Production Example 14>

A glass reaction vessel equipped with a nitrogen inlet tube, a reflux condenser, and a stirrer was charged with 7.0 g of polyethyleneimine (Nippon Shokubai Co., Ltd., average molecular weight 600) and 89.78 g of methoxy polyethylene glycol #1000 acrylate (SHIN-NAKAMURA CHEMICAL Co., Ltd.), and was purged with nitrogen. Thereafter, under stirring, the temperature was raised to 80°C and the reaction was continued for six hours. After completion of the reaction, the complete consumption of the methoxy polyethylene glycol #1000 acrylate was confirmed by liquid chromatography. Thus, an adduct of methoxy polyethylene glycol #1000 acrylate with polyethyleneimine (polymer (12)) was obtained as a polyalkylene oxide-containing compound.

### <Production Example 15>

A glass reaction vessel equipped with a nitrogen inlet tube, a reflux condenser, and a stirrer was charged with 10.0 g of the polymer (12). The temperature was raised to 40°C under stirring. After raising the temperature, 0.36 g of methyl acrylate was added dropwise over 30 minutes, followed by stirring at 50°C for 10 hours. After the reaction, the pressure was reduced at 40°C through a trap tube to remove the remaining methyl acrylate. Thereby, a polymer (polymer (13)) was obtained in which the NH groups in the cationic groups to which no linking group was bonded were reduced by Michael addition.

### <Production Example 16>

A glass reaction vessel equipped with a nitrogen inlet tube, a reflux condenser, and a stirrer was charged with 4.0 g of the polymer (13). The temperature was raised to 50°C under stirring. After raising the temperature, 0.33 g of acetic anhydride was added dropwise over 31 minutes, followed by stirring at 50°C for five hours. Thereby, a polymer (polymer (14)) was obtained in which the NH groups in the cationic groups to which no linking group was bonded were reduced by acetylation.

### <Production Example 17>

A glass reaction vessel equipped with a nitrogen inlet tube, a reflux condenser, and a stirrer was charged with 5.0 g of polyethyleneimine (Nippon Shokubai Co., Ltd., average molecular weight 600), and was purged with nitrogen. Thereafter, the temperature was raised to 90°C under stirring. After raising the temperature, 5.86 g of 1,2-epoxyhexane was added dropwise over 84 minutes, followed by stirring for three hours. A polymer (polymer (15)) in which the NH groups in the cationic groups were reduced was obtained.

### <Production Example 18>

A glass reaction vessel equipped with a nitrogen inlet tube, a reflux condenser, and a stirrer was charged with 1.5 g of the polymer (15) and 8.9 g of methoxy polyethylene glycol #1000 acrylate (SHIN-NAKAMURA CHEMICAL Co., Ltd.), and was purged with nitrogen. Thereafter, under stirring, the temperature was raised to 80°C and the reaction was continued for 13 hours. After completion of the reaction, the complete consumption of the methoxy polyethylene glycol #1000 acrylate was confirmed by liquid chromatography. Thus, an adduct of methoxy polyethylene glycol #1000 acrylate with polyethyleneimine (polymer (16)) was obtained as a polyalkylene oxide-containing compound.

### <Production Example 19>

A glass reaction vessel equipped with a nitrogen inlet tube, a reflux condenser, and a stirrer was charged with 4.0 g of the polymer (16). The temperature was raised to 50°C under stirring. After raising the temperature, 0.31 g of acetic anhydride was added dropwise over 30 minutes, followed by stirring at 50°C for five hours. Thereby, a polymer (polymer (17)) was obtained in which the NH groups in the cationic groups to which no linking group was bonded were reduced by acetylation.

### <Production Example 20>

A glass reaction vessel equipped with a nitrogen inlet tube, a reflux condenser, and a stirrer was charged with 1.1 g of polyethyleneimine (Nippon Shokubai Co., Ltd., average molecular weight 600) and 10.6 g of methoxy polyethylene glycol #2000 methacrylate (SHIN-NAKAMURA CHEMICAL Co., Ltd.), and was purged with nitrogen. Thereafter, under stirring, the temperature was raised to 100°C and the reaction was continued for 17 hours. After completion of the reaction, the consumption of the methoxy polyethylene glycol #1000 methacrylate was confirmed by liquid chromatography. Thus, an adduct of methoxy polyethylene glycol #2000 methacrylate with polyethyleneimine (polymer (18)) was obtained as a polyalkylene oxide-containing compound.

### <Production Example 21>

A glass reaction vessel equipped with a nitrogen inlet tube, a reflux condenser, and a stirrer was charged with 4.0 g of the polymer (18). The temperature was raised to 60°C under stirring. After raising the temperature, 1.02 g of acetic anhydride was added dropwise over 45 minutes, followed by stirring at 60°C for five hours. Thereby, a polymer (polymer (19)) was obtained in which the NH groups in the cationic groups to which no linking group was bonded were reduced by acetylation.

### <Production Example 22>

A glass reaction vessel equipped with a nitrogen inlet tube, a reflux condenser, and a stirrer was charged with 0.5 g of polyethyleneimine (Nippon Shokubai Co., Ltd., average molecular weight 600) and 9.5 g of methoxy polyethylene glycol #4000 methacrylate (NOF CORPORATION), and was purged with nitrogen. Thereafter, under stirring, the temperature was raised to 100°C and the reaction was continued for 20 hours. After completion of the reaction, the consumption of the methoxy polyethylene glycol #4000 methacrylate was confirmed by liquid chromatography. Thus, an adduct of methoxy polyethylene glycol #4000 methacrylate with polyethyleneimine (polymer (20)) was obtained as a polyalkylene oxide-containing compound.

### <Production Example 23>

A glass reaction vessel equipped with a nitrogen inlet tube, a reflux condenser, and a stirrer was charged with 4.0 g of the polymer (20). The temperature was raised to 50°C under stirring. After raising the temperature, 0.58 g of acetic anhydride was added dropwise over 27 minutes, followed by stirring at 50°C for five hours. Thereby, a polymer (polymer (21)) was obtained in which the NH groups in the cationic groups to which no linking group was bonded were reduced by acetylation.

### <Production Example 24>

A glass reaction vessel equipped with a nitrogen inlet tube, a reflux condenser, and a stirrer was charged with 1.5 g of polyethyleneimine (Nippon Shokubai Co., Ltd., average molecular weight 1200) and 9.7 g of methoxy polyethylene glycol #1000 methacrylate (SHIN-NAKAMURA CHEMICAL Co., Ltd.), and was purged with nitrogen. Thereafter, under stirring, the temperature was raised to 100°C and the reaction was continued for 30 hours. After completion of the reaction, the consumption of the methoxy polyethylene glycol #1000 methacrylate was confirmed by liquid chromatography. Thus, an adduct of methoxy polyethylene glycol #1000 methacrylate with polyethyleneimine (polymer (22)) was obtained as a polyalkylene oxide-containing compound.

### <Production Example 25>

A glass reaction vessel equipped with a nitrogen inlet tube, a reflux condenser, and a stirrer was charged with 4.0 g of the polymer (22). The temperature was raised to 50°C under stirring. After raising the temperature, 1.43 g of acetic anhydride was added dropwise over 66 minutes, followed by stirring at 50°C for five hours. Thereby, a polymer (polymer (23)) was obtained in which the NH groups in the cationic groups to which no linking group was bonded were reduced by acetylation.

### <Production Example 26>

A glass reaction vessel equipped with a nitrogen inlet tube, a reflux condenser, and a stirrer was charged with 2.5 g of polyethyleneimine (Nippon Shokubai Co., Ltd., average molecular weight 1200) and 48.1 g of methoxy polyethylene glycol #1000 acrylate (SHIN-NAKAMURA CHEMICAL Co., Ltd.), and was purged with nitrogen. Thereafter, under stirring, the temperature was raised to 70°C and the reaction was continued for 10 hours. After completion of the reaction, the consumption of the methoxy polyethylene glycol #1000 acrylate was confirmed by liquid chromatography. Thus, an adduct of methoxy polyethylene glycol #1000 acrylate with polyethyleneimine (polymer (24)) was obtained as a polyalkylene oxide-containing compound.

### <Production Example 27>

A glass reaction vessel equipped with a nitrogen inlet tube, a reflux condenser, and a stirrer was charged with 15.0 g of the polymer (24). The temperature was raised to 50°C under stirring. After raising the temperature, 1.34 g of acetic anhydride was added dropwise over 61 minutes, followed by stirring at 50°C for five hours. Thereby, a polymer (polymer (25)) was obtained in which the NH groups in the cationic groups to which no linking group was bonded were reduced by acetylation.

### <Production Example 28>

An autoclave equipped with a thermometer, a pressure gauge, and a stirrer was charged with 504.0 g of methoxy poly(n = 25)ethyleneglycol and 0.50 g of potassium hydroxide (FUJIFILM Wako Pure Chemical Corporation). The temperature was raised to 100°C under stirring. The system of the autoclave was purged with nitrogen and then depressurized to 1.2 kPa using a vacuum pump to remove water for one hour from the autoclave. After completion of the removal of water, the pressure in the autoclave was increased to 0.3 MPa with nitrogen, 96.4 g of 1,2-butylene oxide (Tokyo Chemical Industry Co., Ltd.) was gradually introduced over 120 minutes. The temperature was further maintained at 100°C for 40 minutes. Subsequently, the temperature of the reaction system was raised to 120°C and maintained for 150 minutes for ripening. Then, the pressure in the system was returned to atmospheric pressure. Further, a cold trap and a vacuum pump were attached to the autoclave while the temperature in the system was kept at 60°C and unreacted butylene oxide was distilled off under reduced pressure. The remaining product was collected to obtain methoxy poly(n = 25)ethyleneglycol-poly(n = 3)butyleneglycol (compound (2)).

A jacketed glass reactor (internal volume: 1 L) equipped with a thermometer, a stirrer, a water separator, a reflux condenser serving as a condenser, and a nitrogen inlet tube was charged with 538.93 g of the compound (2), 35.48 g of acrylic acid, 19.06 g of p-toluenesulfonic acid monohydrate, 0.14 g of phenothiazine, 0.03 g of 4-hydroxy TEMPO, and 57.44 g of cyclohexane. An esterification reaction was performed at a reaction temperature of 115°C. It was confirmed that the esterification rate reached 97% in 60 hours. To 655.75 g of the obtained esterified reaction solution were added 8.61 g of a 49% aqueous sodium hydroxide solution and 98.50 g of water to neutralize the p-toluenesulfonic acid. The temperature was raised to 105°C and cyclohexane was removed by azeotropic distillation with water. When the internal temperature of the jacketed glass reactor reached 98°C, nitrogen was introduced into the reaction liquid in the reactor to purge the dissolved cyclohexane therefrom. Then, water for adjustment was added to obtain an 80% aqueous esterified product solution (compound (3)).

### <Production Example 29>

A glass reaction vessel equipped with a nitrogen inlet tube, a reflux condenser, and a stirrer was charged with 0.90 g of polyethyleneimine (Nippon Shokubai Co., Ltd., average molecular weight 600) and 9.10 g of the compound (3), and was purged with nitrogen. Thereafter, under stirring, the temperature was raised to 40°C and the reaction was continued for eight hours. After completion of the reaction, the consumption of the esterified product was confirmed by liquid chromatography. Thereby, an adduct of methoxy poly(n = 25)ethyleneglycol-poly(n = 3)butyleneglycol acrylate with polyethyleneimine (polymer (26)) was obtained.

### <Production Example 30>

A glass reaction vessel equipped with a nitrogen inlet tube, a reflux condenser, and a stirrer was charged with 0.47 g of polyethyleneimine (Nippon Shokubai Co., Ltd., average molecular weight 600) and 9.52 g of the compound (3), and was purged with nitrogen. Thereafter, under stirring, the temperature was raised to 40°C and the reaction was continued for eight hours. After completion of the reaction, the consumption of the esterified product was confirmed by liquid chromatography. Thereby, an adduct of methoxy poly(n = 25)ethyleneglycol-poly(n = 3)butyleneglycol acrylate with polyethyleneimine (polymer (27)) was obtained.

### <Production Example 31>

A glass reaction vessel equipped with a nitrogen inlet tube, a reflux condenser, and a stirrer was charged with 0.32 g of polyethyleneimine (Nippon Shokubai Co., Ltd., average molecular weight 600) and 9.68 g of the compound (3), and was purged with nitrogen. Thereafter, under stirring, the temperature was raised to 40°C and the reaction was continued for eight hours. After completion of the reaction, the consumption of the esterified product was confirmed by liquid chromatography. Thereby, an adduct of methoxpoly(n = 25)ethyleneglycol-poly(n = 3)butyleneglycol acrylate with polyethyleneimine (polymer (28)) was obtained.

### <Production Example 32>

An autoclave equipped with a thermometer, a pressure gauge, and a stirrer was charged with 730.3 g of methoxy poly(n = 25)ethyleneglycol and 0.64 g of potassium hydroxide (FUJIFILM Wako Pure Chemical Corporation). The temperature was raised to 120°C under stirring. The system of the autoclave was purged with nitrogen and then depressurized to 1.2 kPa using a vacuum pump to remove water for one hour from the autoclave. After completion of the removal of water, the pressure in the autoclave was increased to 0.3 MPa with nitrogen, 69.7 g of 1,2-butylene oxide (Tokyo Chemical Industry Co., Ltd.) was gradually introduced into the autoclave over 80 minutes. The temperature was further maintained at 120°C for 300 minutes for ripening. Then, the pressure in the system was returned to atmospheric pressure. Further, a cold trap and a vacuum pump were connected while the temperature in the system was kept at 60°C and unreacted butylene oxide was distilled off under reduced pressure. The remaining product was collected to obtain methoxy poly(n = 25)ethyleneglycol-poly(n = 1.5)butyleneglycol (compound (4)).

A jacketed glass reaction vessel (internal volume: 1 L) equipped with a thermometer, a stirrer, a water separator, a reflux condenser serving as a condenser, and a nitrogen inlet tube was charged with 691.40 g of the compound (4), 60.19 g of acrylic acid, 35.62 g of a 70% aqueous solution of p-toluenesulfonic acid monohydrate, 0.19 g of phenothiazine, 0.04 g of 4-hydroxy TEMPO, and 75.16 g of cyclohexane. An esterification reaction was performed at a reaction temperature of 110°C. It was confirmed that the esterification rate reached 89% in 76 hours. To 862.60 g of the obtained esterified reaction solution were added 13.02 g of a 48% aqueous sodium hydroxide solution and 28.18 g of water to neutralize the p-toluenesulfonic acid. The temperature was raised to 105°C and cyclohexane was removed by azeotropic distillation with water. When the internal temperature of the jacketed glass reactor reached 98°C, nitrogen was introduced into the reaction liquid in the reactor to purge the dissolved cyclohexane. Then, water for adjustment was added to obtain a 90% aqueous esterified product solution (compound (5)).

### <Production Example 33>

An autoclave equipped with a thermometer, a pressure gauge, and a stirrer was charged with 664.8 g of methoxy poly(n = 25)ethyleneglycol and 0.60 g of potassium hydroxide (FUJIFILM Wako Pure Chemical Corporation). The temperature was raised to 120°C under stirring. The system of the autoclave was purged with nitrogen and then depressurized to 1.2 kPa using a vacuum pump to remove water for one hour from the autoclave. After completion of the removal of water, the pressure in the autoclave was increased to 0.3 MPa with nitrogen, 85.2 g of propylene oxide (FUJIFILM Wako Pure Chemical Corporation) was gradually introduced into the autoclave over 60 minutes. The temperature was further maintained at 120°C of 240 minutes for ripening. Then, the pressure in the system was returned to atmospheric pressure. Further, a cold trap and a vacuum pump were connected while the temperature in the system was kept at 60°C and unreacted propylene oxide was distilled off under reduced pressure. The remaining product was collected to obtain methoxy poly(n = 25)ethyleneglycol-poly(n = 2.5)propyleneglycol (compound (6)).

A jacketed glass reaction vessel (internal volume: 1 L) equipped with a thermometer, a stirrer, a water separator, a reflux condenser serving as a condenser, and a nitrogen inlet tube was charged with 691.30 g of the compound (6), 58.56 g of acrylic acid, 35.54 g of a 70% aqueous solution of p-toluenesulfonic acid monohydrate, 0.19 g of phenothiazine, 0.04 g of 4-hydroxy TEMPO, and 74.99 g of cyclohexane. An esterification reaction was performed at a reaction temperature of 110°C. It was confirmed that the esterification rate reached 90% in 76 hours. To 860.61 g of the obtained esterified reaction solution were added 12.80 g of a 48% aqueous sodium hydroxide solution and 30.88 g of water to neutralize the p-toluenesulfonic acid. The temperature was raised to 105°C and cyclohexane was removed by azeotropic distillation with water. When the internal temperature of the jacketed glass reactor reached 98°C, nitrogen was introduced into the reaction liquid in the reactor to purge the dissolved cyclohexane. Then, water for adjustment was added to obtain an 90% aqueous esterified product solution (compound (7)).

### <Production Example 34>

An autoclave equipped with a thermometer, a pressure gauge, and a stirrer was charged with 597.0 g of methoxy poly(n = 25)ethyleneglycol and 0.62 g of potassium hydroxide (FUJIFILM Wako Pure Chemical Corporation). The temperature was raised to 120°C under stirring. The system of the autoclave was purged with nitrogen and then depressurized to 1.2 kPa using a vacuum pump to remove water for one hour from the autoclave. After completion of the removal of water, the pressure in the autoclave was increased to 0.3 MPa with nitrogen, 152.9 g of propylene oxide (FUJIFILM Wako Pure Chemical Corporation) was gradually introduced into the autoclave over 120 minutes. The temperature was further maintained at 120°C for 180 minutes for ripening. Then, the pressure in the system was returned to atmospheric pressure. Further, a cold trap and a vacuum pump were connected while the temperature in the system was kept at 60°C and unreacted propylene oxide was distilled off under reduced pressure. The remaining product was collected to obtain methoxy poly(n = 25)ethyleneglycol-poly(n = 5.0)propyleneglycol (compound (8)).

A jacketed glass reaction vessel (internal volume: 1 L) equipped with a thermometer, a stirrer, a water separator, a reflux condenser serving as a condenser, and a nitrogen inlet tube was charged with 694.08 g of the compound (8), 52.88 g of acrylic acid, 35.40 g of a 70% aqueous solution of p-toluenesulfonic acid monohydrate, 0.19 g of phenothiazine, 0.04 g of 4-hydroxy TEMPO, and 74.70 g of cyclohexane. An esterification reaction was performed at a reaction temperature of 110°C. It was confirmed that the esterification rate reached 94% in 76 hours. To 857.29 g of the obtained esterified reaction solution were added 12.52 g of a 48% aqueous sodium hydroxide solution and 31.87 g of water to neutralize the p-toluenesulfonic acid. The temperature was raised to 105°C and cyclohexane was removed by azeotropic distillation with water. When the internal temperature of the jacketed glass reactor reached 98°C, nitrogen was introduced into the reaction liquid in the reactor to purge the dissolved cyclohexane. Then, water for adjustment was added to obtain a 90% aqueous esterified product solution (compound (9)).

### <Production Example 35>

A glass reaction vessel equipped with a nitrogen inlet tube, a reflux condenser, and a stirrer was charged with 1.10 g of polyethyleneimine (Nippon Shokubai Co., Ltd., average molecular weight 600) and 8.83 g of the compound (5), and was purged with nitrogen. Thereafter, under stirring, the temperature was raised to 40°C and the reaction was continued for two hours. After completion of the reaction, the consumption of the esterified product compound (5) was confirmed by liquid chromatography. Thus, an adduct of methoxy poly(n = 25)ethyleneglycol-poly(n = 1.5)butyleneglycol acrylate with polyethyleneimine (polymer (29)) was obtained.

A glass reaction vessel equipped with a nitrogen inlet tube, a reflux condenser, and a stirrer was charged with 3.02 g of the polymer (29). The temperature was raised to 50°C under stirring. After raising the temperature, 0.70 g of acetic anhydride was added dropwise over 10 minutes, followed by stirring at 50°C for five hours. Thereby, a polymer (polymer (30)) was obtained in which the NH groups in the cationic groups to which no linking group was bonded were reduced by acetylation. The resulting reaction solution contained 69% by weight of the polymer (30). The polymer (30) had a number average molecular weight of 6640 as determined by GPC analysis.

A glass reaction vessel equipped with a nitrogen inlet tube, a reflux condenser, and a stirrer was charged with 2.01 g of the polymer (29). The temperature was raised to 50°C under stirring. After raising the temperature, 0.78 g of citric acid (FUJIFILM Wako Pure Chemical Corporation) was added, followed by stirring at 50°C for two hours. Thereby, a polymer (polymer (31)) was obtained in which the NH groups in the cationic groups to which no linking group was bonded were reduced by neutralization with an acid compound. The resulting reaction solution contained 55% by weight of the polymer (31). The polymer (31) had a number average molecular weight of 6490 as determined by GPC analysis.

### <Production Example 36>

A glass reaction vessel equipped with a nitrogen inlet tube, a reflux condenser, and a stirrer was charged with 0.61 g of polyethyleneimine (Nippon Shokubai Co., Ltd., average molecular weight 600) and 9.65 g of the compound (5), and was purged with nitrogen. Thereafter, under stirring, the temperature was raised to 40°C and the reaction was continued for five hours. After completion of the reaction, the consumption of the esterified product compound (5) was confirmed by liquid chromatography. Thus, an adduct of methoxy poly(n = 25)ethyleneglycol-poly(n = 1.5)butyleneglycol acrylate with polyethyleneimine (polymer (32)) was obtained.

A glass reaction vessel equipped with a nitrogen inlet tube, a reflux condenser, and a stirrer was charged with 3.02 g of the polymer (32). The temperature was raised to 50°C under stirring. After raising the temperature, 0.28 g of acetic anhydride was added dropwise over 10 minutes, followed by stirring at 50°C for five hours. Thereby, a polymer (polymer (33)) was obtained in which the NH groups in the cationic groups to which no linking group was bonded were reduced by acetylation. The resulting reaction solution contained 71% by weight of the polymer (33). The polymer (33) had a number average molecular weight of 7510 as determined by GPC analysis.

A glass reaction vessel equipped with a nitrogen inlet tube, a reflux condenser, and a stirrer was charged with 2.00 g of the polymer (32). The temperature was raised to 50°C under stirring. After raising the temperature, 0.31 g of citric acid (FUJIFILM Wako Pure Chemical Corporation) was added, followed by stirring at 50°C for two hours. Thereby, a polymer (polymer (34)) was obtained in which the NH groups in the cationic groups to which no linking group was bonded were reduced by neutralization with an acid compound. The resulting reaction solution contained 64% by weight of the polymer (34). The polymer (34) had a number average molecular weight of 7460 as determined by GPC analysis.

### <Production Example 37>

A glass reaction vessel equipped with a nitrogen inlet tube, a reflux condenser, and a stirrer was charged with 0.40 g of polyethyleneimine (Nippon Shokubai Co., Ltd., average molecular weight 600) and 9.64 g of the compound (5), and was purged with nitrogen. Thereafter, under stirring, the temperature was raised to 40°C and the reaction was continued for 12 hours. After completion of the reaction, the consumption of the esterified product compound (5) was confirmed by liquid chromatography. Thus, an adduct of methoxy poly(n = 25)ethyleneglycol-poly(n = 1.5)butyleneglycol acrylate with polyethyleneimine (polymer (35)) was obtained.

A glass reaction vessel equipped with a nitrogen inlet tube, a reflux condenser, and a stirrer was charged with 3.00 g of the polymer (35). The temperature was raised to 50°C under stirring. After raising the temperature, 0.11 g of acetic anhydride was added dropwise over 10 minutes, followed by stirring at 50°C for five hours. Thereby, a polymer (polymer (36)) was obtained in which the NH groups in the cationic groups to which no linking group was bonded were reduced by acetylation. The resulting reaction solution contained 64% by weight of the polymer (36). The polymer (36) had a number average molecular weight of 8630 as determined by GPC analysis.

A glass reaction vessel equipped with a nitrogen inlet tube, a reflux condenser, and a stirrer was charged with 2.00 g of the polymer (35). The temperature was raised to 50°C under stirring. After raising the temperature, 0.13 g of citric acid (FUJIFILM Wako Pure Chemical Corporation) was added, followed by stirring at 50°C for two hours. Thereby, a polymer (polymer (37)) was obtained in which the NH groups in the cationic groups to which no linking group was bonded were reduced by neutralization with an acid compound. The resulting reaction solution contained 67% by weight of the polymer (37). The polymer (37) had a number average molecular weight of 8440 as determined by GPC analysis.

### <Production Example 38>

A glass reaction vessel equipped with a nitrogen inlet tube, a reflux condenser, and a stirrer was charged with 1.50 g of polyethyleneimine (Nippon Shokubai Co., Ltd., average molecular weight 600) and 10.67 g of the compound (7), and was purged with nitrogen. Thereafter, under stirring, the temperature was raised to 40°C and the reaction was continued for three hours. After completion of the reaction, the consumption of the esterified product of the compound (7) was confirmed by liquid chromatography. Thus, an adduct of methoxy poly(n = 25)ethyleneglycol-poly(n = 2.5)propyleneglycol acrylate with polyethyleneimine (polymer (38)) was obtained.

A glass reaction vessel equipped with a nitrogen inlet tube, a reflux condenser, and a stirrer was charged with 4.00 g of the polymer (38). The temperature was raised to 50°C under stirring. After raising the temperature, 1.06 g of acetic anhydride was added dropwise over 10 minutes, followed by stirring at 50°C for five hours. Thereby, a polymer (polymer (39)) was obtained in which the NH groups in the cationic groups to which no linking group was bonded were reduced by acetylation. The resulting reaction solution contained 72% by weight of the polymer (39). The polymer (39) had a number average molecular weight of 6530 as determined by GPC analysis.

A glass reaction vessel equipped with a nitrogen inlet tube, a reflux condenser, and a stirrer was charged with 2.51 g of the polymer (38). The temperature was raised to 50°C under stirring. After raising the temperature, 1.12 g of citric acid (FUJIFILM Wako Pure Chemical Corporation) was added, followed by stirring at 50°C for two hours. Thereby, a polymer (polymer (40)) was obtained in which the NH groups in the cationic groups to which no linking group was bonded were reduced by neutralization with an acid compound. The resulting reaction solution contained 56% by weight of the polymer (40). The polymer (40) had a number average molecular weight of 6360 as determined by GPC analysis.

### <Production Example 39>

A glass reaction vessel equipped with a nitrogen inlet tube, a reflux condenser, and a stirrer was charged with 0.80 g of polyethyleneimine (Nippon Shokubai Co., Ltd., average molecular weight 600) and 11.37 g of the compound (7), and was purged with nitrogen. Thereafter, under stirring, the temperature was raised to 40°C and the reaction was continued for five hours. After completion of the reaction, the consumption of the esterified product of the compound (7) was confirmed by liquid chromatography. Thus, an adduct of methoxy poly(n = 25)ethyleneglycol-poly(n = 2.5)propyleneglycol acrylate with polyethyleneimine (polymer (41)) was obtained.

A glass reaction vessel equipped with a nitrogen inlet tube, a reflux condenser, and a stirrer was charged with 4.01 g of the polymer (41). The temperature was raised to 50°C under stirring. After raising the temperature, 0.42 g of acetic anhydride was added dropwise over 10 minutes, followed by stirring at 50°C for five hours. Thereby, a polymer (polymer (42)) was obtained in which the NH groups in the cationic groups to which no linking group was bonded were reduced by acetylation. The resulting reaction solution contained 76% by weight of the polymer (42). The polymer (42) had a number average molecular weight of 7300 as determined by GPC analysis.

A glass reaction vessel equipped with a nitrogen inlet tube, a reflux condenser, and a stirrer was charged with 2.51 g of the polymer (41). The temperature was raised to 50°C under stirring. After raising the temperature, 0.46 g of citric acid (FUJIFILM Wako Pure Chemical Corporation) was added, followed by stirring at 50°C for two hours. Thereby, a polymer (polymer (43)) was obtained in which the NH groups in the cationic groups to which no linking group was bonded were reduced by neutralization with an acid compound. The resulting reaction solution contained 68% by weight of the polymer (43). The polymer (43) had a number average molecular weight of 7070 as determined by GPC analysis.

### <Production Example 40>

A glass reaction vessel equipped with a nitrogen inlet tube, a reflux condenser, and a stirrer was charged with 0.56 g of polyethyleneimine (Nippon Shokubai Co., Ltd., average molecular weight 600) and 11.73 g of the compound (7), and was purged with nitrogen. Thereafter, under stirring, the temperature was raised to 40°C and the reaction was continued for eight hours. After completion of the reaction, the consumption of the esterified product of the compound (7) was confirmed by liquid chromatography. Thus, an adduct of methoxy poly(n = 25)ethyleneglycol-poly(n = 2.5)propyleneglycol acrylate with polyethyleneimine (polymer (44)) was obtained.

A glass reaction vessel equipped with a nitrogen inlet tube, a reflux condenser, and a stirrer was charged with 4.00 g of the polymer (44). The temperature was raised to 50°C under stirring. After raising the temperature, 0.21 g of acetic anhydride was added dropwise over 10 minutes, followed by stirring at 50°C for five hours. Thereby, a polymer (polymer (45)) was obtained in which the NH groups in the cationic groups to which no linking group was bonded were reduced by acetylation. The resulting reaction solution contained 77% by weight of the polymer (45). The polymer (45) had a number average molecular weight of 8150 as determined by GPC analysis.

A glass reaction vessel equipped with a nitrogen inlet tube, a reflux condenser, and a stirrer was charged with 2.53 g of the polymer (44). The temperature was raised to 50°C under stirring. After raising the temperature, 0.22 g of citric acid (FUJIFILM Wako Pure Chemical Corporation) was added, followed by stirring at 50°C for two hours. Thereby, a polymer (polymer (46)) was obtained in which the NH groups in the cationic groups to which no linking group was bonded were reduced by neutralization with an acid compound. The resulting reaction solution contained 73% by weight of the polymer (46). The polymer (46) had a number average molecular weight of 7920 as determined by GPC analysis.

### <Production Example 41>

A glass reaction vessel equipped with a nitrogen inlet tube, a reflux condenser, and a stirrer was charged with 1.30 g of polyethyleneimine (Nippon Shokubai Co., Ltd., average molecular weight 600) and 11.25 g of the compound (9), and was purged with nitrogen. Thereafter, under stirring, the temperature was raised to 40°C and the reaction was continued for two hours. After completion of the reaction, the consumption of the esterified product of the compound (9) was confirmed by liquid chromatography. Thus, an adduct of methoxy poly(n = 25)ethyleneglycol-poly(n = 5)propyleneglycol acrylate with polyethyleneimine (polymer (47)) was obtained.

A glass reaction vessel equipped with a nitrogen inlet tube, a reflux condenser, and a stirrer was charged with 4.00 g of the polymer (47). The temperature was raised to 50°C under stirring. After raising the temperature, 0.89 g of acetic anhydride was added dropwise over 10 minutes, followed by stirring at 50°C for five hours. Thereby, a polymer (polymer (48)) was obtained in which the NH groups in the cationic groups to which no linking group was bonded were reduced by acetylation. The resulting reaction solution contained 65% by weight of the polymer (48). The polymer (48) had a number average molecular weight of 7030 as determined by GPC analysis.

A glass reaction vessel equipped with a nitrogen inlet tube, a reflux condenser, and a stirrer was charged with 2.50 g of the polymer (47). The temperature was raised to 50°C under stirring. After raising the temperature, 0.94 g of citric acid (FUJIFILM Wako Pure Chemical Corporation) was added, followed by stirring at 50°C for two hours. Thereby, a polymer (polymer (49)) was obtained in which the NH groups in the cationic groups to which no linking group was bonded were reduced by neutralization with an acid compound. The resulting reaction solution contained 52% by weight of the polymer (49). The polymer (49) had a number average molecular weight of 6960 as determined by GPC analysis.

### <Production Example 42>

A glass reaction vessel equipped with a nitrogen inlet tube, a reflux condenser, and a stirrer was charged with 0.70 g of polyethyleneimine (Nippon Shokubai Co., Ltd., average molecular weight 600) and 12.14 g of the compound (9), and was purged with nitrogen. Thereafter, under stirring, the temperature was raised to 40°C and the reaction was continued for two hours. After completion of the reaction, the consumption of the esterified product of the compound (9) was confirmed by liquid chromatography. Thus, an adduct of methoxy poly(n = 25)ethyleneglycol-poly(n = 5)propyleneglycol acrylate with polyethyleneimine (polymer (50)) was obtained.

A glass reaction vessel equipped with a nitrogen inlet tube, a reflux condenser, and a stirrer was charged with 4.00 g of the polymer (50). The temperature was raised to 50°C under stirring. After raising the temperature, 0.37 g of acetic anhydride was added dropwise over 10 minutes, followed by stirring at 50°C for five hours. Thereby, a polymer (polymer (51)) was obtained in which the NH groups in the cationic groups to which no linking group was bonded were reduced by acetylation. The resulting reaction solution contained 67% by weight of the polymer (51). The polymer (51) had a number average molecular weight of 7750 as determined by GPC analysis.

A glass reaction vessel equipped with a nitrogen inlet tube, a reflux condenser, and a stirrer was charged with 2.50 g of the polymer (50). The temperature was raised to 50°C under stirring. After raising the temperature, 0.39 g of citric acid (FUJIFILM Wako Pure Chemical Corporation) was added, followed by stirring at 50°C for two hours. Thereby, a polymer (polymer (52)) was obtained in which the NH groups in the cationic groups to which no linking group was bonded were reduced by neutralization with an acid compound. The resulting reaction solution contained 61% by weight of the polymer (52). The polymer (52) had a number average molecular weight of 7640 as determined by GPC analysis.

### <Production Example 43>

A glass reaction vessel equipped with a nitrogen inlet tube, a reflux condenser, and a stirrer was charged with 0.44 g of polyethyleneimine (Nippon Shokubai Co., Ltd., average molecular weight 600) and 11.71 g of the compound (9), and was purged with nitrogen. Thereafter, under stirring, the temperature was raised to 50°C and the reaction was continued for seven hours. After completion of the reaction, the consumption of the esterified product of the compound (9) was confirmed by liquid chromatography. Thus, an adduct of methoxy poly(n = 25)ethyleneglycol-poly(n = 5)propyleneglycol acrylate with polyethyleneimine (polymer (53)) was obtained.

A glass reaction vessel equipped with a nitrogen inlet tube, a reflux condenser, and a stirrer was charged with 4.00 g of the polymer (53). The temperature was raised to 50°C under stirring. After raising the temperature, 0.16 g of acetic anhydride was added dropwise over 10 minutes, followed by stirring at 50°C for five hours. Thereby, a polymer (polymer (54)) was obtained in which the NH groups in the cationic groups to which no linking group was bonded were reduced by acetylation. The resulting reaction solution contained 69% by weight of the polymer (54). The polymer (54) had a number average molecular weight of 8970 as determined by GPC analysis.

A glass reaction vessel equipped with a nitrogen inlet tube, a reflux condenser, and a stirrer was charged with 2.50 g of the polymer (53). The temperature was raised to 50°C under stirring. After raising the temperature, 0.16 g of citric acid (FUJIFILM Wako Pure Chemical Corporation) was added, followed by stirring at 50°C for two hours. Thereby, a polymer (polymer (55)) was obtained in which the NH groups in the cationic groups to which no linking group was bonded were reduced by neutralization with an acid compound. The resulting reaction solution contained 66% by weight of the polymer (55). The polymer (55) had a number average molecular weight of 8650 as determined by GPC analysis.

### <Comparative composition (1)>

A comparative composition (1) was prepared by mixing 0.90 g of polyethyleneimine (Nippon Shokubai Co., Ltd., average molecular weight 600) and 20.6 g of methoxy polyethylene glycol #1000 acrylate (SHIN-NAKAMURA CHEMICAL Co., Ltd.).

### <Comparative composition (2)>

A comparative composition (2) was prepared by mixing 0.10 g of polyethyleneimine (Nippon Shokubai Co., Ltd., average molecular weight 600) and 2.29 g of polyethylene glycol monomethyl ether 1000 (Tokyo Chemical Industry Co., Ltd.).

### <Measurement of particulate clay dispersing ability>

A clay dispersing ability was measured by the following technique. First, an aqueous sodium bicarbonate solution, a 3% linear sodium alkylbenzene sulfonate aqueous solution (hereinafter referred to as a 3% LAS aqueous solution), and a 0.1% aqueous polymer (polyalkylene oxide-containing compound) solution were prepared. Pure water was added to 15.4 g of sodium bicarbonate and 100 g of 0.1 M hydrochloric acid to prepare 1000 g of the aqueous sodium bicarbonate solution. Linear sodium alkylbenzene sulfonate was diluted with an appropriate amount of water to prepare the solution having a solid content of 3% by mass. The 0.1% polyalkylene oxide-containing compound aqueous solution was prepared as follows: a polyalkylene oxide-containing compound was diluted with an appropriate amount of water to prepare the solution having a solid content of 0.1% by mass. To 0.5 g of the aqueous sodium bicarbonate solution in a beaker was added 15°dH hard water (prepared with calcium chloride and magnesium chloride, the mass ratio of Ca/Mg = 3) at 25°C to prepare 100 g of a solution. To the solution was added 0.025 g of Japanese Shigaraki clay, followed by stirring for 10 minutes. Then, ultrasonic waves were applied thereto for 10 minutes to prepare a clay dispersion. Thereafter, to the dispersion under stirring were added 0.83 g of the 3% LAS aqueous solution and 0.83 g of the 0.1% aqueous polymer solution, followed by stirring for 10 minutes. The thus obtained clay dispersion was transferred to a 100-mL colorimetric tube and allowed to stand for one hour. Then, 4 mL of the dispersion was collected from the middle of the colorimetric tube. The collected liquid was placed in a 1-cm quartz cell, and subjected to measurement of absorbance at a wavelength of 420 nm using a spectrophotometer (measurement device: UV-1800 available from Shimadzu Corporation). The higher the absorbance, the more the clay particles are dispersed.

### <Measurement of anti-redeposition ability>

Fabrics were subjected to three cycles of a cleaning process including a washing step, a rinsing step, and a drying step, which were described below, performed in this order, and the anti-redeposition ability of a polymer with respect to the fabrics was measured. First, soiled fabrics to be used in the washing step were prepared.

Preparation of fabrics soiled with clay:
Pure water was added to 50 g of Namikoshi (moderately sanded) red clay to make a total of 500 g and the contents were stirred to prepare clay water. While stirring the prepared clay water, a 12 × 18 cm cotton fabric (Kanakin No. 3) was passed through the clay water three times, and then dried at 80°C for 10 minutes. The dried fabric soiled with clay was beaten until the clay dust was no longer visible, and the surface of the fabric was brushed with a sponge. The thus obtained fabric soiled with clay was cut into 5 × 5 cm pieces (hereinafter referred to as clay-soiled fabrics) and used in the cleaning process.

### Washing step

The following two types of cotton fabrics were used as fabrics to be cleaned.

Cotton fabrics (1): Five cotton knitted fabrics (Tanigashira Shoten) each having a size of 5 cm × 5 cm were prepared as fabrics for determination of redeposition.

Cotton fabric (2): Cotton fabric available from Testfabrics was prepared such that the total amount of the cotton fabrics (1) and (2) was 30 g.

As surfactants, the 3% LAS aqueous solution, 0.1% by mass aqueous solutions of the polyalkylene oxide-containing compounds obtained in the production examples, and a 0.1% by mass aqueous solution of the comparative composition (1) were prepared.

The following items were introduced into a Tergot-o-meter (Daiei Kagaku Seiki Mfg. Co., Ltd., product name: TM-4): 900 mL of 15°dH hard water having a temperature of 25°C (prepared with calcium chloride and magnesium chloride, the mass ratio of Ca/Mg = 3); 7.5 g of the 3% LAS aqueous solution; 7.5 g of any one of the 0.1% aqueous solutions of the polymers (1) to (8) as polyalkylene oxide-containing compounds and the 0.1% aqueous solution of the comparative composition (1); the five clay-stained fabrics each having a size of 5 × 5 cm prepared above; and the cotton fabrics (1) and (2). Thereafter, washing was performed at a stirring speed of 120 rpm and 25°C for 10 minutes.

### Rinsing step

After the washing step, the fabrics to be cleaned and the clay-stained fabrics were spun for 1.5 minutes, and then, 900 mL of 15°dH hard water having a temperature of 25°C was added thereto. These fabrics were rinsed at 120 rpm and 25°C for three minutes. This operation (spinning and rinsing) was repeated twice.

### Drying step

The rinsed fabrics to be washed and the rinsed clay-stained fabrics were spun for 1.5 minutes. Then, only the cotton fabrics (1) were taken out and each fabric was sandwiched between other cotton fabrics and dried with an iron.

The cotton fabrics (1) subjected to one to three cycles of the cleaning process and the cotton fabrics (1) before the cleaning process were subjected to measurement of reflectance (Z value) using a reflectometer (spectroscopic chromometer available from Nippon Denshoku Industries Co., Ltd., product name: SE6000). The anti-redeposition rate was calculated from the following formula.

Anti-redeposition rate = (Z value of cotton fabrics (1) subjected to one to three cycles of cleaning process)/(Z value of cotton fabrics (1) before cleaning process) × 100

Table 1 shows the anti-redeposition rates of the cotton fabrics (1) subjected to three cycles of the cleaning process.

### <Measurement of anti-redeposition ability (2)>

Fabrics were subjected to a cleaning process including a washing step, a rinsing step, and a drying step, which were described below, performed in this order, and the anti-redeposition ability of a polymer with respect to the fabrics was measured.

### Washing step

The following two types of cotton fabrics were used as fabrics to be cleaned.

Cotton fabrics (1): Five cotton knitted fabrics (Tanigashira Shoten) each having a size of 5 cm × 5 cm were prepared as fabrics for determination of redeposition.

Cotton fabric (2): Cotton fabric available from Testfabrics was prepared such that the total amount of the cotton fabrics (1) and (2) was 30 g.

As surfactants, the 3% LAS aqueous solution, 0.1% by mass aqueous solutions of the polyalkylene oxide-containing compounds obtained in the production examples, and a 0.1% by mass aqueous solution of the comparative composition (2) were prepared.

A 1-L pot of a Tergot-o-meter (Daiei Kagaku Seiki Mfg. Co., Ltd., product name: TM-4) was charged with 876 g of pure water at 25°C, followed by adding 4.5 g of a 1.5% by mass aqueous sodium hydrogen carbonate solution. The contents were stirred. Thereafter, the following items were introduced into the pot: 4.5 g of 3000°dH hard water (prepared with calcium chloride and magnesium chloride, the mass ratio of Ca/Mg = 3); 7.5 g of the 3% LAS aqueous solution; 7.5 g of any one of the 0.1% aqueous solutions of the polymers (10) to (19) as polyalkylene oxide-containing compounds and the 0.1% by mass aqueous solution of the comparative composition (2), followed by stirring. Thereafter, 0.9 g of Namikoshi (moderately sanded) red clay was added thereto and stirred. Thereafter, the cotton fabrics (1) and (2) were added thereto and washed at a stirring speed of 120 rpm and 25°C for 10 minutes.

### Rinsing step

The washed fabrics to be cleaned were spun for 1.5 minutes, and then, 900 mL of 15°dH hard water having a temperature of 25°C was added thereto. The fabrics were rinsed at 120 rpm and 25°C for three minutes. This operation (spinning and rinsing) was repeated twice.

### Drying step

The rinsed fabrics were spun for 1.5 minutes. Then, only the cotton fabrics (1) were taken out and each fabric was sandwiched between other cotton fabrics and dried with an iron.

The cotton fabrics (1) subjected to the cleaning process and the cotton fabrics (1) before the cleaning process were subjected to measurement of reflectance (Z value) using a reflectometer (spectroscopic chromometer available from Nippon Denshoku Industries Co., Ltd., product name: SE6000). The anti-redeposition rate was calculated from the following formula.

Anti-redeposition rate = (Z value of cotton fabrics (1) subjected to cleaning process)/(Z value of cotton fabrics (1) before cleaning process) × 100

Table 2 shows the anti-redeposition rates (2) of the cotton fabrics (1) subjected to the cleaning process.

### <Biodegradability test>

The obtained polyalkylene oxide-containing compounds were subjected to OECD 301F biodegradability test.

### Preparation of medium

Medium stock solutions A to D were prepared by the following technique.

Solution A: 0.850 g of potassium dihydrogen phosphate (KH₂PO₄), 2.175 g of dipotassium hydrogen phosphate (K₂HPO₄), 6.7217 g of disodium hydrogen phosphate dodecahydrate (Na₂HPO₄·12H₂O), and 0.050 g of ammonium chloride (NH₄Cl) were weighed into a 50-ml sampling bottle and dissolved in an appropriate amount of water; the solution was transferred to a 100-ml volumetric flask; and water was added up to the marked line.

Solution B: 3.640 g of calcium chloride dihydrate (CaCl₂·2H₂O) was dissolved in an appropriate amount of water; the solution was transferred to a 100-ml volumetric flask; and water was added up to the marked line.

Solution C: 2.250 g of magnesium sulfate heptahydrate (MgSO₄·7H₂O) was dissolved in an appropriate amount of water; the solution was transferred to a 100-ml volumetric flask; and water was added up to the marked line.

Solution D: 0.025 g of iron (III) chloride hexahydrate (FeCl₃·6H₂O) was dissolved in an appropriate amount of water; the solution was transferred to a 100-ml volumetric flask; and water was added up to the marked line.

The temperatures of the medium stock solutions A to D were adjusted to 25°C. The stock solution A in an amount of 10 ml was added to a 1-L volumetric flask using a volumetric pipette, and diluted with about 800 ml of water. Thereafter, 1 ml each of the stock solutions B, C, and D was added using a volumetric pipette and the mixture was diluted to the marked line with water having a temperature of 25°C. The required number of the thus-prepared media for the test were prepared. Each of the prepared media was transferred to a 5-L beaker, mixed, and bubbled for at least one hour under stirring.

### Preparation of sludge solution

The sludge used for the biodegradability test was obtained from the Minami Suita wastewater treatment plant. First, the concentration of the sludge obtained was measured by the following technique. Bubbling of the obtained sludge was performed under stirring and 5 ml of the sludge taken with a volumetric pipette was subjected to suction filtration using filter paper. Five sheets of filter paper on which the sludge was collected in this manner were prepared, and dried in a drier at 105°C for one hour. The average weight loss of the five sheets was calculated to determine the concentration of the sludge. The sludge was diluted with the medium prepared above to prepare a 1000 ppm sludge solution.

### Preparation of aqueous polymer solution

The polymer (1) obtained in Production Example (1) was diluted with pure water to obtain a 2% by mass aqueous polymer solution. Separately, as a standard substance, sodium benzoate was diluted with pure water to obtain a 2% by mass aqueous sodium benzoate solution.

### BOD test

The BOD was measured using a pressure sensor BOD meter. In an incubation bottle, 144.75 g of the medium prepared above was weighted out, and 0.75 g of the 2% aqueous polymer solution was added thereto. Separately, for blank measurement, 0.75 g of pure water was added, and for standard substance measurement, 0.75 g of the 2% aqueous sodium benzoate solution was added. Thereafter, the pH of the solution in the incubation bottle was measured, and adjusted with a 0.1 M aqueous hydrochloric acid solution to fall within 7.4 ± 0.2. To the solution was added 4.5 ml of the 1000 ppm sludge solution to prepare a test solution. A stir bar was placed in the incubation bottle. A CO₂ absorbent holder containing 1.8 g of a CO₂ absorbent (YABASHI LIME) was inserted into the bottle and a BOD sensor was attached to the bottle. The incubation bottle equipped with the BOD sensor was placed in a constant temperature bath at 24°C and the contents were stirred. Thereby, the BOD value was calculated from the pressure sensor.

### Calculation of degradation rate

The theoretical oxygen demand (ppm) of the polymers was calculated, the difference between the BOD value obtained from the blank measurement and the BOD value obtained from the measurement using the polyalkylene oxide-containing compounds was determined, and the degradation rate was calculated from the difference. The degradation rate 28 days after the start of the test was defined as the biodegradation rate.

The degradation rate of the polymer (1) 28 days after the start of the test was 71%. Degradation rate (%) = (Biochemical oxygen consumption derived from polymer)/(Theoretical oxygen demand of polymer) × 100

### <Alkaline degradation test>

A glass test tube equipped with a thermometer and a stir bar was charged with 0.180 g of any of the polymers. Then, 5.820 g of a 0.1 M NaOH aqueous solution (FUJIFILM Wako Pure Chemical Corporation) was added thereto to prepare a 3% solution of the polymer. Subsequently, under stirring, the polymer solution was heated to 80°C and maintained for two hours. The heated polymer solution was diluted 10 times with the eluent described in the above molecular weight measurement conditions, and subjected to measurement of molecular weight. Table 3 shows the number average molecular weights before and after alkaline degradation and the ratio of the molecular weight after alkaline degradation to the molecular weight before alkaline degradation.

**[Table 1]**

| Polymer in examples | Clay dispersing ability | Anti-redeposition rate |
|---|---|---|
| Polymer (1) | 1.3 | 94.5% |
| Polymer (2) | 0.78 | 95.0% |
| Polymer (3) | 0.82 | 94.5% |
| Polymer (4) | 1.04 | 91.2% |
| Polymer (5) | 0.96 | 92.5% |
| Comparative composition (1) | 0.61 | 87.4% |

The results in Table 1 demonstrate that the polymers (1) to (5) as the polyalkylene oxide-containing compounds of the present disclosure exhibit an excellent clay dispersing ability and an anti-redeposition ability.

**[Table 2]**

| Polymer in examples | Anti-redeposition rate (2) |
|---|---|
| Polymer (10) | 74.8% |
| Polymer (11) | 77.8% |
| Polymer (13) | 79.9% |
| Polymer (14) | 75.6% |
| Polymer (16) | 74.9% |
| Polymer (17) | 75.2% |
| Polymer (19) | 74.5% |
| Polymer (21) | 76.6% |
| Polymer (23) | 75.8% |
| Polymer (25) | 77.3% |
| Polymer (26) | 75.8% |
| Polymer (27) | 75.6% |
| Polymer (28) | 74.8% |
| Polymer (30) | 79.2% |
| Polymer (31) | 77.9% |
| Polymer (33) | 80.3% |
| Polymer (34) | 78.0% |
| Polymer (36) | 81.4% |
| Polymer (37) | 80.8% |
| Polymer (39) | 78.6% |
| Polymer (40) | 77.1% |
| Polymer (42) | 79.0% |
| Polymer (43) | 77.5% |
| Polymer (45) | 80.4% |
| Polymer (46) | 80.6% |
| Polymer (48) | 80.3% |
| Polymer (49) | 78.7% |
| Polymer (51) | 80.9% |
| Polymer (52) | 77.5% |
| Polymer (54) | 80.1% |
| Polymer (55) | 79.7% |
| Comparative composition (2) | 73.3% |
| No additive | 72.1% |

The results in Table 2 demonstrate that the polymers (10) to (55) as the polyalkylene oxide-containing compounds of the present disclosure exhibit an excellent anti-redeposition ability.

**[Table 3]**

| Polymer in examples | Number average molecular weight (before decomposition) | Number average molecular weight (after decomposition) | After decomposition /Before decomposition |
|---|---|---|---|
| Polymer (8) | 6830 | 940 | 0.14 |
| Polymer (11) | 6600 | 1060 | 0.16 |
| Polymer (30) | 6510 | 1100 | 0.17 |
| Polymer (39) | 6530 | 1170 | 0.18 |
| Polymer (48) | 7020 | 1400 | 0.20 |

The results in Table 3 demonstrate that the polymers (8), (11), (30), (39), and (48) as the polyalkylene oxide-containing compounds of the present disclosure exhibit excellent alkaline degradation properties.

## Claims

1. A polyalkylene oxide-containing compound comprising:
a cationic group;
a linking group; and
a structural unit derived from a polyalkylene oxide,
the linking group being bonded to the structural unit derived from a polyalkylene oxide.

2. The polyalkylene oxide-containing compound according to claim 1,
wherein the linking group is one or more substituents selected from ester, thioester, amide, thioamide, acetal, hemiacetal, and hemiketal groups.

3. The polyalkylene oxide-containing compound according to claim 1 or 2, comprising:
a substituent represented by the following formula (1); and
a cationic group containing two or more nitrogen atoms,
the substituent represented by the following formula (1) being bonded to at least one of the nitrogen atoms in the cationic group,
the formula (1) being as follows: 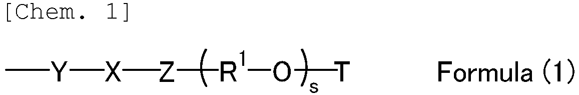 wherein R¹s are the same as or different from each other and each represent a C2-C6 hydrocarbon group; X represents one or more selected from a -C(=α¹)-β¹- group, a >C=α¹ group, a -α¹-CR²R³-β¹- group, and a -CR⁴(OH)-α¹- group, where α¹ and β¹ are the same as or different from each other and each represent a heteroatom or a group in which a hydrogen atom is bonded to a heteroatom, R², R³, and R⁴ are the same as or different from each other and each represent a hydrogen atom or a C1-C10 organic group; s is an integer from 1 to 300; Y represents a direct bond, a linear or branched C1-C10 hydrocarbon group, or a substituent represented by the following formula (2); Z represents a direct bond, a - (CH₂)ₙ-(S)ₘ-(CH₂)ₚ-O- group, a -(CH₂)ₙ-α²- group, or a substituent represented by the following formula (3), where α² represents a heteroatom or a group in which a hydrogen atom is bonded to a heteroatom, m is 0 or 1, n is an integer from 1 to 10, and p is an integer from 1 to 10; and T represents a hydrogen atom or a C1-C30 organic group,
the formula (2) being as follows: 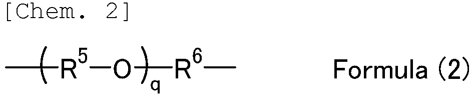 wherein q is an integer from 1 to 300; and R⁵ and R⁶ are the same as or different from each other and each represent a C2-C6 hydrocarbon group,
the formula (3) being as follows: 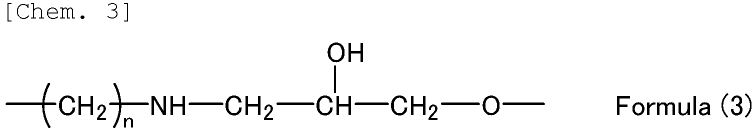 wherein n is an integer from 1 to 10.

4. The polyalkylene oxide-containing compound according to claim 3,
wherein the heteroatom for α¹ and β¹ in X in the substituent represented by the formula (1) is an oxygen atom or a nitrogen atom.

5. The polyalkylene oxide-containing compound according to claim 3 or 4,
wherein Y in the substituent represented by the formula (1) is a C2-C4 hydrocarbon group.

6. The polyalkylene oxide-containing compound according to any one of claims 1 to 5, comprising:
a cationic group to which no linking group is bonded; and
an unreacted NH group in the cationic group to which no linking group is bonded is present in an amount of 80 mol% or less based on the total number of moles of nitrogen atoms in the polyalkylene oxide-containing compound.

7. The polyalkylene oxide-containing compound according to any one of claims 1 to 6,
wherein a ratio of a number average molecular weight of a degradation product obtained by degrading the polyalkylene oxide-containing compound by a degradation test by alkaline hydrolysis or enzymatic degradation is 0.5 or less relative to a number average molecular weight of the polyalkylene oxide-containing compound before the degradation test.

8. A composition comprising:
the polyalkylene oxide-containing compound according to any one of claims 1 to 7; and
an acid compound.

9. A method for producing the polyalkylene oxide-containing compound according to any one of claims 1 to 6, the method comprising
Michael addition of an α,β-unsaturated carbonyl compound having a polyalkylene oxide chain to an amino group as a cationic group.

10. A method for producing the polyalkylene oxide-containing compound according to any one of claims 1 to 6, the method comprising:
a first step of Michael addition of an α,β-unsaturated carbonyl compound to an amino group as a cationic group; and
a second step of introducing a polyalkylene oxide chain into a product obtained in the first step.

11. A method for producing the polyalkylene oxide-containing compound according to any one of claims 1 to 6, the method comprising:
a ring-opening addition reaction of one or more compounds selected from cyclic lactone compounds and cyclic lactam compounds with an amino group as a cationic group; and
introducing a polyalkylene oxide chain into active hydrogen generated in the ring-opening addition reaction.

12. A method for producing the polyalkylene oxide-containing compound according to any one of claims 1 to 6, the method comprising
reducing an amount of an unreacted NH group in a cationic group to which no linking group is bonded by one or more reactions selected from a Michael addition reaction, an acetylation reaction, an amidation reaction with a carboxylic acid anhydride, an amidation reaction with a carboxylic acid halide, and an epoxy compound addition reaction.

13. A method for producing the polyalkylene oxide-containing compound according to any one of claims 1 to 6, the method comprising
neutralizing with an acid compound an unreacted NH group in a cationic group to which no linking group is bonded.

14. A detergent or cleaning composition comprising the polyalkylene oxide-containing compound according to any one of claims 1 to 6.

15. The detergent or cleaning composition according to claim 14,
wherein the detergent or cleaning composition is liquid and is selected from the group consisting of a laundry detergent composition, a hard surface cleaning composition, a hand dishwashing composition, and an automatic dishwashing composition.

16. The detergent or cleaning composition according to claim 14,
wherein the detergent or cleaning composition is selected from the group consisting of a laundry detergent composition, a hard surface cleaning composition, a hand dishwashing composition, and an automatic dishwashing composition and is a liquid detergent or a liquid cleaning composition, and the liquid detergent or the liquid cleaning composition is enclosed in a single-compartment water-soluble pouch in a single-phase unit dose form or in a multi-compartment water-soluble pouch in a multiphase unit dose form.

17. The detergent or cleaning composition according to any one of claims 14 to 16, further comprising
a surfactant selected from the group consisting of anionic surfactants, cationic surfactants, nonionic surfactants, amphoteric surfactants, zwitterionic surfactants, and mixtures thereof.

18. The detergent or cleaning composition according to claim 17,
wherein the surfactant is an anionic surfactant selected from the group consisting of alkylbenzene sulfonates, alkoxylated alkyl sulfates, alkyl sulfates, and mixtures thereof.

19. The detergent or cleaning composition according to any one of claims 14 to 18,
wherein the detergent or cleaning composition is a liquid laundry detergent composition further comprising one or more cleaning adjunct additives selected from the group consisting of builders, structurants or thickeners, clay-soil removal or clay-soil anti-redeposition agents, stain release polymers, dispersant polymers, grease cleaning polymers, enzymes, enzyme stabilizing systems, bleaching compounds, bleaching agents, bleach activators, bleach catalysts, brighteners, dyes, hueing agents, dye transfer inhibitors, chelating agents, defoamers, softeners, fragrances, and mixtures thereof.

20. The detergent or cleaning composition according to any one of claims 14 to 19,
wherein the detergent or cleaning composition is substantially free of a zeolite builder nor a phosphate builder.

21. A softener comprising the polyalkylene oxide-containing compound according to any one of claims 1 to 7.

22. A film composition comprising the polyalkylene oxide-containing compound according to any one of claims 1 to 7.

23. A method for storing the polyalkylene oxide-containing compound according to any one of claims 1 to 7 at a water concentration of 10% or less.
